# EUROPEAN PATENT SPECIFICATION

(11) **EP 2 230 302 B1**
(45) Date of publication and mention of the grant of the patent: **24.07.2013**
(21) Application number: 10002572.5
(22) Date of filing: 11.03.2010
(51) Int. Cl.: C12N 15/09, C12P 13/12, C12R 1/185, C12R 1/22, C12R 1/42, C12R 1/425

(54) **An L-cysteine-producing bacterium and a method for producing L-cysteine**
Ein L-cysteine-produzierendes Bakterium und eine Methode zur Herstellung von L-cysteine
Bactérie produisant de la L-cystéine et méthode de production de L-cystéine

(30) Priority: 12.03.2009 JP 2009059792
(43) Date of publication of application: 22.09.2010
(73) Proprietor: Ajinomoto Co., Inc., Tokyo 104-8315 (JP)
(72) Inventor: Nonaka, Gen, Kawasaki-shi Kanagawa 210-8681 (JP)
(74) Representative: Strehl Schübel-Hopf & Partner

(56) References cited:
- SERMON JAN ET AL: "CorA affects tolerance of Escherichia coli and Salmonella enterica serovar typhimurium to the lactoperoxidase enzyme system but not to other forms of oxidative stress" APPLIED AND ENVIRONMENTAL MICROBIOLOGY, vol. 71, no. 11, November 2005 (2005-11), pages 6515-6523, XP002579973 ISSN: 0099-2240
- BUTLER J D ET AL: "Amino acid composition and N-terminal sequence of purified Cystine Binding Protein of Escherichia coli" LIFE SCIENCES, PERGAMON PRESS, OXFORD, GB LNKD- DOI:10.1016/0024-3205(93)90103-A, vol. 52, no. 14, 1 January 1993 (1993-01-01), pages 1209-1215, XP023766767 ISSN: 0024-3205 [retrieved on 1993-01-01]
- HOSIE ARTHUR H F ET AL: "Bacterial ABC transporters of amino acids" RESEARCH IN MICROBIOLOGY, vol. 152, no. 3-4, April 2001 (2001-04), pages 259-270, XP002579974 ISSN: 0923-2508
- KIRKPATRICK C ET AL: "Acetate and formate stress: opposite-responses in the proteome of Escherichia coli" JOURNAL OF BACTERIOLOGY, AMERICAN SOCIETY FOR MICROBIOLOGY, WASHINGTON, DC; US LNKD- DOI:10.1128/JB.183.21.6466-6477.2001, vol. 183, no. 21, 1 November 2001 (2001-11-01), pages 6466-6477, XP003009622 ISSN: 0021-9193

## Description

### BACKGROUND OF THE INVENTION

### Field of the Invention

The present invention relates to a method for producing L-cysteine or related substances. L-cysteine and L-cysteine-related substances are useful in the fields of drugs, cosmetics, and food.

### Background Art

L-cysteine is obtained by extraction from keratin-containing substances such as hair, horns and feathers, or by the conversion of DL-2-aminothiazoline-4-carboxylic acid as a precursor using a microbial enzyme. It is also planned to produce L-cysteine in a large scale by an immobilized enzyme method utilizing a novel enzyme.

Furthermore, it is also attempted to produce L-cysteine by fermentation utilizing a bacterium. For example, the inventors of the present invention disclosed a method for producing L-cysteine using an *Escherichia* bacterium having a suppressed L-cysteine decomposition system and a serine acetyltransferase (EC 2.3.1.30, henceforth also referred to as "SAT") in which feedback inhibition by L-cysteine is attenuated (Japanese Patent Laid-open (Kokai) No. 11-155571). Furthermore, as bacteria in which L-cysteine-producing ability is enhanced by suppressing the L-cysteine decomposition system, there are known coryneform bacteria or *Escherichia* bacteria in which activity of cystathionine-β-lyase (Japanese Patent Laid-open No. 11-155571), tryptophanase (Japanese Patent Laid-open No. 2003-169668), or O-acetylserine sulfhydrylase B (Japanese Patent Laid-open No. 2005-245311) is attenuated or deleted. A method for producing L-cysteine by using a bacterium in which L-cysteine metabolism is decontrolled by using a DNA sequence coding for SAT that has a specific mutation for attenuating feedback inhibition by L-cysteine is also known (National Publication of Translated Version in Japan (Kohyo) No. 2000-504926).

Furthermore, it is known that the *ydeD* gene which encodes the YdeD protein (Dabler et al., Mol. Microbiol., 36, 1101-1112 (2000)) and the *yfiK* gene which encodes the YfiK protein (Japanese Patent Laid-open No. 2004-49237) participate in secretion of the metabolic products of the cysteine pathway. Furthermore, there are also known techniques of enhancing L-cysteine-producing ability by increasing expression of the *mar*-locus, *acr*-locus, *cmr*-locus, *mex-*gene, *bmr*-gene or *qacA*-gene, which encode proteins suitable for secreting a toxic substance from cells (U.S. Patent No. 5,972,663), or *emrAB, emrKY, yojlH, acrEF, bcr* or *cusA* gene (Japanese Patent Laid-open No. 2005-287333).

As an L-cysteine-producing bacterium, there is also known *Escherichia coli* in which the activity of the positive transcription control factor of the cysteine regulon encoded by the *cysB* gene is increased (International Patent Publication WO01/27307).

Furthermore,, in the production of L-amino acids by fermentation, not only secretion of L-amino acids out of cells, but also uptake of L-amino acids is important. Microorganisms have an ability to take up many kinds of amino acids into cells from the environment, in which each microorganism grow, and use them. For example, *Escherichia coli* (*E. coli*) has a large number of transporters, and it is supposed that many of them participate in uptake of L-amino acids. There is even an estimation that among those predicted to be "membrane tranporter proteins" concerning the functions thereof, 14% of them participate in transport of amino acids (Paulsen et al., J. Mol. Biol., 277, 573-592 (1998)). However, there are generally a large number of various paralogues of transporters concerning substrate specificity, and many overlaps of functions (there are plural uptake systems for the same substrate). Therefore, it is extremely difficult to identify a function as a transporter (Hosie et al., Res. Microbiol., 152, 259-270 (2001)). As described above, functions and physiological roles of transporters are very complicated. Therefore, characteristics of a transporter simply estimated on the basis of homology or phenotype may not reflect physiological functions as an actual transporter. For example, it cannot be expected transportation of which substrate is a physiologically important function among transportation of plural kinds of substrates, or the like. Furthermore, even if a certain substance is found to be transported, there may be plural factors which also transport that substance. Therefore, when a microorganism used for amino acid production by fermentation is modified, it is not easy to use a transporter as a target.

Furthermore, although there are several findings as described below concerning uptake systems of bacteria for cystine among L-cysteine and related compounds thereof, there is substantially no important finding about uptake of L-cysteine and S-sulfocysteine.

It is expected that *E*. *coli* has at least two kinds of cystine uptake systems showing different kinetic characteristics (Berger et al., J. Biol. Chem., 247,7684-7694 (1972)). FliY has been demonstrated to bind to cystine in an in vitro experiment system (Butler et al., Life Sci., 52, 1209-1215 (1993)), and the *fliY* gene is expected to form an operon together with *yecC, yecS* and *yecO* existing nearby, and function as an ABC transporter (Hosie et al., Res. Microbiol., 152,259-270 (2001)). However, it has not been experimentally demonstrated yet whether they function as a physiological cystine uptake system in *E. coli.*

Although it is similarly expected that three cystine uptake systems of different kinetics are also present in *Salmonella* bacteria (Baptist et al., J. Bacteriol., 131, 111-118 (1977)), proteins involved in them and genes coding for them have not been identified yet. Furthermore, three kinds of cystine uptake systems (YckKJI, YtmJKLMN, YhcL) have been reported for *Bacillus subtilis,* and if these three kinds of systems are deleted, the bacterium becomes unable to grow with cystine as a sole sulfur source (Burguiere et al., J. Bacteriol., 186,4875-4884 (2004)).

Although it has been reported that YdjN of *E. coli* has a homology of 45% to TcyP, which is known to be involved in cystine uptake of *Bacillus subtilis* (Burguiere et al., J. Bacteriol., 186,4875-4884 (2004)), it has not been confirmed whether it actually has cystine uptake activity.

It is known that in *Lactobacillusfermentum* BR11, *bspA* codes for a cystine uptake system (Turner et al., J. Bacteriol., 181, 2192-2198 (1999)). Furthermore, although it is expected that there are two kinds of cysteine uptake systems showing different kinetics in *Legionella pneumophila* (Ewann et al., Appl. Environ. Microbiol., 72, 3993-4000 (2006)), neither genes nor proteins thereof have not been identified yet.

### SUMMARY OF THE INVENTION

An object of the present invention is to develop novel techniques for improving bacterial production of L-cysteine, and thereby provide an L-cysteine-producing bacterium, as well as a method for producing L-cysteine, L-cystine, a derivative or precursor thereof, or a mixture of these using such a bacterium.

The inventors of the present invention conducted various researches in order to achieve the aforementioned object, and as a result, found that L-cysteine-producing ability of a bacterium could be improved by codifying the bacterium to decrease activity of a protein encoded by the *ydjN* gene, and the L-cysteine-producing ability could be further improved by modifying the bacterium to decrease activity of a protein encoded by the *fliY* gene in addition to the foregoing protein, and accomplished the present invention.

The present invention thus provides the followings.
[1] A method for producing L-cysteine, L-cystine, a derivative or precursor thereof, or a mixture of these, which comprises culturing a bacterium belonging to the family *Enterobacteriaceae* in a medium and collecting L-cysteine, L-cystine, a derivative or precursor thereof, or a mixture of these from the medium, wherein said bacterium is able to produce L-cysteine, and has been modified so that expression of the *ydjN* gene coding for YdjN protein is reduced by introducing a missense mutation, a nonsense mutation, or a frame shift mutation into the coding region of the *ydjN* gene on a chromosome, by deleting a part of, or the entire coding region of the *ydjN* gene on a chromosome, or by modifying an expression control sequence of the ydjN gene, or the *ydjN* gene is disrupted.
[2] The method according to [1] above, wherein the YdjN protein has the amino acid sequence of SEQ ID NO: 2 or 4, or a variant thereof having the amino acid sequence of SEQ ID NO: 2 or 4 including substitutions, deletions, insertions or additions of one to 20 amino acid residues.
[3] The method according to [1] above, wherein the *ydjN* gene is selected from the group consisting of:
   (a) a DNA comprising the nucleotide sequence of SEQ ID NO: 1 or 3,
   (b) a DNA which is able to hybridize with a sequence complementary to the nucleotide sequence of SEQ ID NO: 1 or 3 under stringent conditions comprising washing at 60°C, 0.1×SSC, 0.1% SDS,
   (c) a DNA which has a homology of 95% or more to the nucleotide sequence of SEQ ID NO: 1 or 3.
[4] The method according to any one of [1] to [3] above, wherein said bacterium has been further modified so that expression of the *fliY* gene coding for FliY protein is reduced by introducing a missense mutation, a nonsense mutation, or a frame shift mutation into the coding region of the *fliY* gene on a chromosome, by deleting a part of, or the entire coding region of the *fliY* gene on a chromosome, or by modifying an expression control sequence of the *jliY* gene, or the *fliY* gene is disrupted.
[5] The method according to [4] above, wherein the FliY protein has the amino acid sequence of SEQ ID NO: 6 or 8, or a variant thereof having the amino acid sequence of SEQ ID NO: 6 or 8 including substitutions, deletions, insertions or additions of one to 20 amino acid residues.
[6] The method according to [4] or [5] above, wherein the *fliY* gene is selected from the group consisting of:
   (a) a DNA comprising the nucleotide sequence of SEQ ID NO: 5 or 7,
   (b) a DNA which is able to hybridize with a sequence complementary to the nucleotide sequence of SEQ ID NO: 5 or 7 under stringent conditions comprising washing at 60°C, 0.1×SSC, 0.1% SDS,
   (c) a DNA which has a homology of 95% or more to the nucleotide sequence of SEQ ID NO: 5 or 7.
[7] The method according to any one of [1] to [6], wherein said bacterium further has at least one of the following characteristics:
   i) it has been modified to increase serine acetyltransferase activity by increasing the copy number of a gene coding for serine acetyltransferase, and/or modifying an expression control sequence of a gene coding for serine acetyltransferase,
   ii) it has been modified to increase expression of the *yeaS* gene by increasing the copy number of the *yeaS* gene, and/or modifying an expression control sequence of the *yeaS* gene,
   iii) it has been modified to incorporate a mutant *serA* gene coding for a mutant 3-phosphoglycerate dehydrogenase for which the feedback inhibition is eliminated or attenuated,
   iv) it has been modified to enhance expression of the *cysPTWAM* cluster genes coding for the sulfate/thiosulfate transport system proteins.
[8] The method according to any one of [1] to [7] above, wherein said bacterium belongs to the genus *Pantoea.*
[9] The method according to [8] above, wherein said bacterium is *Pantoea ananatis.*
[10] The method according to any one of [1] to [7] above, wherein said bacterium is *Escherichia coli.*

According to the present invention, L-cysteine-producing ability of bacteria belonging to the family *Enterobacteriaceae* can be improved. Furthermore, according to the present invention, L-cysteine, L-cystine, derivatives and precursors thereof, and mixtures of them can be efficiently produced.

### BRIEF DESCRIPTION OF THE DRAWINGS

Fig. 1 shows uptake of S-sulfocysteine by *E. coli* MG1655.
Fig. 2 shows uptake of cystine by *E*. *coli* MG1655.
Fig. 3 shows uptake of cysteine by *E*. *coli* MG1655.
Fig. 4 shows uptake of S-sulfocysteine by *P. ananatis ydjN.*
Fig. 5 shows uptake of cystine by *fliY*-deficient *E*. *coli.*
Fig. 6 shows uptake of cystine by *fliY*-enhanced *E*. *coli.*
Fig. 7 shows uptake of cysteine by *fliY*-deficient *E. coli.*
Fig. 8 shows the sequence of the promoter Pnlp (SEQ ID NO: 62).

### DETAILED DESCRIPTION OF EXEMPLARY EMBODIMENTS

### <1> Bacterium

The bacterium used in the present invention belongs to the family *Enterobacteriaceae,* which is able to produce L-cysteine, and has been modified to decrease activity of the YdjN protein. A preferred embodiment of the bacterium used in the present invention is the bacterium as described above, which has been further modified to decrease the activity of the FliY protein in addition to the YdjN protein. The YdjN and FliY proteins are encoded by the *fliY and ydjN* genes, respectively. These proteins and genes will be explained later.

The L-cysteine-producing ability refers to an ability of the bacterium to produce L-cysteine in a medium or cells and cause accumulation of L-cysteine in such an amount that L-cysteine can be collected from the medium or cells when the bacterium is cultured in the medium. Furthermore, a bacterium having L-cysteine-producing ability means a bacterium which can produce and cause accumulation of a larger amount L-cysteine in a medium or cells as compared with a wild-type, parent, or unmodified strain, preferably a microorganism which can produce and cause accumulation of L-cysteine in a medium in an amount of, preferably 0.3 g/L or more, more preferably 0.4 g/L or more, particularly preferably 0.5 g/L or more.

A portion of the L-cysteine produced by the microorganism may be converted into L-cystine in the medium by the formation of a disulfide bond. Furthermore, as described below, S-sulfocysteine may be generated by the reaction of L-cysteine and thiosulfuric acid which are present in the medium (Szczepkowski T.W., Nature, vol. 182 (1958)). Furthermore, L-cysteine generated in bacterial cells may be condensed with a ketone, aldehyde, or, for example, pyruvic acid, which is present in the cells, to produce a thiazolidine derivative via a hemithioketal (refer to Japanese Patent No. 2992010). Thiazolidine derivative and hemithioketal may exist as an equilibrated mixture. Therefore, the ability to produce L-cysteine is not limited to the production of only L-cysteine in a medium or cells, but also includes the production of L-cystine or a derivative or precursor thereof, or a mixture of these, in addition to L-cysteine,. Examples of the aforementioned derivative of L-cysteine or L-cystine include, for example, S-sulfocysteine, thiazolidine derivatives, hemithioketals, and so forth. Examples of the precursor of L-cysteine or L-cystine include, for example, O-acetylserine, which is a precursor of L-cysteine. The precursors of L-cysteine or L-cystine also include derivatives of the precursors, and examples include, for example, N-acetylserine, which is a derivative of O-acetylserine, and so forth.

O-Acetylserine (OAS) is a precursor of L-cysteine biosynthesis. OAS is a metabolite of bacteria and plants, and is produced by acetylation of L-serine induced as an enzymatic reaction catalyzed by serine acetyltransferase (SAT). OAS is further converted into L-cysteine in cells.

The ability to produce L-cysteine can be inherent to the bacterium, or it may be obtained by modifying a microorganism such as those described below by mutagenesis or a recombinant DNA technique. In the present invention, unless specially mentioned, the term L-cysteine may be used to refer to reduced type L-cysteine, L-cystine, a derivative or precursor such as those mentioned above or a mixture thereof.

The bacterium used for the present invention is not particularly limited so long as the bacterium belongs to the family *Enterobacteriaceae* such as those of the genera *Escherichia, Enterobacter, Pantoea, Klebsiella, Serratia, Erwinia, Salmonella* and *Morganella,* and has L-cysteine-producing ability. Specifically, those classified into the family *Enterobacteriaceae* according to the taxonomy used in the NCBI (National Center for Biotechnology Information) database (http://www.ncbi.nlm.nih.gov/Taxonomy/Browser/wwwtax.cgi?id=91347) can be used. As a parent strain of the family *Enterobacteriaceae* used for the modification, a bacterium of the genus *Escherichia, Enterobacter, Pantoea, Erwinia,* or *Klebsiella* can be used.

Although the *Escherichia* bacteria are not particularly limited, specifically, those described in the work of Neidhardt et al. (Hackmann B.J., 1996, Derivations and Genotypes of some mutant derivatives of Escherichia coli K-12, p.2460-2488, Table 1, In F.D. Neidhardt (ed.), Escherichia coli and Salmonella Cellular and Molecular Biology/Second Edition, American Society for Microbiology Press, Washington, D.C.) can be used. *Escherichia coli* is preferable. Examples of *Escherichia coli* include bacteria derived from the prototype wild-type strain, K12 strain, such as *Escherichia coli* W3110 (ATCC 27325), *Escherichia coli* MG1655 (ATCC 47076) and so forth.

These strains are available from, for example, the American Type Culture Collection (Address: 12301 Parklawn Drive, Rockville, Maryland 20852, P.O. Box 1549, Manassas, VA 20108, United States of America). That is, registration numbers are given to each of the strains, and the strains can be ordered by using these registration numbers (refer to http://www.atcc.org/). The registration numbers of the strains are listed in the catalogue of the American Type Culture Collection.

Examples of the *Enterobacter* bacteria include *Enterobacter agglomerans, Enterobacter aerogenes* and so forth, and examples of the *Pantoea* bacteria include *Pantoea ananatis.* Some strains of *Enterobacter agglomerans* were recently reclassified into *Pantoea agglomerans, Pantoea ananatis,* or *Pantoea stewartii* on the basis of nucleotide sequence analysis of 16S rRNA etc. A bacterium belonging to the genus *Enterobacter* or *Pantoea* may be used so long as it is classified into the family *Enterobacteriaceae.*

In particular, *Pantoea* bacteria, *Erwinia* bacteria, and *Enterobacter* bacteria are classified as γ-proteobacteria, and they are taxonomically very close to one another (J. Gen. Appl. Microbiol., 1997, 43, 355-361; International Journal of Systematic Bacteriology, Oct. 1997, pp.1061-1067). In recent years, some bacteria belonging to the genus *Enterobacter* were reclassified as *Pantoea agglomerans, Pantoea dispersa,* or the like, on the basis of DNA-DNA hybridization experiments etc. (International Journal of Systematic Bacteriology, July 1989, 39(3), pp.337-345). Furthermore, some bacteria belonging to the genus *Erwinia* were reclassified as *Pantoea ananas* or *Pantoea stewartii* (refer to International Journal of Systematic Bacteriology, Jan. 1993; 43(1), pp.162-173).

Examples of the *Enterobacter* bacteria include, but are not limited to, *Enterobacter agglomerans, Enterobacter aerogenes,* and so forth. Specifically, the strains exemplified in European Patent Publication No. 952221 can be used. A typical strain of the genus *Enterobacter* is the *Enterobacter agglomeranses* ATCC 12287 strain.

Typical strains of the *Pantoea* bacteria include, but are not limited to, *Pantoea anonalis, Pantoea stewartii, Pantoea agglomerans,* and *Pantoea citrea.* Specific examples of *Pantoea ananatis* include the *Pantoea ananatis* AJ13355 strain, SC17 strain, and SC17(0) strain. The SC17 strain was selected as a low phlegm-producing mutant strain from the AJ 13355 strain (FERM BP-6614) isolated from soil in Iwata-shi, Shizuoka-ken, Japan as a strain that can proliferate in a low pH medium containing L-glutamic acid and a carbon source (U.S. Patent No. 6,596,517). The SC17(0) strain was constructed to be resistant to the λ Red gene product for performing gene disruption in *Pantoea ananatis* (WO2008/075483). The SC17 strain was deposited at the National Institute of Advanced Industrial Science and Technology, International Patent Organism Depository (address: Tsukuba Central 6, 1-1, Higashi 1-Chome, Tsukuba-shi, Ibaraki-ken, 305-8566, Japan) on February 4, 2009, and assigned an accession number of FERM ABP-11091. The SC17(0) strain was deposited at the Russian National Collection of Industrial Microorganisms (VKPM), GNII Genetika (address: Russia, 117545 Moscow, 1 Dorozhny proezd. 1) on September 21, 2005 with an accession number of VKPM B-9246.

The *Pantoea ananatis* AJ13355 strain was deposited at the National Institute of Bioscience and Human-Technology, Agency of Industrial Science and Technology, Ministry of International Trade and Industry (currently, the National Institute of Advanced Industrial Science and Technology, International Patent Organism Depositary, Address: Tsukuba Central 6, 1-1, Higashi 1-Chome, Tsukuba-shi, Ibaraki-ken, 305-8566, Japan) on February 19,1998 and assigned an accession number of FERM P-16644. It was then converted to an international deposit under the provisions of Budapest Treaty on January 11, 1999 and assigned an accession number of FERM BP-6614. This strain was identified as *Enterobacter agglomerans* when it was isolated and deposited as the *Enterobacter agglomerans* AJ13355 strain. However, it was recently reclassified as *Pantoea ananatis* on the basis of nucleotide sequencing of 16S rRNA and so forth.

Examples of the *Erwinia* bacteria include, but are not limited to, *Erwinia amylovora* and *Erwinia carotovora,* and examples of the *Klebsiella* bacteria include *Klebsiella planticola.*

### Impartation or enhancement of L-cysteine-producing ability

Hereinafter, methods for imparting L-cysteine-producing ability to bacteria belonging to *Enterobacteriaceae,* or methods for enhancing L-cysteine-producing ability of such bacteria, are described.

To impart the ability to produce L-cysteine, methods conventionally employed in the breeding of coryneform bacteria or bacteria of the genus *Escherichia* (see "Amino Acid Fermentation", Gakkai Shuppan Center (Ltd.), 1st Edition, published May 30, 1986, pp. 77-100) can be used. Such methods include by acquiring the properties of an auxotrophic mutant, an analogue-resistant strain, or a metabolic regulation mutant, or by constructing a recombinant strain so that it overexpresses L-cysteine biosynthesis enzyme. Here, in the breeding of an L-cysteine-producing bacteria, one or more of the above described properties such as auxotrophy, analogue resistance, and metabolic regulation mutation may be imparted. The expression of L-cysteine biosynthesis enzyme(s) can be enhanced alone or in combinations of two or more. Furthermore, the methods of imparting properties such as an auxotrophy, analogue resistance, or metabolic regulation mutation may be combined with enhancement of the biosynthesis enzymes.

An auxotrophic mutant strain, L-cysteine analogue-resistant strain, or metabolic regulation mutant strain with the ability to produce L-cysteine can be obtained by subjecting a parent strain or wild-type strain to conventional mutatagenesis, such as exposure to X-rays or UV irradiation, or treatment with a mutagen such as N-methyl-N'-nitro-N-nitrosoguanidine, ethyl methanesulfonate (EMS) etc., and then selecting those which exhibit autotrophy, analogue resistance, or a metabolic regulation mutation and which also have the ability to produce L-cysteine from the obtained mutant strains.

L-Cysteine-producing ability of a bacterium can be improved by enhancing activity of an enzyme of the L-cysteine biosynthesis pathway or an enzyme involved in production of a compound serving as a substrate of that pathway such as L-serine, for example, 3-phosphoglycerate dehydrogenase, serine acetyltransferase, and so forth. 3-Phosphoglycerate dehydrogenase is subject to feedback inhibition by serine, and therefore the enzymatic activity thereof can be enhanced by incorporating a mutant *serA* gene coding for a mutant 3-phosphoglycerate dehydrogenase for which the feedback inhibition is eliminated or attenuated into a bacterium.

Furthermore, serine acetyltransferase is subject to feedback inhibition by L-cysteine. Therefore, the enzymatic activity thereof can be enhanced by incorporating a mutant *cysE* gene coding for a mutant serine acetyltransferase for which the feedback inhibition is eliminated or attenuated into a bacterium.

The L-cysteine-producing ability can also be improved by enhancing the activity of the sulfate/thiosulfate transport system. The sulfate/thiosulfate transport system protein group is encoded by the *cysPTWAM* gene cluster (Japanese Patent Laid-open No. 2005-137369, European Patent No. 1528108).

The L-cysteine-producing ability of a bacterium can also be improved by increasing expression of the *yeaS* gene (European Patent Laid-open No. 1016710). The nucleotide sequence of the *yeaS* gene and the amino acid sequence encoded by the gene are shown in SEQ ID NOS: 15 and 16, respectively. It is known that bacteria use various codons such as GTG besides ATG as the start codon (http://depts.washington.edu/agro/genomes/students/stanstart.htm). Although the amino acid corresponding to the initial codon gtg is indicated as Val in SEQ ID NOS: 15 and 16, it is highly possible that it is actually Met.

Specific examples of L-cysteine-producing bacteria include, but not limited to, *E. coli* JM15 transformed with multiple kinds of *cysE* gene alleles encoding serine acetyltransferase (SAT) resistant to feedback inhibition (U.S. Patent No. 6,218,168), *E*. *coli* W3110 in which a gene encoding a protein responsible for excretion of cytotoxic substances is overexpressed (U.S. Patent No. 5,972,663), *E. coli* strain having decreased cysteine desulfhydrase activity (Japanese Patent Laid-open No. 11-155571), and *E. coli* W3110 in which activity of the positive transcriptional control factor of the cysteine regulon encoded by the *cysB* gene is increased (WO01/27307).

For *E. coli,* proteins are known which have an activity of secreting L-cysteine, such as the protein encoded by *ydeD* (Japanese Patent Laid-open No. 2002-233384), the protein encoded by *yfik* (Japanese Patent Laid-open No. 2004-49237) and the proteins encoded by *emrAB, emrKY, yojlH, acrEF, bcr,* and *cusA,* respectively (Japanese Patent Laid-open No. 2005-287333) as described above. Activities of these L-cysteine secreting proteins can be increased.

Hereafter, as the method for imparting an ability to produce L-cysteine, enhancing an activity of L-cysteine biosynthesis system enzyme will be described.

Examples of the L-cysteine biosynthesis enzyme include, for example, serine acetyltransferase (SAT). The SAT activity in cells of a bacterium belonging to the family *Enterobacteriaceae* can be enhanced by increasing the copy number of a gene coding for SAT, or modifying an expression control sequence such as promoter of the gene coding for SAT. For example, a recombinant DNA can be prepared by ligating a gene fragment coding for SAT with a vector, such as a multi-copy vector, which is able to function in the chosen host bacterium belonging to the family *Enterobacteriaceae* to prepare a recombinant DNA. This recombinant DNA can then be introduced into a host bacterium belonging to the family *Enterobacteriaceae* to transform it.

Methods for enhancing expression of the SAT gene will be described below. Similar methods can also be applied to other L-cysteine biosynthesis systems enzyme genes, the *yeaS* gene, and genes of proteins having cysteine secretion activity.

Modification for enhancing expression of the SAT gene can be attained by, for example, increasing copy number of the SAT gene in the cells by means of genetic recombination techniques. For example, a recombinant DNA can be prepared by ligating a DNA fragment containing the SAT gene with a vector, preferably a multi-copy vector, which is able to function in a host bacterium, and introduced into a bacterium to transform it.

For example, the SAT gene of *Escherichia coli* can be obtained by PCR using chromosomal DNA of *Escherichia coli* as a template and primers prepared on the basis of the nucleotide sequence of SEQ ID NO: 9. The SAT genes of other bacteria can also be obtained from chromosomal DNA or chromosomal DNA library of the bacteria by hybridization using a probe prepared on the basis of the aforementioned sequence information.

Copy number of the SAT gene can also be increased by introducing multiple copies of the SAT gene into a chromosomal DNA of a bacterium. To introduce multiple copies of the SAT gene into a chromosomal DNA of a bacterium, homologous recombination can be performed with targeting a sequence present on a chromosomal DNA in a multiple copy number. A repetitive DNA or inverted repeat present at the end of a transposable element can be used as the sequence present on a chromosomal DNA in a multiple copy number. Alternatively, as disclosed in Japanese Patent Laid-open No. 2-109985, multiple copies of the SAT gene can be introduced into a chromosomal DNA by incorporating them into a transposon and transferring it.

Furthermore, besides the amplification of copy number of gene described above, expression of the SAT gene can also be enhanced by replacing an expression regulatory sequence of the SAT gene such as a promoter on a chromosomal DNA or a plasmid with a stronger promoter, by amplifying a regulator which increases expression of the SAT gene, or by deleting or attenuating a regulator which reduces expression of the SAT gene. As strong promoters, for example, *lac* promoter, *trp* promoter, *trc* promoter and so forth are known. Furthermore, a promoter of the SAT gene can also be modified to be stronger by introducing substitution of nucleotides or the like into the promoter region of the SAT gene. The aforementioned substitution or modification of the promoter enhances expression of the SAT gene. Examples of methods for evaluating strength of promoters and strong promoters are described in an article by Goldstein and Doi (Goldstein, M.A. and Doi R.H., 1995, Prokaryotic promoters in biotechnology, Biotechnol. Annu. Rev., 1, 105-128), and so forth. Modification of an expression regulatory sequence can be combined with the increasing copy number of the SAT gene. Furthermore, in order to enhance production of the SAT protein, a mutation can be introduced near the translation initiation site of the SAT gene to increase translation efficiency, and this can be combined with enhancement of expression of the SAT gene.

Increase of expression of the SAT gene and increase of the SAT protein amount can be confirmed by quantifying mRNA or by Western blotting using an antibody, as the confirmation of decrease in transcription amount of a target gene and the confirmation of decrease in a target protein amount described later.

As the SAT gene, an SAT gene derived from *Escherichia* bacteria or an SAT gene derived from other organisms can be used. As the gene coding for SAT of *Escherichia coli, cycE* has been cloned from a wild-type strain and an L-cysteine excretion mutant strain, and the nucleotide sequence thereof has been elucidated (Denk, D. and Boeck, A., J. General Microbiol., 133,515-525 (1987)). The nucleotide sequence thereof and the amino acid sequence encoded by the nucleotide sequence are shown in SEQ ID NOS: 9 and 10, respectively. A SAT gene can be obtained by PCR utilizing primers prepared based on the nucleotide sequence and chromosomal DNA of *Escherichia* bacterium as the template (refer to Japanese Patent Laid-open No. 11-155571). Genes coding for SAT of other organisms can also be obtained in a similar manner. Expression of the SAT gene as described above can be enhanced in the same manner as that for the *cysE* gene explained above.

When a suppression mechanism such as "feedback inhibition by L-cysteine" exists for the expression of the SAT gene, expression of the SAT gene can also be enhanced by modifying an expression regulatory sequence or a gene involved in the suppression so that the expression of the SAT gene is insensitive to the suppression mechanism.

For example, the SAT activity can be further increased by mutating the SAT so that the feedback inhibition by L-cysteine is reduced or eliminated in the bacterium (henceforth also referred to as "mutant SAT"). Examples of the mutant SAT include SAT having a mutation replacing an amino acid residue corresponding to the methionine residue at position 256 of a wild-type SAT (SEQ ID NO: 10) with an amino acid residue other than lysine residue and leucine residue, or a mutation deleting a C-terminus side region from an amino acid residue corresponding to the methionine residue as position 256. Examples of the amino acid residues other than lysine and leucine include the 17 amino acid residues which typically make up proteins except for methionine, lysine and leucine. Isoleucine and glutamic acid are more preferred examples. To introduce a desired mutation into a wild-type SAT gene, site-specific mutagenesis can be used. As a mutant SAT gene, a mutant *cysE* coding for a mutant SAT of *Escherichia coli* is known (refer to International Patent Publication WO97/15673 and Japanese Patent Laid-open No. 11-155571). *Escherichia coli* JM39-8 strain harboring a plasmid pCEM256E containing a mutant *cysE* coding for a mutant SAT in which methionine residue at position 256 is replaced with a glutamic acid residue (*E. coli* JM39-8(pCEM256E), private number: AJ13391) was deposited at the National Institute of Bioscience and Human-Technology, Agency of Industrial Science and Technology (Postal code: 305, 1-3 Higashi 1-Chome, Tsukuba-shi, Ibaraki-ken, Japan) on November 20, 1997 and assigned an accession number of FERM P-16527. The deposit was then converted to an international deposit under the provisions of Budapest Treaty on July 8, 2002, and assigned an accession number of FERM BP-8112.

Although a "SAT insensitive to feedback inhibition by L-cysteine" can be SAT which has been modified so that it is insensitive to the feedback inhibition by L-cysteine, it can be a SAT which in its native form is insensitive to the feedback inhibition by L-cysteine. For example, SAT of *Arabidopsis thaliana* is known to be not subject to the feedback inhibition by L-cysteine and can be suitably used for the present invention. As a plasmid containing the SAT gene derived from *Arabidopsis thaliana,* pEAS-m is known (FEMS Microbiol. Lett., 179 (1999) 453-459).

Furthermore, the ability to produce L-cysteine can also be improved by enhancing expression of the *cysPTWAM* cluster genes coding for the sulfate/thiosulfate transport system proteins (Japanese Patent Laid-open No. 2005-137369, EP 1528108).

Furthermore, a sulfide can be incorporated into O-acetyl-L-serine via a reaction catalyzed by the O-acetylserine (thiol)-lyase A or B encoded by the *cysK* and *cysM* genes, respectively, to produce L-cysteine. Therefore, the ability to produce L-cysteine can also be improved by enhancing expression of the genes coding for these enzymes.

Moreover, L-cysteine-producing ability can also be improved by suppressing the L-cysteine decomposition system. "L-cysteine decomposition system is suppressed" can mean that intracellular L-cysteine decomposition activity is decreased as compared to that of a non-modified strain such as a wild-type or parent strain. As proteins responsible for the L-cysteine decomposition system, cystathionine-β-lyase (*metC* product, Japanese Patent Laid-open No. 11-155571, Chandra et al., Biochemistry, 21 (1982) 3064-3069), tryptophanase (*tnyA* product, Japanese Patent Laid-open No. 2003-169668, Austin Newton et al., J. Biol. Chem., 240 (1965) 1211-1218)), O-acetylserine sulfhydrylase B (*cysM* gene product, Japanese Patent Laid-open No. 2005-245311) and the *malY* gene product (Japanese Patent Laid-open No. 2005-245311) are known. By decreasing the activities of these proteins, L-cysteine-producing ability can be improved.

Modification for decreasing activity of a protein can be attained in the same manner as those for the *fliY* or *ydjN* gene described later.

The nucleotide sequence of the *cysM* gene of *Escherichia coli* and the amino acid sequence encoded by the gene are shown in SEQ ID NOS: 25 and 26, respectively.

### Decrease of activities of YdjN protein and FliY protein

The bacterium used in the present invention can be obtained by modifying such a bacterium belonging to the family *Enterobacteriaceae* and having L-cysteine-producing ability as described above to decrease the activity of the YdjN protein or the activities of the YdjN protein and the FliY protein. After a bacterium is modified to decrease the activity of the YdjN protein or the activities of the YdjN and FliY proteins, L-cysteine-producing ability may be imparted to the bacterium. The YdjN protein and the FliY protein are proteins encoded by the *ydjN* gene and the *fliY* gene, respectively. The activities of the YdjN and FliY proteins of a bacterium can be decreased by, for example, modifying the bacterium having the *fliY* and *ydjN* genes to decrease the activities of FliY and YdjN encoded by these genes. In order to enhance the L-cysteine-producing ability, either the FliY activity or the YdjN activity may be decreased, but it is preferable to decrease the YdjN activity, and it is more preferable to decrease both the activities.

In the present invention, "decrease" of activity include decrease of the activity of a modified strain to a level lower than that of a wild-type or a non-modified strain, and complete disappearance of the activity, unless especially specified.

The inventors of the present invention found novel genes coding for proteins of which deletion from the chromosomal DNA of *Pantoea ananatis* enhanced the L-cysteine-producing ability, and designated them *fliY and ydjN,* respectively, since they showed high homology to *fliY* and *ydjN* of *E*. *coli* (78% and 80%, respectively). In this specification, "homology" may means "identity".

In the present invention, in addition to the *fliY* and *ydjN* genes of *E*. *coli*, *fliY* and *ydjN* genes of *Pantoea ananatis,* and homologue genes of those genes of other bacteria may also be called, *fliY* gene *and ydjN* gene, respectively.

Specific examples of the *fliY* gene include a gene comprising the nucleotide sequence shown in SEQ ID NO: 5 or 7. Specific examples of the *ydjN* gene include a gene comprising the nucleotide sequence shown in SEQ ID NO: 1 or 3.

The *fliY* gene of the *Escherichia coli* MG1655 strain is shown in SEQ ID NO: 5, and the amino acid sequence encoded by the gene is shown in SEQ ID NO: 6. The *fliY* gene of the *Pantoea ananatis* SC17 strain is shown in SEQ ID NO: 7, and the amino acid sequence encoded by the gene is shown in SEQ ID NO: 8.

The nucleotide sequence of the *ydjN* gene of the *Escherichia coli* MG1655 strain is shown in SEQ ID NO: 1, and the amino acid sequence encoded by the gene is shown in SEQ ID NO: 2. The nucleotide sequence of the *ydjN* gene of the *Pantoea ananatis* SC17 strain is shown in SEQ ID NO: 3, and the amino acid sequence encoded by the gene is shown in SEQ ID NO: 4.

The FliY and YdjN proteins are not limited to proteins having the aforementioned amino acid sequences and homologues thereof, and they may be a variant thereof. The *fliY* or *ydjN* gene may be a gene coding for a variant of the FliY or YdjN protein. A variant of the FliY or the YdjN protein means a protein having the amino acid sequence of SEQ ID NO: 2, 4, 6 or 8 including substitutions, deletions, insertions or additions of one or several amino acid residues at one or several positions, and having the function of the FliY or YdjN protein. Although the number meant by the aforementioned term "one or several" may differ depending on positions of amino acid residues in the three-dimensional structure of the protein or the types of amino acid residues, specifically, it is 1 to 20, preferably 1 to 10, more preferably 1 to 5.

A *ydjN* gene-deficient strain shows decreased uptake of S-sulfocysteine and L-cystine as shown in Examples section. Therefore, it is estimated that the YdjN protein has a function to participate in uptake of S-sulfocysteine and L-cystine.

On the other hand, although there is a reference pointing out the possibility of participation of FliY in uptake of L-cystine (Butler et al., Life Sci., 52, 1209-1215 (1993); Hosie et al., Res. Microbiol., 152, 259-270 (2001)), it is estimated that it does not participate in the uptake of L-cystine, or the activity thereof is lower than that of YdjN, if it participates in that uptake, as shown in Examples section. In any case, if both the *ydjN* and *fliY* genes are deleted, the L-cysteine-producing ability is markedly increased as compared with that obtainable by deletion of either one of them. Therefore, although the function of FliY is still indefinite, it is characterized that deletion thereof improves the L-cysteine-producing ability.

The aforementioned substitutions, deletions, insertions, or additions of one or several amino acid residues are a conservative mutation that preserves the normal function of the protein. The conservative mutation is-typically a conservative substitution. The conservative substitution is a mutation wherein substitution takes place mutually among Phe, Trp and Tyr, if the substitution site is an aromatic amino acid; among Leu, Ile and Val, if the substitution site is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having a hydroxyl group. Specific examples of conservative substitutions include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution ofTrp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val. The above-mentioned amino acid substitution, deletion, insertion, addition, inversion etc. can be the result of a naturally-occurring mutation (mutant or variant) due to an individual difference, a difference of species, or the like of a bacterium from which the gene is derived.

Furthermore, the gene having such a conservative mutation as described above can be a gene encoding a protein showing a homology of 80% or more, preferably 90% or more, more preferably 95% or more, still more preferably 97% or more, particularly preferably 99% or more, to the entire encoded amino acid sequence. Sequence information of the genes coding for a protein homologous to such FliY or YdjN can be easily obtained from databases opened to public by BLAST searching or FASTA searching using the wild-type *fliY* or *ydjN* gene of the aforementioned *Escherichia coli* strain as a query sequence, and the genes can be obtained by using oligonucleotides produced based on such known gene sequences as primers.

The *fliY* or *YdjN* gene can be a gene which hybridizes with a sequence complementary to the aforementioned nucleotide sequences or a probe that can be prepared from the aforementioned nucleotide sequences under stringent conditions, so long as the function of the protein encoded by the *fliY* or *YdjN* gene is maintained. The "stringent conditions" are conditions of washing at 60°C, 0.1×SSC, 0.1% SDS, once or preferably twice or three times.

The probe used for the aforementioned hybridization can have a partial sequence of a complementary sequence of the gene. Such a probe can be prepared by PCR using oligonucleotides prepared based on the known nucleotide sequences of the gene as primers, and a DNA fragment containing these sequences as the template. When a DNA fragment of a length of about 300 bp is used as the probe, the conditions of washing after hybridization can be, for example, 50°C, 2×SSC, and 0.1% SDS.

Methods for decreasing the activities of the FliY or YdjN protein will be explained below. The activities of the proteins of the L-cysteine decomposition system can also be decreased by the same methods. In the following descriptions, an objective protein of which activity is to be decreased is referred to as a "target protein", and a gene coding for the target protein is referred to as a "target gene".

Activity of a target protein can be decreased by, for example, reducing expression of a target gene. Specifically, for example, intracellular activity of the target protein can be reduced by deleting a part of, or the entire coding region of the target gene on a chromosome. For decrease of activity of a target protein, expression of the target gene can also be decreased by modifying an expression control sequence of the target gene such as promoter and Shine-Dalgarno (SD) sequence. Furthermore, the expression amount of the gene can also be reduced by modification of a non-translation region other than the expression control sequence. Furthermore, the entire gene including the sequences on both sides of the gene on a chromosome can be deleted. Furthermore, the expression of the gene can also be reduced by introducing a mutation for an amino acid substitution (missense mutation), a stop codon (nonsense mutation), or a frame shift mutation which adds or deletes one or two nucleotides into the coding region of the target gene on a chromosome (Journal of Biological Chemistry, 272:8611-8617 (1997); Proceedings of the National Academy of Sciences, USA, 95 5511-5515 (1998); Journal of Biological Chemistry, 266, 20833-20839 (1991)). Activity of a target protein can also be decreased by enhancing activity of a regulator which down-regulates the target protein, or suppressing activity of a regulator which up-regulates the target protein. Activity of a target protein can also be decreased by adding a substance which down-regulates activity or expression of the target protein, or eliminating a substance which up-regulates activity or expression of the target protein.

Furthermore, the modification can be a modification caused by a typical mutagenesis caused by X-ray or ultraviolet irradiation, or by use of a mutagen such as N-methyl-N'-nitro-N-nitrosoguanidine, so long as the modification results in a decrease of the activity of the target protein.

Modification of an expression control sequence is performed for preferably one or more nucleotides, more preferably two or more nucleotides, particularly preferably three or more nucleotides. When a coding region is deleted, the region to be deleted can be an N-terminal region, an internal region or a C-terminal region, or even the entire coding region, so long as the function of the target protein is decreased or deleted. Deletion of a longer region can usually more surely inactivate a gene. Furthermore, reading frames upstream and downstream of the region to be deleted can be the same or different.

To inactivate a gene by inserting a sequence into the coding region of the gene, the sequence can be inserted into any part of the coding region of the gene. The longer the inserted sequence, the greater the likelihood of inactivating the gene. Reading frames located upstream and downstream of the insertion site can be the same or different. The sequence to be inserted is not particularly limited so long as the insertion decreases or deletes the function of the encoded target protein, and examples include, for example, a transposon carrying an antibiotic resistance gene, a gene useful for L-cysteine production and so forth.

A target gene on the chromosome can be modified as described above by, for example, preparing a deletion-type version of the gene in which a partial sequence of the gene is deleted so that the deletion-type version of the gene does not produce a target protein which normally functions, and transforming a bacterium with a DNA containing the deletion-type gene to cause homologous recombination between the deletion-type gene and the native gene on the chromosome, and thereby substitute the deletion-type gene for the gene on the genome. The target protein encoded by the deletion-type gene has a conformation different from that of the wild-type protein, if it is even produced, and thus the function is reduced or deleted. Such gene disruption based on gene substitution utilizing homologous recombination has been already established, and there are a method called Red-driven integration (Datsenko, K.A., and Wanner, B.L., Proc. Natl. Acad. Sci. USA, 97:6640-6645 (2000)), a method of using a linear DNA such as a method utilizing the Red driven integration in combination with an excision system derived from λ phage (Cho, E.H., Gumport, R.I., Gardner, J.F., J. Bacteriol., 184:5200-5203 (2002)) (refer to W02005/010175), a method of using a plasmid containing a temperature sensitive replication origin or a plasmid capable of conjugative transfer, a method of utilizing a suicide vector not having replication origin in a host (U.S. Patent No. 6,303,383, Japanese Patent Laid-open No. 05-007491), and so forth.

Decrease of transcription level of a target gene can be confirmed by comparing amount of mRNA transcribed from the gene with that of the wild-type or non-modified strain. Examples of the method for confirming mRNA amount include Northern hybridization, RT-PCR (Molecular Cloning, Cold Spring Harbor Laboratory Press, Cold Spring Harbor (USA), 2001), and so forth.

Decrease of amount of a target protein can also be confirmed by Western blotting using an antibody (Molecular Cloning, Cold Spring Harbor Laboratory Press, Cold Spring Harbor (USA), 2001).

Furthermore, when the target protein is the YdjN protein, decrease of the amount of the protein can also be confirmed by measuring activity to take up S-sulfocysteine or L-cystine of the cell.

Whether a protein has the activity to take up the aforementioned compound can be confirmed by preparing a bacterium in which expression of a gene coding for the protein is increased from a wild strain or a parent strain, culturing this strain in a medium, and quantifying amount of L-cysteine, L-cystine, a derivative or precursor thereof, or a mixture of them accumulated in the medium. Alternatively, the activity can also be confirmed by preparing a bacterium in which expression of a gene coding for the protein is decreased or deleted from a wild-type or parent strain, culturing the strain in a medium containing S-sulfocysteine or L-cystine, and confirming decrease of the decreased amount of the compound added to the medium. Specific examples are described in Examples section.

When the *fliY* or *ydjN* gene of *Escherichia coli* is used as the *fliY* or *ydjN* gene, the *fliY* or *ydjN* gene can be obtained by PCR using chromosomal DNA of *Escherichia coli* as a template and primers prepared on the basis of the nucleotide sequence of SEQ ID NO: 5 or 1. Similarly, the *fliY* or *ydjN* gene of *Pantoea ananatis* can be obtained by PCR using chromosomal DNA of *Pantoea ananatis* as a template and primers prepared on the basis of the nucleotide sequence of SEQ ID NO: 7 or 3. The *fliY* or *ydjN* gene of other bacteria can also be obtained from chromosomes or chromosomal DNA library of the bacteria by hybridization or PCR using a probe or primers prepared on the basis of the aforementioned sequence information.

When it is necessary to increase expression of the *fliY* or *ydjN* gene in order to confirm whether the FliY or YdjN protein has the activity to take up S-sulfocysteine, L-cystine or L-cysteine, multiple copies of the gene can be introduced into a bacterium. In order to introduce multiple copies of the *fliY* or *ydjN* gene into a bacterium, the method of using a multi-copy type vector, the method of introducing multiple copies of genes into chromosomal DNA by homologous recombination, and so forth can be used as described for the SAT gene.

### <2> Method for producing L-cysteine, L-cystine, derivative or precursor thereof or mixture thereof of the present invention

These compounds can be produced by culturing the bacterium used in the present invention obtained as described above in a medium, and collecting L-cysteine, L-cystine, a derivative or precursor thereof or a mixture thereof from the medium. Examples of the derivative or precursor of L-cysteine include S-sulfocysteine, a thiazolidine derivative, a hemithioketal corresponding the thiazolidine derivative mentioned above, and so forth.

Examples of the medium used for the culture can include ordinary media containing a carbon source, nitrogen source, sulfur source, inorganic ions, and other organic components as required.

As the carbon source, saccharides such as glucose, fructose, sucrose, molasses and starch hydrolysate, and organic acids such as fumaric acid, citric acid and succinic acid can be used.

As the nitrogen source, inorganic ammonium salts such as ammonium sulfate, ammonium chloride and ammonium phosphate, organic nitrogen such as soybean hydrolysate, ammonia gas, aqueous ammonia and so forth can be used.

As the sulfur source, inorganic sulfur compounds, such as sulfates, sulfites, sulfides, hyposulfites and thiosulfates can be used.

As organic trace amount nutrients, it is desirable to add required substances such as vitamin B₁, yeast extract and so forth in appropriate amounts. Other than these, potassium phosphate, magnesium sulfate, iron ions, manganese ions and so forth are added in small amounts.

The culture is preferably performed under aerobic conditions for 30 to 90 hours. The culture temperature is preferably controlled to be at 25°C to 37°C, and pH is preferably controlled to be 5 to 8 during the culture. For pH adjustment, inorganic or organic acidic or alkaline substances, ammonia gas and so forth can be used. Collection of L-cysteine from the culture can be attained by, for example, any combination of known ion exchange resin methods, precipitation and other known methods.

L-Cysteine obtained as described above can be used for production of L-cysteine derivatives. The cysteine derivatives include methylcysteine, ethylcysteine, carbocysteine, sulfocysteine, acetylcysteine, and so forth.

Furthermore, when a thiazolidine derivative of L-cysteine is accumulated in the medium, L-cysteine can be produced by collecting the thiazolidine derivative from the medium to break the reaction equilibrium between the thiazolidine derivative and L-cysteine so that L-cysteine is excessively produced. Furthermore, when S-sulfocysteine is accumulated in the medium, it can be converted into L-cysteine by reduction with a reducing agent such as dithiothreitol.

L-Cysteine, a derivative thereof, and so forth collected in the present invention may contain cells of microorganism, medium components, moisture, and by-products of microbial metabolism in addition to the objective compound. Purity of the collected objective compound is 50% or higher, preferably 85% or higher, particularly preferably 95% or higher.

### Examples

Hereinafter, the present invention will be explained more specifically.

In the following descriptions, cysteine means L-cysteine.

### Example 1: Identification of protein having activity to take up cysteine or cystine

### (1) Acquisition of mutant strain unable to utilize S-sulfocysteine as sole cysteine source

### (1-1) Acquisition of cysE gene-deficient strain from E. coli MG1655 strain (ATCC No. 47076)

Tthe *cysE* gene was deleted by the method called "Red-driven integration" developed by Datsenko, Wanner et al. (Proc. Natl. Acad. Sci. USA, 2000, vol. 97, No. 12, pp.6640-6645) and the excisive system derived from λ phage (J. Bacteriol., 2000, 184, 5200-5203 (2002)). According to the Red-driven integration, a gene-disrupted strain can be constructed in one step by using a PCR product obtained with synthetic oligonucleotides designed so as to have a part of an objective gene on the 5' side, and a part of an antibiotic resistance gene on the 3' side. By further combining the excisive system derived from λ-phage, the antibiotic resistance gene incorporated into the gene-disrupted strain can be eliminated. Methods for deleting a gene of *E. coli* using this Red-driven integration and the excisive system derived from λ-phage are described in detail in Japanese Patent Laid-open No. 2005-058227, W02007/119880, and so forth. A *cysE* gene-deficient strain was also obtained in the same manner.

A DNA fragment containing an antibiotic resistance gene (kanamycin resistance gene (Km^{r})) between sequences homologous to the both ends of the *cysE* gene was obtained by PCR. Specific experimental procedure and experimental materials were the same as those described in Japanese Patent Laid-open No. 2005-058227, except that DcysE(Ec)-F (ccggcccgcg cagaacgggc cggtcattat ctcatcgtgt ggagtaagca tgaagcctgc ttttttatac taagttggca, SEQ ID NO: 50), and DcysE(Ec)-R (actgtaggcc ggatagatga ttacatcgca tccggcacga tcacaggaca cgctcaagtt agtataaaaa agctgaacga, SEQ ID NO: 51) were used as primers, and pMW118-(λattL-Km^{r}-λattR) (WO2006/093322A2) was used as a template. The obtained deficient strain was designated MG1655ΔcysE.

### (1-2) Preparation of transposon-mutated strain library from the MG1655ΔcysE strain

By using EZ-Tn5<KAN-2>Tnp Transposome Kit (EPICENTRE), a library of mutant strains in which Tn5 was randomly inserted was prepared from the MG1655AcysE strain. As for specific experimental procedure, the experiment was performed according to the instruction attached to the kit.

### (1-3) Screening of mutant strain library for mutant strain unable to utilize S-sulfocysteine as sole cysteine source

The aforementioned library was screened for a mutant strain unable to utilize S-sulfocysteine as a sole cysteine source. The "cysteine source" herein refers to a substrate which is taken up into cells and used for the production of cysteine. When cysteine cannot be synthesized in cells, cysteine itself is encompassed by the cysteine source. The mutant strains were each spotted with a toothpick on each of the M9 agar medium (Sambrook and Russell, Molecular Cloning: A Laboratory Manual (Third Edition), Cold Spring Harbor Laboratory Press) containing 20 µM cysteine and the M9 agar medium containing 20 µM S-sulfocysteine (cat# C2196, SIGMA) to perform screening for a mutant strain able to grow on a cysteine-containing medium, but unable to grow on an S-sulfocysteine-containing medium. One strain was obtained as the target mutant strain from about 1000 strains. When the genome region of inserted Tn5 was identified, it was found that Tn5 was inserted into the *ydjN* gene.

### (1-4) Analysis of S-sulfocysteine-assimilating ability of ydjN gene-deficient strain

In order to elucidate whether the phenotype of the mutant strain obtained by insertion of Tn5 was due to functional deficiency of the *ydjn* gene, a *ydjN* gene-deficient strain was constructed from the MG1655ΔcysE strain by the Red-driven integration described above. For this construction, primers DydjN(Ec)-F (cactatgact gctacgcagt gatagaaata ataagatcag gagaacgggg tgaagcctgc ttttttatac taagttggca, SEQ ID NO: 52), and DydjN(Ec)-R (aaagtaaggc aacggcccct atacaaaacg gaccgttgcc agcataagaa cgctcaagtt agtataaaaa agctgaacga, SEQ ID NO: 53) were used. The constructed deficient strain was designated MG1655ΔcysEΔydjN::Km strain. MG1655ΔydjN::Km strain was also obtained from MG1655 by the same method.

Growth of the strains on the M9 agar medium (provided that MgCl₂ was used instead of MgSO₄ at the same concentration as the medium component) containing 50 µM cysteine, 50 µM cystine or 50 µM S-sulfocysteine is shown in Table 1.

**Table 1**

| Strain | M9 (w/o sulfur) | M9+ cysteine | M9 + S-sulfocysteine | M9+cystine |
|---|---|---|---|---|
| MG1655 | + | + | + | + |
| MG1655ΔcysE | - | + | + | + |
| MG1655ΔydjN::Km | + | + | + | + |
| MG1655ΔcysEAydjN::Km | - | + | - | + |

Although any sulfur source was not added to the M9 agar medium in this experiment (M9 w/o sulfur), the *cysE*-non-disrupted strains (MG1655 strain, MG1655ΔydjN::Km strain etc.) can grow with a trace amount of contaminating sulfur compounds. Therefore, in order to investigate whether use of S-sulfocysteine as a cysteine source is possible or not, the background of the *cysE* deficiency should be determined. That is, the MG1655ΔcysE strain cannot grow without cysteine source (w/o sulfur), but it can grow by using cysteine, cystine or S-sulfocysteine as a cysteine source, if they exist. It was found that the *ydjN*-deficient strain could grow with cysteine or cystine as a cysteine source, but it could not grow with S-sulfocysteine as a cysteine source (Table 1, MG1655ΔcysEΔydjN::Km strain). From this result, it was found that *ydjN* was a gene indispensable to the assimilation of S-sulfocysteine. On the other hand, even if *ydjN* was deleted, cysteine and cystine could be assimilated.

### (2) Functional analysis of ydjN and fliY

### (2-1) Cloning of ydjN gene from E. coli MG 1655 strain and P. ananatis SC 17 strain

When the *ydjN* gene was cloned from the *E*. *coli* MG1655 strain and the *Pantoea ananatis* SC17 strain (U.S. Patent No. 6,596,517), expression vectors based on pMIV-Pnlp8 and pMIV-Pnip0 were used. The potent nlp8 promoter (or nlp0 promoter) and an rrnB terminator were integrated into these expression vectors, and by inserting a target gene between the promoter and the terminator, it can be functioned as an expression unit. "Pnlp0" indicates a promoter of a wild-type *nlpD* gene, and "Pnlp8" indicates a mutant promoter of the *nlpD* gene. The details of the construction of these expression vectors are described in Example 3 as construction of pMIV-Pnlp8-YeaS7 and pMIV-Pnlp0-YeaS3. In pMIV-Pnlp8-yeaS7 and pMIV-PnlpO-yeaS3, the *yeaS* gene is cloned between the nlp8 or nlp0 promoter and the rrnB terminator using the *Sal*I and *Xba*I sites. If the *Sal*I and *Xba*I sites are designed in primers beforehand, the *ydjN* gene can also be inserted into those vectors in the same manner as that for *yeaS.* That is, the expression plasmids to be constructed correspond to expression plasmids having structures of pMIV-Pnlp8-yeaS7 and pMIV-Pnlp0-yeaS3 mentioned later in which the *yeaS* gene is replaced by the *ydjN* gene.

The *ydjN* gene of *E*. *coli* was amplified by using the genomic DNA of the MG1655 strain as a template as well as *ydjN*(Ec)-SalIF W2 (acgcgtcgac atgaactttc cattaattgc gaacatcgtg gtg, SEQ ID NO: 54) and *ydjN*(Ec)-xbaIRV2 (ctagtctaga ttaatggtgt gccagttcgg cgtcg, SEQ ID NO: 55) as primers with a PCR cycle consisting of 94°C for 5 minutes, followed by 30 cycles of 98°C for 5 seconds, 55°C for 5 seconds and 72°C for 90 seconds, and final incubation at 4°C. In the case of *P. ananatis,* the *ydjN* gene was amplified by using the genomic DNA of the SC17 strain as a template as well as ydjN2(Pa)-SalIFW (acgcgtcgac atggatattc ctcttacgc, SEQ ID NO: 56) and ydjN2(Pa)-xbaIRV (tgctctagat tagctgtgct ctaattcac, SEQ ID NO: 57) as primers with a PCR cycle consisting of 94°C for 5 minutes, followed by 30 cycles of 98°C for 5 seconds, 55°C for 5 seconds and 72°C for 2 minutes, and final incubation at 4°C. *Sal*I and *Xba*I sites were designed at the both ends in all the primers. The amplified fragments were each integrated into the pMIV-PnIp0 vector, and the constructed plasmids were designated according to the origin of the gene (*E*. *coli* (Ec) or *P. ananatis* (Pa)) as pMIV-Pnlp0-ydjN(Ec) and pMIV-Pnlp0-ydjN(Pa), respectively. Furthermore, as an empty vector corresponding to these plasmids for control, pMIV-5JS (Japanese Patent Laid-open No. 2008-99668) was used.

### (2-2) Functional analysis of ydjN

When *ydjN* was deleted, the strains could not grow with S-sulfocysteine as a sole cysteine source. Therefore, it was suspected that the *ydjN* gene might code for a transporter (uptake factor) of S-sulfocysteine. Therefore, it was examined whether there was any difference in the ability to take up S-sulfocysteine between the *ydjN-*deficient strain of the MG1655 strain (MG1655ΔydjN::Km strain described above) and *ydjN*-enhanced strain of the MG1655 strain (MG1655 strain transformed with pMIV-Pnlp0-ydjN(Ec)). Similar investigation was also conducted for cystine and cysteine as related compounds similar to S-sulfocysteine.

The S-sulfocysteine uptake experiment was performed as follows. First, the MG1655ΔydjN::Km strain and a control strain therefor, MG1655 strain, as well as the MG1655/pMIV-Pnlp0-ydjN(Ec) strain and a control strain therefor, MG1655/pMIV-5JS strain, were cultured overnight in the LB liquid medium (3-ml test tube, 37°C, shaking culture). The cells were collected from the culture medium, washed twice with the M9 minimal medium containing 0.4% glucose, and then suspended in the M9 minimal medium containing 0.4% glucose at a density twice higher than that of the original culture medium. The cell suspension of each strain prepared as described above was inoculated in a volume of 40 µl to 4 ml of the M9 minimal medium containing 0.4% glucose, and culture was performed at 37°C with shaking by using an automatically OD measuring culture apparatus, BIO-PHOTORECORDER TN-1506 (ADVANTEC).

When OD reached around 0.3 (culture for about 5 hours), 20 µl of 100 mM S-sulfocysteine was added (final concentration: 0.5 mM), and the medium was sampled (0.2 ml of the culture medium was taken and mixed with 0.8 ml of 1N hydrochloric acid) over time for 2 hours after the addition of S-sulfocysteine (0 hour). Amino acid analysis of the sample of each time point was performed with an amino acid analyzer (L-8900, Hitachi), and S-sulfocysteine concentration in the medium was determined by comparison with a 0.4 mM standard sample similarly prepared with 1 N hydrochloric acid. In the culture, 25 mg/L of chloramphenicol was added to the medium for all the plasmid-harboring strains.

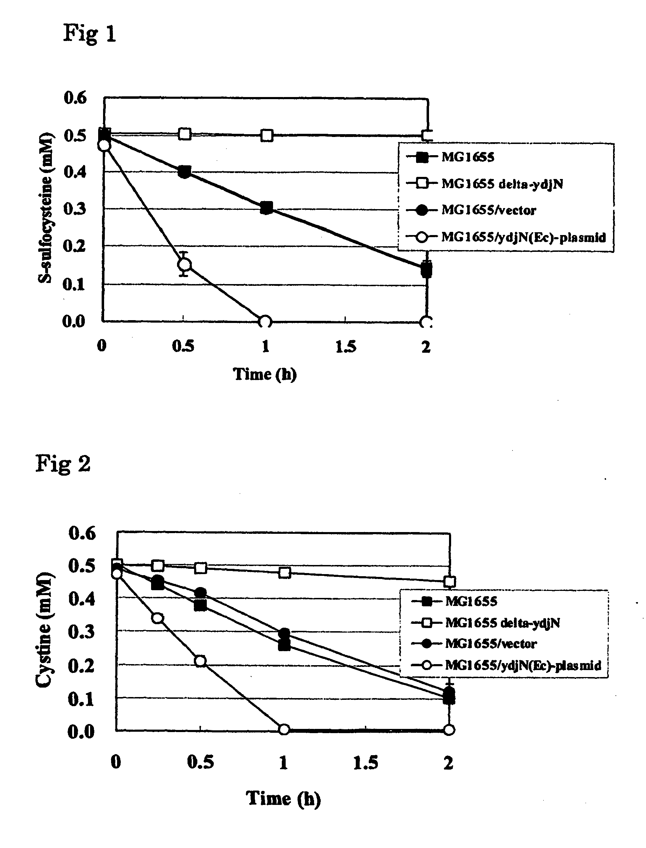

Change of S-sulfocysteine concentration in the culture medium of each strain is shown in Fig. 1. In the graph, the MG1655/pMIV-Pnlp0-ydjN(Ec), MG1655/pMIV-5JS strain, and MG1655ΔydjN::Km strains are abbreviated as MG1655/ydjN(Ec)-plasmid, MG1655/vector and MG1655 delta-ydjN, respectively. It was found that the S-sulfocysteine concentration in the medium gradually decreased with the wild-type strain, but S-sulfocysteine did not decrease at all with the *ydjN*-deficient strain, and decrease of S-sulfocysteine was accelerated with the *ydjN*-enhanced strain as compared with the control strain. Furthermore, when YdjN was analyzed with a membrane protein prediction program SOSUI (http://bp.nuap.nagoya-u.acjp/sosui/), ten transmembrane domains were found in YdjN, and therefore it was expected to be a membrane protein. The results described above strongly suggested a possibility that *ydjN* coded for a transporter (uptake factor) of S-sulfocysteine. Furthermore, since *ydjN* deficiency invited incapability of assimilating S-sulfocysteine (Table 1), *ydjN* is considered to be a sole S-sulfocysteine transporter in *E. coli.*

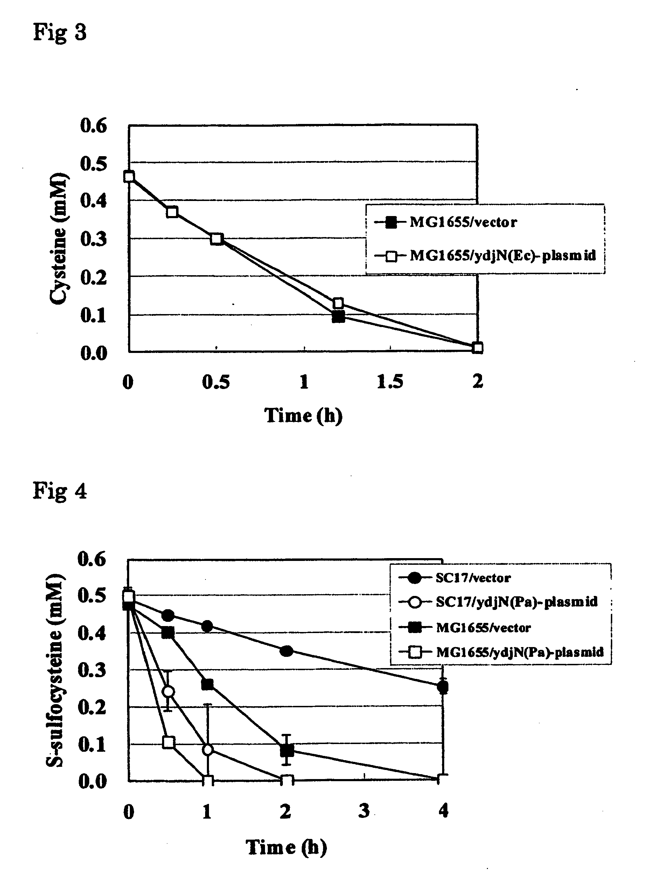

Furthermore, uptake of cystine or cysteine by YdjN was also examined by using the same experiment system and adding cystine or cysteine as a substrate instead of S-sulfocysteine. The results are shown in Figs. 2 and 3. The strain names in the graphs are the same as those used in Fig. 1.

As shown in Fig. 2, the same results as those for S-sulfocysteine were obtained when cystine was used as the substrate, and therefore it was suggested that YdjN had the activity to take up cystine. In the *ydjN-*deficient strain, uptake of cystine markedly decreased, and the residual activity to take up cystine became extremely weak. Therefore, it is considered that YdjN is a major transporter of cystine in the *E*. *coli* MG1655 strain. However, since the *ydjN*-deficient strain could also grow with cystine as the cysteine source (Table 1), it was considered that there was an active transporter of cysteine other than at least YdjN. On the other hand, when cysteine was used as the substrate, the uptake was not promoted even when *ydjN* was enhanced as shown in Fig. 3, and therefore it was suspected that it might not participate in the uptake of cysteine.

Furthermore, the plasmid pMIV-Pnlp0-ydjN(Pa) expressing *ydjN* derived from *P. ananatis* was introduced into *E. coli* and *P. ananatis* to enhance *ydjN,* and uptake of S-sulfocysteine under such enhancement of *ydjN* was examined. The results are shown in Fig. 4. In the graph, pMIV-Pnlp0-ydjN(Pa) and pMIV-5JS are abbreviated as ydjN(Pa)-plasmid and vector, respectively.

It was confirmed that YdjN of *P. ananalis* also had the activity to take up S-sulfocysteine, like YdjN of *E. coli.* Furthermore, the amino acid sequences of YdjN of *P. ananatis* and YdjN of *E*. *coli* show a homology of 80%.

### (2-3) Functional analysis of fliY

Then, in order to examine whether *fliY* participated in uptake of cystine and cysteine, *fliY*-deficient and enhanced strains were constructed first.

Deletion of the *fliY* gene was attained by using the aforementioned Red-driven integration and excisive system derived from λ-phage. DfliY(Ec)-FW (atgaaattag cacatctggg acgtcaggca ttgatgggtg tgatggccgt tgaagcctgc ttttttatac taagttggca, SEQ ID NO: 58) and DfliY(Ec)-RV (ttatttggtc acatcagcac caaaccattt ttcggaaagg gcttgcagag cgctcaagtt agtataaaa agctgaacga, SEQ ID NO: 59) were used as primers, and pMW 118-(λattL-Cm^{R}-λattR) (Katashkina ZhI et al., Mol. Biol. (Mosk), 39(5):823-31, 2005) was used as a template. MG1655AfliY strain was obtained from the MG1655 strain, and MG1655ΔydjN::KmΔfliY::Cm strain was obtained from the MG1655ΔydjN::Km strain described above.

Furthermore, in order to enhance the *fliY* gene using a plasmid, pMIV-Pnlp0-fliY(Ec) was constructed. The construction method was the same as the method of cloning the *ydjN* gene into pMIV-Pnlp0 mentioned above, but in this experiment, for amplification of the *fliY* gene, fliY(Ec)SalI-F (acgcgtcgac atgaaattag cacatctggg acg, SEQ ID NO: 60) and fliY(Ec)XbaI-R (ctagtctaga ttatttggtc acatcagcac c, SEQ ID NO: 61) were used as primers.

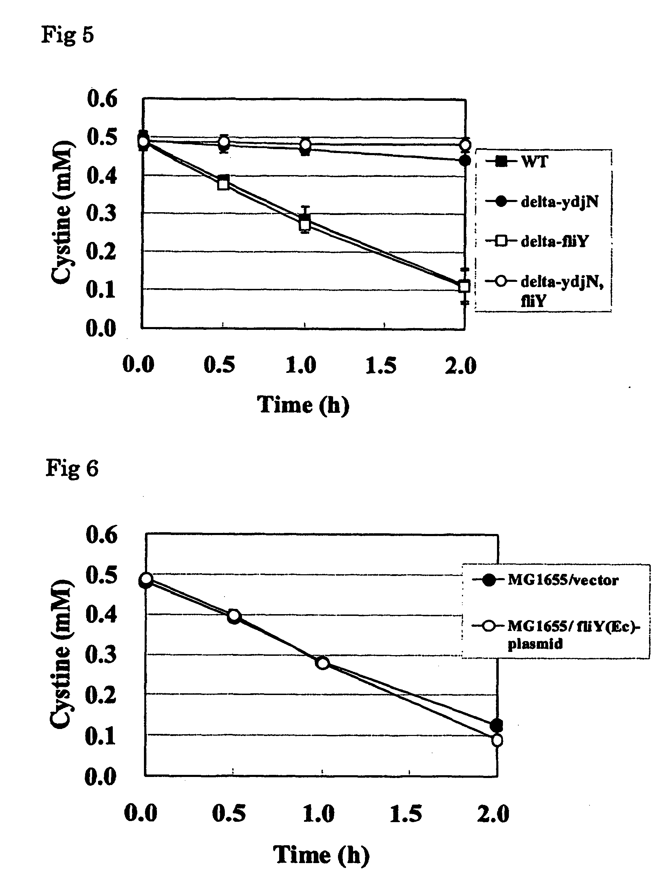

First, in order to investigate the effect of *fliY* deficiency on the cystine uptake, an uptake experiment was performed by using cystine as a substrate with 4 strains, the MG1655, MG1655ΔydjN::Km, MG1655ΔfliY, and MG1655ΔydjN::KmΔfliY::Cm strains. The results are shown in Fig. 5. In the graph, "WT" represents the MG 1655 strain, "delta-ydjN" represents the MG1655ΔydjN::Km strain, "delta fliY" represents the MG1655ΔfliY strain, and "delta-ydjN,fliY" represents the MG1655ΔydjN::KmΔfliY::Cm strain. As a result, the cystine uptake rate of the MG1655ΔfliY strain, in which only the *fliY* gene was deleted, was not different from that of the MG1655 strain (Fig. 5). Furthermore, although it was observed that the cystine uptake rate of the MG1655ΔydjNΔfliY strain, which was a *fliY* and *ydjN* gene-double deficient strain, seemed to decrease as compared with that of the MG1655ΔydjN strain, in which only *ydjN* was deficient, the difference was very small, and therefore it was unclear whether it was a significant difference induced by the *fliY* deficiency (Fig. 5).

Furthermore, uptake of cystine by the *fliY*-enhanced *E*. *coli* MG1655 strain was also investigated. The results are shown in Fig. 6. In the graph, "fliY(Ec)-plasmid" represents pMIV-Pnlp0-fliY(Ec), and "vector" represents pMIV-5JS. Also when *fliY* was enhanced, significant difference in uptake of cystine was not observed (Fig. 6).

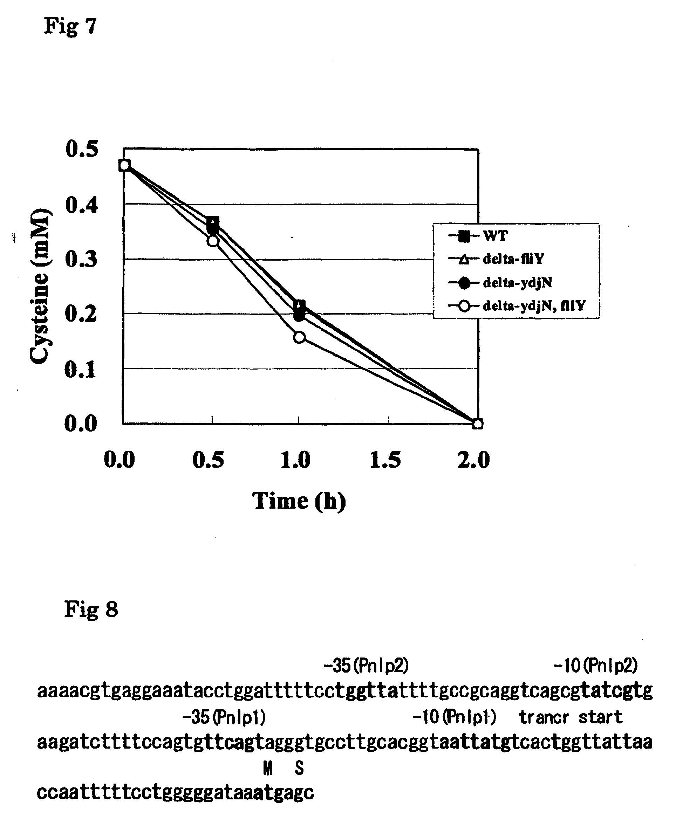

Although there were references suggesting a possibility of participation of *fliY* in uptake of cysteine (Butler et al., Life Sci., 52, 1209-1215 (1993) etc.), there were no findings directly proving it through experiments. In fact, results showing participation of FliY in uptake of cysteine were not obtained also in this experiment. Furthermore, from the results of this experiment, it was predicted that even if FliY participated in uptake of cystine, it was not a highly active transporter, like YdjN. In addition, since the *ydjN* and *fliY* double deficient strain could grow with cystine as a sole cysteine source, it is considered that a transporter of cystine exists besides these two kinds of transporters. Furthermore, although the cysteine uptake activity of the *fliY*-deficient strain was also examined, such a significant difference as seen for *ydjN* was not seen compared with the non-deficient strain, and it was considered that FliY did not participate in the uptake of cysteine (Fig. 7). On the other hand, since cysteine in the medium gradually decreased (Fig. 7), uptake of cysteine into cells was expected, and it is supposed that a certain cysteine transporter (uptake system) exists in *E*. *coli.*

### Example 2: Cysteine production by ydjN and/or fliY deficient E. coli

### (1) Construction of cysteine-producing E. coli strain

In order to impart cysteine-producing ability to a *ydjN* and/or *fliY*-deficient *E*. *coli* strain, a plasmid containing a mutant *cysE* coding for a mutant serine acetyltransferase for which feedback inhibition by L-cysteine was reduced (U.S. Patent Published Application No. 2005/0112731(A1)) was constructed. Specifically, a pACYC-DE1 plasmid was constructed according to the method for constructing pACYC-DES described in Japanese Patent Laid-open No. 2005-137369 (U.S. Patent Published Application No. 2005/0124049(A1), EP 1528108(A1)) provided that the step of incorporating a mutant *serA5* gene coding for a phosphoglycerate dehydrogenase desensitized to feedback inhibition by serine (described in U.S. Patent No. 6,180,373) was omitted. While pACYC-DES carried the aforementioned mutant *serA5,* the gene coding for the mutant SAT desensitized to the feedback inhibition, the *cysEX* gene, and the *ydeD* gene coding for the L-cysteine and acetylserine secretion factor (U.S. Patent No. 5,972,663), pACYC-DE1 constructed above did not contain *serA5* mentioned above, but contained *cysEX* and *ydeD.* For expression of all the genes, the ompA promoter was used.

Then, pACYC-DE1 was digested with *Mnu*I and self-ligated to construct a plasmid in which about 330 bp of the internal sequence of the *ydeD* gene ORF was deleted. This plasmid obtained as described above not expressing YdeD functioning as a cysteine secretion factor, but carrying only *cysEX* was designated pACYC-E1, and used for the following experiments. With this pACYC-E1, 5 kinds of strains, *E*. *coli* MG1655, MG1655ΔfliY, MG1655ΔfliY::Km, MG1655ΔydjN::Km, and MG1655ΔfliYΔydjN::Km, were transformed to impart cysteine-producing ability to each strain.

### (2) Investigation of effect of ydjN deficiency and fliY deficiency in E. coli on cysteine production

In order to investigate effect of *ydjN* deficiency and *fliY* deficiency on production of cysteine and cysteine-related compounds by fermentation, culture for fermentative production was performed with cysteine-producing bacteria obtained by introducing pACYC-E1 into the MG1655 strain, *ydjN* or *fliY*-deficient strains, *and ydjN* and *fliY*-double deficient strain derived from the MG1655 strain, and production amounts of cysteine and cysteine-related compounds were compared. For the culture, an *E. coli* cysteine production medium having the following composition was used.

### E. coli cysteine production medium (concentrations of the components are final concentrations):

| Component 1: | | |
|---|---|---|
| | (NH₄)₂SO₄ | 15 g/L |
| | KH₂PO₄ | 1.5 g/L |
| | MgSO₄-7H₂O | 1 g/L |
| | Tryptone | 10 g/L |
| | Yeast extract | 5 g/L |
| | NaCl | 10 g/L |
| | L-Histidine monohydrochloride | |
| | monohydrate | 135 mg/L |
| | L-Methionine | 300 mg/L |

| Component 2: | | |
|---|---|---|
| | Glucose | 40 g/L |

| Component 3: | | |
|---|---|---|
| | Sodium thiosulfate | 7 g/L |

| Component 4: | | |
|---|---|---|
| | Pyridoxine hydrochloride | 2 mg/L |

| Component 5: | | |
|---|---|---|
| | Calcium carbonate | 20 g/L |

For these components, 100/47.5-fold (Component 1), 100/47.5-fold (Component 2), 50-fold (Component 3), and 1000-fold (Component 4) concentration stock solutions were prepared, and mixed upon use, and the volume of the mixture was adjusted to a predetermined volume with sterilized water to obtain the final concentrations. Sterilization was performed by autoclaving at 110°C for 30 minutes (Components 1 and 2), hot air sterilization at 180°C for 5 hours or longer (Component 5), and filter sterilization (Components 3 and 4).

The cysteine production culture was performed according to the following procedures. The cysteine-producing strains were each applied and spread on the LB agar medium, and precultured overnight at 37°C. Then, the cells corresponding to about 7 cm on the plate were scraped twice with an inoculating loop of 10 µl size (Blue Loop, NUNC), and inoculated into 2 ml of the aforementioned *E*. *coli* cysteine production medium contained in a large test tube (internal diameter: 23 mm, length: 20 cm). The amounts of inoculated cells were adjusted so that the cell amounts at the time of the start of the culture should be substantially the same. The culture was performed at 32°C with shaking, and terminated after 40 hours. The cysteine accumulated in the medium was quantified by the method described by Gaitonde, M.K. (Biochem. J., 1967 Aug., 104(2):627-33). Cysteine quantified above contained cystine, derivatives thereof such as S-sulfocysteine, thiazolidine derivatives and hemithioketals, or a mixture of them, in addition to cysteine, and the same shall apply to cysteine quantified below as described above, unless especially specified. Cysteine and other compounds quantified as described above may be described as L-cysteine related compounds. The experiment was performed in hexaplicate for each of the strains, and averages and standard deviations of the results of them are shown in Table 2.

**Table 2**

| Strain | Genotype | Cysteine related compounds (g/L) |
|---|---|---|
| MG1655/pACYC-E1 | Wild-type | 0.056 ± 0.0055 |
| MG1655ΔfliY/pACYC-E1 | ΔfliY | 0.062 ± 0.0214 |
| MG1655ΔfliY::Km/pACYC-E1 | ΔfliY(::Km^{R}) | 0.082 ± 0.0172 |
| MG1655ΔydjN::Km/pACYC-E1 | ΔydjN(::KM^{R}) | 0.124 ± 0.0113 |
| MG1655ΔfliY DydjN::Km/pACYC-E1 | ΔfliY ΔydjN(::Km^{R}) | 0.273 ± 0.0381 |

As shown in Table 2, it was found that both the *fliY* deficiency *and ydjN* deficiency were effective for increasing accumulation of the cysteine related compounds. Moreover, it was found that the double deficiency of *fliY* and *ydjN* exerted a synergistic effect of markedly increasing the cysteine related compounds compared with deficiency of each gene alone.

### Example 3: Production of cysteine by P. ananatis deficient in ydjN and/or fliY

### (1) Preparation of cysteine-producing P. ananatis EYPS1976(s) strain

A cysteine-producing bacterium of *P. ananatis* was constructed by introducing *cysE5* coding for a mutant serine acetyltransferase (U.S. Patent Published Application No. No. 2005/0112731), and *serA348* coding for a mutant 3-phosphoglycerate dehydrogenase (J. Biol. Chem., 1996, 271 (38):23235-8), and enhancing *yeaS* coding for a secretion factor for various amino acids (Japanese Patent Laid-open No. 2000-189180) and the *cysPTWA* cluster coding for a sulfur source uptake factor. The details of the construction method are described below.

### (1-1) Introduction of CysES and YeaS into P. ananatis SC17 strain

First, a plasmid for constructing the aforementioned strain was constructed. The method for it is described below.

By PCR using the chromosomal DNA of *E*. *coli* MG1655 (ATCC No. 47076) as a template as well as P1 (agctgagtcg acccccagga aaaattggtt aataac, SEQ ID NO: 30) and P2 (agctgagcat gcttccaact gcgctaatga cgc, SEQ ID NO: 31) as primers, a DNA fragment containing a promoter region of the *nlpD* gene (Pnlp0) of about 300 bp was obtained. At the 5' and 3' ends of the aforementioned primers, sites for the restriction enzymes *Sal*I and *Pae*I were designed, respectively. The PCR cycle was as follows: 95°C for 3 minutes, then 2 cycles of 95°C for 60 seconds, 50°C for 30 seconds, and 72°C for 40 seconds, 25 cycles of 94°C for 20 seconds, 55°C for 20 seconds, and 72°C for 15 seconds, and 72°C for 5 minutes as the final cycle. The obtained fragment was treated with *Sal*I and *Pae*I, and inserted into pMIV-5JS (Japanese Patent Laid-open No. 2008-99668) at the *Sal*I-*Pae*I site to obtain a plasmid pMIV-Pnlp0. The nucleotide sequence of the *Pae*I-*Sal*I fragment of the Pnlp0 promoter inserted into this pMIV-Pnlp0 plasmid was as shown in SEQ ID NO: 27.

Then, by PCR using the chromosomal DNA of MG1655 as a template, as well as P3 (agctgatcta gaaaacagaa tttgcctggc ggc, SEQ ID NO: 32) and P4 (agctgaggat ccaggaagag tttgtagaaa cgc, SEQ ID NO: 33) as primers, a DNA fragment containing a terminator region of the *rrnB* gene of about 300 bp was obtained. At the 5' ends of the aforementioned primers, sites for the restriction enzymes *Xba*I and *Bam*HI were designed, respectively. The PCR cycle was as follows: 95°C for 3 minutes, then 2 cycles of 95°C for 60 seconds, 50°C for 30 seconds, and 72°C for 40 seconds, 25 cycles of 94°C for 20 seconds, 59°C for 20 seconds, and 72°C for 15 seconds, and 72°C for 5 minutes as the final cycle. The obtained fragment was treated with *XbaI* and *Bam*HI*,* and inserted into pMIV-Pnlp0 at the *Xba*I*-Bam*HI site to obtain a plasmid pMIV-Pnlp0-ter.

Then, by PCR using the chromosomal DNA of the MG1655 strain as a template, as well as P5 (agctgagtcg acgtgttcgc tgaatacggg gt, SEQ ID NO: 34) and P6 (agctgatcta gagaaagcat caggattgca gc, SEQ ID NO: 35) as primers, a DNA fragment of about 700 bp containing the *yeaS* gene was obtained. At the 5' ends of the aforementioned primers, sites for the restriction enzymes *Sal*I and *Xba*I were designed, respectively. The PCR cycle was as follows: 95°C for 3 minutes, then 2 cycles of 95°C for 60 seconds, 50°C for 30 seconds, and 72°C for 40 seconds, 25 cycles of 94°C for 20 seconds, 55°C for 20 seconds, and 72°C for 15 seconds, and 72°C for 5 minutes as the final cycle. The obtained fragment was treated with *Sal*I and *Xba*I, and inserted into pMIV-Pnlp0-ter at the *Sal*I-*Xba*I site to obtain a plasmid pMIV-Pnlp0-YeaS3. As described above, *a yeaS* expression unit comprising the pMIV-5JS vector on which the nlpD promoter, the *yeaS* gene, and the *rrnB* terminator were ligated in this order was constructed.

In order to modify the -10 region of the nlpD promoter to make it a stronger promoter, the -10 region was randomized by the following method. The nlpD promoter region contains two of regions presumed to function as promoters (Fig. 8), and they are indicated as pnlp1 and pnlp2, respectively, in the drawing. By PCR using the plasmid pMIV-Pnlp0 as a template as well as P1 and P7 (atcgtgaaga tcttttccag tgttnannag ggtgccttgc acggtnatna ngtcactgg ("n" means that the corresponding residue can be any of a, t, g and c), SEQ ID NO: 36) as primers, a DNA fragment in which the - 10 region contained in the 3' end sequence of the nlpD promoter (referred to as - 10(Pnlp1)) was randomized was obtained. The PCR cycle was as follows: 95°C for 3 minutes, then 2 cycles of 95°C for 60 seconds, 50°C for 30 seconds, and 72°C for 40 seconds, 25 cycles of 94°C for 20 seconds, 60°C for 20 seconds, and 72°C for 15 seconds, and 72°C for 5 minutes as the final cycle.

Furthermore, by PCR using the plasmid pMIV-Pnlp0 as a template as well as P2 and P8 (tggaaaagat cttcannnnn cgctgacctg cg ("n" means that the corresponding residue can be any of a, t, g and c), SEQ ID NO: 37) as primers, a DNA fragment in which the -10 region contained in the 5' end sequence of the nlpD promoter (referred to as -10(Pnlp2)) was randomized was similarly obtained (Fig. 1). The PCR cycle was as follows: 95°C for 3 minutes, then 2 cycles of 95°C for 60 seconds, 50°C for 30 seconds, and 72°C for 40 seconds, 25 cycles of 94°C for 20 seconds, 60°C for 20 seconds, and 72°C for 15 seconds, and 72°C for 5 minutes as the final cycle.

The obtained 3' and 5' end fragments could be ligated using the *Bgl*II sites designed in the primers P7 and P8, and the full length of the nlpD promoter in which two -10 regions were randomized could be constructed by such ligation. By PCR using this fragment as a template as well as P1 and P2 as primers, a DNA fragment corresponding to a modified type nlpD promoter of the full length was obtained. The PCR cycle was as follows: 95°C for 3 minutes, then 2 cycles of 95°C for 60 seconds, 50°C for 30 seconds, and 72°C for 40 seconds, 12 cycles of 94°C for 20 seconds, 60°C for 20 seconds, and 72°C for 15 seconds, and 72°C for 5 minutes as the final cycle.

The amplified fragment was treated with the restriction enzymes *Sal*I and *Pae*I, for which sites were designed in the 5' ends of the primers, and inserted into the plasmid pMIV-Pnlp0-YeaS3 similarly treated with *Sal*I and *Pae*I to substitute the mutant Pnlp for the wild-type nlpD promoter region (Pnlp0) on the plasmid. From such plasmids, one having the promoter sequence (Pnlp8) shown in SEQ ID NO: 28 was selected, and designated pMIV-Pnlp8-YeaS7 (the nucleotide sequence of the *Pae*I-*Sal*I fragment of the Pnlp8 promoter inserted into this plasmid was as shown in SEQ ID NO: 28). In the same manner, a DNA fragment of the nlpD promoter region containing a mutation was inserted into the plasmid pMIV-Pnlp0-ter treated with *Sal*I and *Pae*I to substitute the mutant Pnlp for the nlpD promoter region (region of Pnlp0) on the plasmid. One of them was designated pMIV-Pnlp23-ter. The nucleotide sequence of the *Pae*I-*Sal*I fragment of the Pnlp23 promoter inserted into this plasmid was as shown in SEQ ID NO: 29.

Then, from pMW-Pomp-cysE5 (W02005/00784 1), the Pomp-cysE5 cassette portion was excised with *Pae*I and *Sac*I, and inserted into the same site of pMIV-5JS to construct pMIV-Pomp-CysE5. pMW-Pomp-cysE5 was a plasmid obtained by inserting the *cysE5* gene coding for the mutant SAT ligated with the *ompC* gene promoter into pMW118. From pACYC184 (GenBank/EMBL accession number X06403, available from NIPPON GENE), the tetracycline resistence gene was excised with *Xba*I and *Eco*88I, and this gene fragment was treated with the Klenow fragment, and then inserted into pMIV-Pomp-CysE5 at the PvuI site to construct pMT-Pomp-CysE5. Then, pMIV-Pnlp8-YeaS7 was digested with *Hind*III, blunt-ended with the Klenow fragment, and then digested with *Nco*I to excise a fragment containing the cassette of the Pnlp8-YeaS-rrnB terminator and the chloramphenicol resistance marker. This fragment was ligated with a *Sma*I and *Nco*I digestion fragment of pMT-Pomp-CysE5 similarly having pMIV-5JS as the backbone to construct pMT-EY2. pMT-EY2 is a plasmid having the Pnlp8- YeaS-*rrnB* terminator cassette and the Pomp-CysE5 cassette on one plasmid.

pMT-EY2 described above has the attachment sites of Mu phage originated from pMIV-5JS (Japanese Patent Laid-open No. 2008-99668). By allowing this plasmid to coexist with the helper plasmid pMH10 having Mu transposase (Zimenkov D. et al., Biotechnologiya and (in Russian), 6, 1-22 (2004)) in the same cell, the cassette of PompC-cysE5-Pnlp8-YeaS-rrnB terminator including the chloramphenicol resistance marker located between the attachment sites of Mu phage on this pMT-EY2 plasmid can be inserted into the chromosome of the *P. ananatis* SC17 strain (U.S. Patent No. 6,596,517). Furthermore, since the chloramphenicol resistance marker located on the pMT-EY2 plasmid exists between two attachment sites of λ phage (λattR and λattL), the chloramphenicol resistance marker can be excised and removed by the method described later.

First, an SC 17 strain introduced with pMH 10 by electroporation was selected by overnight culture at 30°C on the LB agar medium containing 20 mg/L of kanamycin. The obtained transformant was cultured at 30°C, and pMT-EY2 was further introduced into this strain by electroporation. This strain transformed with both pMH10 and pMT-EY2 was given a heat shock with the conditions of 42°C for 20 minutes, and colonies of chloramphenicol resistant strains were selected on the LB agar medium containing 20 mg/L of chloramphenicol. The culture temperature for this selection was 39°C. As described above, about 50 clones were obtained, and curing of pMH 10 and pMT-EY2 was performed by culturing each clone at 39°C for 48 hours on the LB agar medium. A strain showing chloramphenicol resistance due to the insertion of the cassette on the chromosome and showing kanamycin and ampicillin sensitivities due to the curing of both the plasmids was obtained. Furthermore, it was confirmed that the objective cassette was inserted in the chromosome of the obtained strain by PCR using the chromosomal DNA of this strain as a template as well as P1 and P6 as primers. All the obtained clones were designated EY01 to EY50, respectively, and L-cysteine production culture was performed by using the EY01 to EY50 strains as described below. The EY19 strain was selected, which was a clone that produced L-cysteine in the largest amount as a result of the culture.

An L-cysteine production medium (composition: 15 g/L of ammonium sulfate, 1.5 g/L of potassium dihydrogenphosphate, 1 g/L of magnesium sulfate heptahydrate, 0.1 g/L of tryptone, 0.05 g/L of yeast extract, 0.1 g/L sodium chloride, 20 g/L of calcium carbonate, 40 g/L of glucose, and 20 mg/L of tetracycline) was used for the culture.

The L-cysteine production culture was performed by the following procedure. The SC17/pMT-PompCysE5 strain and SC17/pMT-EY2 strain were each applied on the LB agar medium to perform preculture overnight at 34°C, then cells corresponding to 1/8 of the plate were scraped with an inoculation loop, inoculated into 2 ml of the L-cysteine production medium contained in a large test tube (internal diameter: 23 mm, length: 20 cm), and cultured at 32°C with shaking at 220 to 230 rpm, and the culture was terminated after two days.

The chloramphenicol resistance marker introduced into the EY19 strain was removed with an excision system derived from λ phage. Specifically, the EY 19 strain was transformed with pMT-Int-Xis2 (WO2005/010175) carrying the *Int-Xis* gene of λ phage, and an EY19(s) strain showing chloramphenicol sensitivity was obtained from the obtained transformants. Examples of removal of a marker using the excision system derived from λ phage are described in detail in Japanese Patent Laid-open No. 2005-058227, WO2007/119880 and so forth.

### (1-2) Preparation of cysPTWA gene expression-enhanced strain from EY19(s) strain

Then, in order to enhance expression of the *cysPTWA* gene, the promoter located upstream of the *cysPTWA* gene cluster on the chromosome was replaced with the aforementioned potent promoter Pnlp8. A DNA fragment containing the nlp8 promoter of about 300 bp was obtained by PCR using pMIV-Pn1p8-YeaS7 as a template as well as P1 and P2 as primers. The PCR cycle was as follows: 95°C for 3 minutes, then 2 cycles of 95°C for 60 seconds, 50°C for 30 seconds, and 72°C for 40 seconds, 20 cycles of 94°C for 20 seconds, 59°C for 20 seconds, and 72°C for 15 seconds, and 72°C for 5 minutes as the final cycle.

The amplified DNA fragment containing nlp8 promoter was treated with the Klenow fragment, inserted into the plasmid pMW118-(λattL-KmR-λattR) (WO2006/093322A2) digested with *Xba*I and then treated with the Klenow fragment to obtain a plasmid pMW-Km-Pnlp8. By PCR using pMW-Km-Pnlp8 as a template as well as P9 (tccgctcacg atttttttca tcgctggtaa ggtcatttat cccccaggaa aaattggtta, SEQ ID NO: 38) and P10 (tttcacaccg ctcaaccgca gggcataacc ggcccttgaa gcctgctttt ttatactaag ttg, SEQ ID NO: 39) as primers, a DNA fragment of about 1.6 kb containing the Km-Pnlp8 cassette was amplified. The PCR cycle for this amplification was as follows: 95°C for 3 minutes, then 2 cycles of 95°C for 60 seconds, 50°C for 30 seconds, and 72°C for 40 seconds, 30 cycles of 94°C for 20 seconds, 54°C for 20 seconds, and 72°C for 90 seconds, and 72°C for 5 minutes as the final cycle. On both the primers, a sequence serving as a target on the chromosome for inserting an objective fragment by λ-dependent integration (the method called "Red-driven integration" (Proc. Natl. Acad. Sci. USA, 2000, vol. 97, No. 12, pp.6640-6645)) (in this case, a sequence near the promoter of *cysPTWA*) was designed. Therefore, if the obtained DNA fragment is inserted into the objective strain by this λ-dependent integration, there is provided a structure that Km-Pnlp8 is inserted immediately before the *cysPTWA* gene on the chromosome, and the *cysPTWA* gene is ligated with the nlp8 promoter. The nucleotide sequence of the *cysPTWA* gene cluster is shown in SEQ ID NO: 19, and the amino acid sequences encoded by the *cysP, cysT* and *cysW* genes are shown in SEQ ID NOS: 20 to 22, respectively. The nucleotide sequence of the *cysA* gene and the amino acid sequence encoded by this gene are shown in SEQ ID NOS: 23 and 24, respectively.

The *P. ananalis* SC 17(0)/RSF-Red-TER strain is a host strain for efficiently performing the λ-dependent integration, and it is a strain obtained by introducing the helper plasmid RSF-Red-TER which expresses *gam, bet* and exo genes of λ (henceforth referred to as "λ Red genes") into the SC 17(0) strain, which is a λ Red gene product-resistant *P. ananalis* strain (W02008/075483). A method for constructing the RSF-Red-TER plasmid is disclosed in detail in WO2008/075483.

The aforementioned SC17(0)/RSF-Red-TER strain was cultured with addition of IPTG for inducing expression of λ Red genes to prepare cells for electroporation. The aforementioned objective DNA fragment was introduced into these cells by electroporation, and a recombinant strain in which the nlp8 promoter was inserted upstream of the *cysPTWA* gene by λ-dependent integration was obtained by using the kanamycin resistance as a marker. By PCR using the chromosomal DNA of the obtained strain as a template, as well as P11 (ctttgtccct ttagtgaagg, SEQ ID NO: 40) and P12 (agctgatcta gaagctgact cgagttaatg gcctcccaga cgac, SEQ ID NO: 41) as primers, it was confirmed that the objective structure, Km-Pnlp8-*cysPTWA,* was formed, and this strain was designated SC17(0)-Pnlp8-PTWA strain.

Then, the chromosomal DNA of the SC17(0)-Pnlp8-PTWA strain was purified, and 10 µg of this chromosomal DNA was introduced into the EY19(s) strain by electroporation to obtain a kanamycin resistant strain. Amplification was performed by PCR using the chromosomal DNA of the obtained strain as a template as well as P11 and P12 as primers to confirm that the structure of Km-Pnlp8-cysPTWA had been introduced into the chromosome of the EY19(s) strain. The strain obtained as described above was designated EYP197 strain. Furthermore, the kanamycin resistance marker was removed from the chromosome by using pMT-Int-Xis2 as described above, and the strain that became kanamycin sensitive was designated EYP197(s) strain.

### (1-3) Preparation of mutant 3-phosphoglycerate dehydrogenase (serA348) gene-carrying strain of EYP197(s) strain

As a gene of 3-phosphoglycerate dehydrogenase to be introduced into the L-cysteine-producing bacterium, the *serA348* gene which is a gene coding for 3-phosphoglycerate dehydrogenase of *Pantoea ananalis* and coding for a mutant enzyme including a mutation for substitution of an alanine residue for the asparagine residue at the 348th position (N348A) (J. Biol. Chem., 1996, 271 (38):23235-8) was constructed by the following method.

The sequence of the wild-type *serA* gene derived from *Pantoea ananatis* and the amino acid sequence are shown in SEQ ID NOS: 17 and 18, respectively. In order to obtain a 3'-end side DNA fragment of the *serA* gene into which the aforementioned mutation was introduced, PCR was performed by using the chromosomal DNA of the SC17 strain as a template as well as P13 (agctgagtcg acatggcaaa ggtatcactg gaa, SEQ ID NO: 42) and P14 (gagaacgccc gggcgggctt cgtgaatatg cagc, SEQ ID NO: 43) as primers (95°C for 3 minutes, then 2 cycles of 95°C for 60 seconds, 50°C for 30 seconds, and 72°C for 40 seconds, 25 cycles of 94°C for 20 seconds, 60°C for 20 seconds, and 72°C for 60 seconds, and 72°C for 5 minutes as the final cycle). Then, in order to obtain a 5'-end side DNA fragment into which the mutation was introduced, PCR was performed in the same manner by using the chromosomal DNA of the SC17 strain as a template as well as P15 (agctgatcta gacgtgggat cagtaaagca gg, SEQ ID NO: 44) and P16 (aaaaccgccc gggcgttctc ac, SEQ ID NO: 45) as primers (95°C for 3 minutes, then 2 cycles of 95°C for 60 seconds, 50°C for 30 seconds, and 72°C for 40 seconds, 20 cycles of 94°C for 20 seconds, 60°C for 20 seconds, and 72°C for 20 seconds, and 72°C for 5 minutes as the final cycle). Both the obtained PCR fragments were treated with the restriction enzyme SmaI, and ligated by using a DNA ligase to obtain a DNA fragment corresponding to a full length mutant *serA* gene including the objective mutation (N348A). This DNA fragment was amplified by PCR using it as a template as well as P13 and P15 as primers (95°C for 3 minutes, then 2 cycles of 95°C for 60 seconds, 50°C for 30 seconds, and 72°C for 40 seconds, 1 cycles of 94°C for 20 seconds, 60°C for 20 seconds, and 72°C for 75 seconds, and 72°C for 5 minutes as the final cycle). The *Sal*I and the *Xba*I restriction enzyme sites designed in the P13 and P15 primers were treated with *Sal*I and *Xba*I, and the fragment was inserted into pMIV-Pnlp8-ter similarly treated with *Sal*I and *Xba*I to prepare pMIV-Pnlp8-serA348.

The constructed pMIV-Pn1p8-serA348 carried the attachment site of Mu originating in pMIV-5JS (Japanese Patent Laid-open No. 2008-99668). By using this plasmid together with the helper plasmid pMH10 having Mu transposase, the cassette of Pnlp8-serA348-rrnB terminator including the chloramphenicol resistance marker can be inserted into the chromosome of the *P. ananatis* SC 17 strain, as described above. The pMIV-Pnlp8-serA348 plasmid and pMH10 were introduced into the SC17(0) strain to obtain a strain in which the cassette of PnlpB-serA348-rrnB terminator was inserted into the chromosome. By PCR using the primers P1 and P15, it was confirmed that the objective cassette existed in the cells. The 3-phosphoglycerate dehydrogenase activity in cell extracts of about 50 of the obtained clones was measured, and a strain which showed the highest activity was selected, and designated SC17int-serA348 strain. Then, 10 µg of the chromosomal DNA of the SC17int-serA348 strain was introduced into the EYP197(s) strain by electroporation to obtain a chloramphenicol resistant strain, and by PCR using the primers P1 and P15, it was confirmed that the structure of Pn1p8-serA348 had been introduced together with the chloramphenicol resistance marker into the chromosome of the EYP197(s) strain. The strain obtained as described above was designated EYPS1976 strain. By the aforementioned method for removing marker using pMT-Int-Xis2, the chloramphenicol resistance marker was removed, and the strain that became chloramphenicol sensitive was designated EYPS1976(s) strain.

### (2) Construction of ydjN and/or fliY-deficient cysteine-producing bacteria

Strains deficient in *ydjN* and/or *fliY* were constructed from the EYPS 1976(s) strain. A *ydjN* gene-deficient strain and a *fliY* region-deficient strain were prepared by λ-dependent integration using the *aforementioned P. ananatis* SC17(0)/RSF-Red-TER strain as a host bacterium.

The *yecS* gene (nucleotide sequence: SEQ ID NO: 11, amino acid sequence: SEQ ID NO: 12) and the *yecC* gene (nucleotide sequence: SEQ ID NO: 13, amino acid sequence: SEQ ID NO: 14) are present downstream from the *fliY* gene, and these may possibly form an operon and function as an ABC transporter (information that *yecS* and *yecC* may form one transcription unit is mentioned in a database open to public on an internet website (http://ecocyc.org/)). Therefore, for the *fliY* deficiency, it was decided to delete all three of these genes (*fliY* flanking regions).

To obtain a DNA fragment for a *ydjN* gene-deficient strain, a primer DydjN(Pa)-F (acctctgctg ctctcctgac cagggaatgc tgcattacat cggagttgct tgaagcctgc ttttttatac taagttggca, SEQ ID NO: 46) and a primer DydjN(Pa)-R (agacaaaaac agagagaaag acctggcggt gtacgccagg tctggcgtga cgctcaagtt agtataaaaa agctgaacga, SEQ ID NO: 47) were used, and to obtain a DNA fragment for a *fliY* region-deficient strain, a primer DfliY-FW (atggctttct cacagattcg tcgccaggtg gtgacgggaa tgatggcggt tgaagcctgc ttttttatac taagttggca, SEQ ID NO: 48) and a primer DyecC-RV (ttacgccgcc aacttctggc ggcaccgggt ttattgatta agaaatttat cgctcaagtt agtataaaaa agctgaacga, SEQ ID NO: 49) were used. As a template, pMW118-(λattL-Km^{r}-λattR) (WO2006/093322A2) mentioned above was used, and PCR was performed at 94°C for 5 minutes, followed by 30 cycles of 94°C for 30 seconds, 55°C for 30 seconds and 72°C for 2 minutes and 30 seconds to obtain a DNA fragment comprising Km^{r} between the homologous sequences each used for the recombination.

Each of the obtained DNA fragments was introduced into *P. ananatis* SC17(0)/RSF-Red-TER strain by electroporation to construct SC17(0)ΔydjN::Km strain (the λattL-Kmr-λattR fragment was inserted into the *ydjN* gene region) and SC17(0)ΔfliY::Km strain (the λattL-Kmr-λattR fragment was inserted into the *fliY* gene region). Then, the EYPS 1976(s) strain was transformed with the chromosomal DNAs prepared from the SC17(0)ΔydjN::Km strain and the SC17(0)ΔfliY::Km strain to obtain strains deficient in each gene, EYPSΔydjN::Km strain and EYPSΔfliY::Km strain, from the EYPS1976(s) strain. Furthermore, pMT-Int-Xis2 (WO2005/010175) mentioned above was introduced into the EYPSΔfliY::Km strain, and the kanamycin resistance gene was excised by using the excisive system derived from λ phage to obtain kanamycin-sensitive EYPSΔfliY strain. Then, the EYPSΔfliY strain was transformed with chromosomal DNA prepared the from the SC17(0)ΔydjN::Km strain to obtain a double deficient strain, EYPSΔfliYAydjN::Km strain. In such a manner, the *ydjN* gene-deficient strain, the *fliY* region-deficient strains, and the double deficient strain for these genes based on the cysteine-producing bacterium, EYPS 1976(s) strain, were constructed.

### (3) Investigation of effect of ydjN deficiency and fliY deficiency in P. ananatis on cysteine production

Culture for fermentative production was performed with the *ydjN* gene-deficient strain, the *fliY* region-deficient strains, and the double deficient strain for these genes obtained above, and production amounts of the cysteine related compounds were compared. For the culture, a *P. ananatis* cysteine production medium having the following composition was used.

### P. ananatis cysteine production medium (concentration of the components are final concentrations):

| Component 1: | | |
|---|---|---|
| | (NF₄)₂SO₄ | 15 g/L |
| | KH₂PO₄ | 1.5 g/L |
| | MgSO₄-7H₂O | 1 g/L |
| | Thiamine hydrochloride | 0.1 mg/L |

| Component 2: | | |
|---|---|---|
| | FeSO₄·7H₂O | 1.7 mg/L |
| | Na₂MoO₄·2H₂O | 0.15 mg/L |
| | CoCl₂·6H₂O | 0.7 mg/L |
| | MnCl·4H₂O | 1.6 mg/L |
| | ZnSO₄·H₂O | 0.3 mg/L |
| | CuSO₄·5H₂O | 0.25 mg/L |

| Component 3: | | |
|---|---|---|
| | Tryptone | 0.6 g/L |
| | Yeast extract | 0.3 g/L |
| | NaCl | 0.6 g/L |

| Component 4: | | |
|---|---|---|
| | Calcium carbonate | 20 g/L |

| Component 5: | | |
|---|---|---|
| | L-Histidine monohydrochloride | |
| | monohydrate | 135 mg/L |

| Component 6: | | |
|---|---|---|
| | Sodium thiosulfate | 6 g/L |

| Component 7: | | |
|---|---|---|
| | Pyridoxine hydrochloride 2 mg/L | |

| Component 8: | | |
|---|---|---|
| | Glucose | 40 g/L |

For these components, 10-fold (Component 1), 1000-fold (Component 2), 100/6-fold (Component 3), 100-fold (Component 5), 350/6-fold (Component 6), 1000-fold (Component 7) and 10-fold (Component 8) concentration stock solutions were prepared, and mixed upon use, and the volume of the mixture was adjusted to a predetermined volume with sterilized water to obtain the final concentrations. Sterilization was performed by autoclaving at 110°C for 30 minutes (Components 1, 2, 3, 5 and 8), hot air sterilization at 180°C for 5 hours or longer (Component 4), and filter sterilization (Components 6 and 7).

The cysteine production culture was performed according to the following procedures. The cysteine-producing strains were each applied and spread on the LB agar medium, and precultured overnight at 34°C. Then, the cells corresponding to about 7 cm on the plate were scraped twice with an inoculating loop of 10 µl size (Blue Loop, NUNC), and inoculated into 2 ml of the aforementioned *P. ananatis* cysteine production medium contained in a large test tube (internal diameter: 23 mm, length: 20 cm). The amounts of inoculated cells were adjusted so that the cell amounts at the time of the start of the culture should be substantially the same.

The culture was performed at 32°C with shaking, and terminated after 43 hours. At this time, complete consumption of glucose in the medium was confirmed. The quantification of cysteine accumulated in the medium was performed by the method described by Gaitonde, M.K. (Biochem. J., 1967 Aug., 104(2):627-33). The experiment was performed in hexaplicate for each of the strains, and averages and standard deviations of the results are shown in Table 3. For the strain deficient in only *fliY*, increase of the cysteine related compounds was not observed, and for the strain deficient in only *ydjN,* the cysteine related compounds slightly increased. Furthermore, for the double deficient strain for *fliY* and *ydjN,* increase of the cysteine related compounds was observed in a much higher degree compared with that obtainable with deficiency of one of the genes. It was found that *ydjN* deficiency in *P. ananatis* was effective for the production of the cysteine related compounds, and further combination of *fliY* deficiency exerted a synergistic effect.

**Table 3**

| Strain | Genotype | Cysteine related compounds (g/L) |
|---|---|---|
| EYPS 1976(s) | Wild-type | 0.80 ± 0.031 |
| EYPSΔfliY | ΔfliY | 0.71 ± 0.046 |
| EYPSΔydjN::Km | ΔydjN(::Km^{R}) | 0.92 ± 0.069 |
| EYPSΔfliYΔydjN::Km | ΔfliY ΔydjN(::Km^{R}) | 1.78 ± 0.081 |

### Explanation of Sequence Listing

SEQ ID NO: 1: Nucleotide sequence of *ydjN* gene of *Escherichia coli*
SEQ ID NO: 2: Amino acid sequence of YdjN of *Escherichia coli*
SEQ ID NO: 3: Nucleotide sequence of *ydjn* gene of *Pantoea ananatis*
SEQ ID NO: 4: Amino acid sequence of YdjN of *Pantoea ananatis*
SEQ ID NO: 5: Nucleotide sequence of *fliY* gene of *Escherichia coli*
SEQ ID NO: 6: Amino acid sequence of FliY of *Escherichia coli*
SEQ ID NO: 7: Nucleotide sequence of *fliY* gene of *Pantoea ananatis*
SEQ ID NO: 8: Amino acid sequence of FliY *of Pantoea ananatis*
SEQ ID NO: 9: Nucleotide sequence of *cysE* gene of *Escherichia coli*
SEQ ID NO: 10: Amino acid sequence of SAT encoded by *cysE* gene of *Escherichia coli*
SEQ ID NO: 11: Nucleotide sequence *of yecS* gene of *Pantoea ananatis*
SEQ ID NO: 12: Amino acid sequence encoded by *yecS* gene of *Pantoea ananatis*
SEQ ID NO: 13: Nucleotide sequence of *yecC* gene of *Pantoea ananatis*
SEQ ID NO: 14: Amino acid sequence encoded by *yecC* gene of *Pantoea ananatis*
SEQ ID NO: 15: Nucleotide sequence *of yeaS* gene of *Escherichia coli*
SEQ ID NO: 16: Amino acid sequence encoded by *yeaS* gene of *Escherichia coli*
SEQ ID NO: 17: Nucleotide sequence of *serA* gene of *Pantoea ananatis*
SEQ ID NO: 18: Amino acid sequence encoded by *serA* gene of *Pantoea ananatis*
SEQ ID NO: 19: Nucleotide sequence of *cysPTWA* gene cluster
SEQ ID NO: 20: Amino acid sequence encoded by *cysP* gene
SEQ ID NO: 21: Amino acid sequence encoded by *cysT* gene
SEQ ID NO: 22: Amino acid sequence encoded by *cysW* gene
SEQ ID NO: 23: Nucleotide sequence of *cysA* gene
SEQ ID NO: 24: Amino acid sequence encoded by *cysA* gene
SEQ ID NO: 25: Nucleotide sequence of *cysM* gene
SEQ ID NO: 26: Amino acid sequence encoded by *cysM* gene
SEQ ID NO: 27: Nucleotide sequence of Pnlp0
SEQ ID NO: 28: Nucleotide sequence of Pnlp8
SEQ ID NO: 29: Nucleotide sequence of Pnlp23
SEQ ID NOS: 30 to 45: Primers P1 to P16
SEQ ID NO: 46: Nucleotide sequence of primer DydjN(Pa)-F
SEQ ID NO: 47: Nucleotide sequence of primer DydjN(Pa)-R
SEQ ID NO: 48: Nucleotide sequence of primer DfliY-FW
SEQ ID NO: 49: Nucleotide sequence of primer DyecC-RV
SEQ ID NO: 50: Primer DcysE(Ec)-F
SEQ ID NO: 51: Primer DcysE(Ec)-R
SEQ ID NO: 52: Primer DydjN(Ec)-F
SEQ ID NO: 53: Primer DydjN(Ec)-R
SEQ ID NO: 54: Primer ydjN(Ec)-SalIFW2
SEQ ID NO: 55: Primer ydjN(Ec)-xbaIRV2
SEQ ID NO: 56: Primer ydjN2(Pa)-SalIFW
SEQ ID NO: 57: Primer ydjN2(Pa)-xbaIRV
SEQ ID NO: 58: Primer DfliY(Ec)-FW
SEQ ID NO: 59: Primer DfliY(Ec)-RV
SEQ ID NO: 60: Primer fliY(Ec)SalI-F
SEQ ID NO: 61: Primer fliY(Ec)XbaI-R
SEQ ID NO: 62: Nucleotide sequence of the promoter Pnlp.

## Claims

1. A method for producing L-cysteine, L-cystine, a derivative or precursor thereof, or a mixture of these, which comprises culturing a bacterium belonging to the family *Enterobacteriaceae* in a medium and collecting L-cysteine, L-cystine, a derivative or precursor thereof, or a mixture of these from the medium, wherein said bacterium is able to produce L-cysteine, and has been modified so that expression of the *ydjN* gene coding for YdjN protein is reduced by introducing a missense mutation, a nonsense mutation, or a frame shift mutation into the coding region of the *ydjN* gene on a chromosome, by deleting a part of, or the entire coding region of the *ydjN* gene on a chromosome, or by modifying an expression control sequence of the ydjN gene, or the *ydjn* gene is disrupted.

2. The method according to claim 1, wherein the YdjN protein has the amino acid sequence of SEQ ID NO: 2 or 4, or a variant thereof having the amino acid sequence of SEQ ID NO: 2 or 4 including substitutions, deletions, insertions or additions of one to 20 amino acid residues.

3. The method according to claim 1, wherein the *ydjN* gene is selected from the group consisting of:
(a) a DNA comprising the nucleotide sequence of SEQ ID NO: 1 or 3,
(b) a DNA which is able to hybridize with a sequence complementary to the nucleotide sequence of SEQ ID NO: 1 or 3 under stringent conditions comprising washing at 60°C, 0.1×SSC, 0.1% SDS,
(c) a DNA which has a homology of 95% or more to the nucleotide sequence of SEQ ID NO: 1 or 3.

4. The method according to any one of claims 1 to 3, wherein said bacterium has been further modified so that expression of the fliY gene coding for FliY protein is reduced by introducing a missense mutation, a nonsense mutation, or a frame shift mutation into the coding region of the *fliY* gene on a chromosome, by deleting a part of, or the entire coding region of the *fliY* gene on a chromosome, or by modifying an expression control sequence of the fliY gene, or the *fliY* gene is disrupted.

5. The method according to claim 4, wherein the FliY protein has the amino acid sequence of SEQ ID NO: 6 or 8, or a variant thereof having the amino acid sequence of SEQ ID NO: 6 or 8 including substitutions, deletions, insertions or additions of one to 20 amino acid residues.

6. The method according to claim 4 or 5, wherein the *fliY* gene is selected from the group consisting of:
(a) a DNA comprising the nucleotide sequence of SEQ ID NO: 5 or 7,
(b) a DNA which is able to hybridize with a sequence complementary to the nucleotide sequence of SEQ ID NO: 5 or 7 under stringent conditions comprising washing at 60°C, 0.1×SSC, 0.1% SDS,
(c) a DNA which has a homology of 95% or more to the nucleotide sequence of SEQ ID NO: 5 or 7.

7. The method according to any one of claims 1 to 6, wherein said bacterium further has at least one of the following characteristics:
i) it has been modified to increase serine acetyltransferase activity by increasing the copy number of a gene coding for serine acetyltransferase, and/or modifying an expression control sequence of a gene coding for serine acetyltransferase,
ii) it has been modified to increase expression of the *yeaS* gene by increasing the copy number of the *yeaS* gene, and/or modifying an expression control sequence of the *yeaS* gene,
iii) it has been modified to incorporate a mutant *serA* gene coding for a mutant 3-phosphoglycerate dehydrogenase for which the feedback inhibition is eliminated or attenuated,
iv) it has been modified to enhance expression of the *cysPTWAM* cluster genes coding for the sulfate/thiosulfate transport system proteins.

8. The method according to any one of claims 1 to 7, wherein said bacterium belongs to the genus *Pantoea.*

9. The method according to claim 8, wherein said bacterium is *Pantoea ananatis.*

10. The method according to any one of claims 1 to 7, wherein said bacterium is *Escherichia coli.*

## Patentansprüche

1. Verfahren zum Herstellen von L-Cystein, L-Cystin, eines Derivats oder Vorläufers davon oder eines Gemisches davon, welches das Kultivieren eines Bakteriums der Familie Enterobacteriaceae in einem Medium und das Gewinnen von L-Cystein, L-Cystin, eines Derivats oder Vorläufers davon oder eines Gemisches davon aus dem Medium umfasst, wobei das Bakterium L-Cystein produzieren kann und so modifiziert worden ist, dass die Expression des für das YdjN-Protein kodierenden ydjN-Gens verringert ist, indem eine Falschsinnmutation, eine Unsinnmutation oder eine Leserahmenmutation in die kodierende Region des *ydjN*-Gens auf einem Chromosom eingeführt ist, ein Teil oder die gesamte kodierende Region des *ydjN*-Gens auf einem Chromosom entfernt ist oder eine Expressionskontrollsequenz des *ydjN*-Gens modifiziert ist, oder das *ydjN*-Gen unterbrochen ist.

2. Verfahren nach Anspruch 1, wobei das YdjN-Protein die Aminosäuresequenz der SEQ ID NO: 2 oder 4 aufweist, oder eine Variante davon mit der Aminosäuresequenz der SEQ ID NO: 2 oder 4, einschließlich Substitutionen, Deletionen, Insertionen oder Additionen von 1 bis 20 Aminosäureresten.

3. Verfahren nach Anspruch 1, wobei das ydjN-Gen aus der Gruppe ausgewählt ist, die aus folgendem besteht:
(a) einer DNA, welche die Nukleotidsequenz der SEQ ID NO: 1 oder 3 umfasst,
(b) einer DNA, die mit einer zu der Nukleotidsequenz der SEQ ID NO: 1 oder 3 komplementären Sequenz unter stringenten Bedingungen, welche das Waschen bei 60°C, 0,1×SSC, 0,1% SDS umfasst, hybridisieren kann,
(c) einer DNA, die eine Homologie von 95% oder mehr zu der Nukleotidsequenz der SEQ ID NO: 1 oder 3 aufweist.

4. Verfahren nach einem der Ansprüche 1 bis 3, wobei das Bakterium zusätzlich so modifiziert worden ist, dass die Expression des für das FliY-Protein kodierenden *fliY*-Gens verringert ist, indem eine Falschsinnmutation, eine Unsinnmutation oder eine Leserahmenmutation in den kodierenden Bereich des *fliY*-Gens auf einem Chromosom eingeführt ist, ein Teil oder die gesamte kodierende Region des *fliY*-Gens auf einem Chromosom entfernt ist oder eine Expressionskontrollsequenz auf dem *fliY*-Gen modifiziert ist, oder das *fliY*-Gen unterbrochen ist.

5. Verfahren nach Anspruch 4, wobei das FliY-Protein die Aminosäuresequenz der SEQ ID NO: 6 oder 8 aufweist, oder eine Variante davon mit der Aminosäuresequenz der SEQ ID NO: 6 oder 8, einschließlich Substitutionen, Deletionen, Insertionen oder Additionen von 1 bis 20 Aminosäureresten.

6. Verfahren nach Anspruch 4 oder 5, wobei das *fliY*-Gen aus der Gruppe ausgewählt ist, die aus folgendem besteht:
(a) einer DNA, welche die Nukleotidsequenz der SEQ ID NO: 5 oder 7 umfasst,
(b) einer DNA, die mit einer zu der Nukleotidsequenz der SEQ ID NO: 5 oder 7 komplementären Sequenz unter stringenten Bedingungen, welche das Waschen bei 60°C, 0,1×SSC, 0,1% SDS umfasst, hybridisieren kann,
(c) einer DNA, die eine Homologie von 95% oder mehr zu der Nukleotidsequenz der SEQ ID NO: 5 oder 7 aufweist.

7. Verfahren nach einem der Ansprüche 1 bis 6, wobei das Bakterium zusätzlich mindestens eine der folgenden Eigenschaften aufweist:
i) es ist modifiziert worden, wodurch die Serinacetyltransferaseaktivität erhöht ist, indem die Kopienzahl eines für Serinacetyltransferase kodierenden Gens erhöht ist und/oder eine Expressionskontrollsequenz eines für Serinacetyltransferase kodierenden Gens modifiziert ist,
ii) es ist modifiziert worden, wodurch die Expression des yeaS-Gens erhöht ist, indem die Kopienzahl des yeaS-Gens erhöht ist und/oder eine Expressionskontrollsequenz des *yeaS-*Gens modifiziert ist,
iii) es ist modifiziert worden, wodurch ein mutiertes *serA*-Gen eingeführt worden ist, das für eine mutierte 3-Phosphoglyceratdehydrogenase kodiert, deren Rückkopplungshemmung ausgeschaltet oder verringert ist,
iv) es ist modifiziert worden, wodurch die Expression der für die Sulfat/Thiosulfattransportsystemproteine kodierenden *cysPTWAM*-Clustergene erhöht ist.

8. Verfahren nach einem der Ansprüche 1 bis 7, wobei das Bakterium zu der Gattung *Pantoea* gehört.

9. Verfahren nach Anspruch 8, wobei das Bakterium *Pantoea ananatis* ist.

10. Verfahren nach einem der Ansprüche 1 bis 7, wobei das Bakterium *Escherichia coli* ist.

## Revendications

1. Procédé pour produire de la L-cystéine, de la L-cystine, un dérivé ou précurseur de celles-ci, ou un mélange de celles-ci, qui comprend la culture d'une bactérie appartenant à la famille des *Enterobacteriaceae* dans un milieu et la collecte de la L-cystéine, de la L-cystine, d'un dérivé ou précurseur de celles-ci, ou d'un mélange de celles-ci à partir du milieu, où ladite bactérie est capable de produire de la L-cystéine, et a été modifiée de sorte que l'expression du gène *ydjN* codant la protéine YdjN est réduite par introduction d'une mutation faux sens, d'une mutation non sens ou d'une mutation par décalage du cadre dans la région codante du gène *ydjN* sur un chromosome, par délétion d'une partie ou de la totalité de la région codante du gène *ydjN* sur un chromosome, ou par modification d'une séquence de contrôle de l'expression du gène ydjN, ou bien le gène *ydjN* est rompu.

2. Procédé selon la revendication 1, où la protéine YdjN a la séquence d'aminoacides de SEQ ID NO:2 ou 4, ou une variante de celle-ci ayant la séquence d'aminoacides de SEQ ID NO:2 ou 4 incluant des substitutions, des délétions, des insertions ou des additions de 1 à 20 résidus d'aminoacides.

3. Procédé selon la revendication 1, où le gène *ydjN* est choisi dans le groupe consistant en :
(a) un ADN comprenant la séquence nucléotidique de SEQ ID NO:1 ou 3,
(b) un ADN qui est capable de s'hybrider avec une séquence complémentaire de la séquence nucléotidique de SEQ ID NO:1 ou 3 dans des conditions stringentes comprenant un lavage à 60°C, 0,1xSSC, 0,1 % de SDS,
(c) un ADN qui a une homologie de 95 % ou plus avec la séquence nucléotidique de SEQ ID NO:1 ou 3.

4. Procédé selon l'une quelconque des revendications 1 à 3, où ladite bactérie a été modifiée en outre de sorte que l'expression du gène *fliY* codant la protéine FliY est réduite par introduction d'une mutation faux sens, d'une mutation non sens ou d'une mutation par décalage du cadre dans la région codante du gène *fliY* sur un chromosome, par délétion d'une partie ou de la totalité de la région codante du gène *fliY sur* un chromosome, ou par modification d'une séquence de contrôle de l'expression du gène *fliY,* ou bien le gène *fliY* est rompu.

5. Procédé selon la revendication 4, où la protéine FliY a la séquence d'aminoacides de SEQ ID NO:6 ou 8, ou une variante de celle-ci ayant la séquence d'aminoacides de SEQ ID NO:6 ou 8 incluant des substitutions, des délétions, des insertions ou des additions de 1 à 20 résidus d'aminoacides.

6. Procédé selon la revendication 4 ou 5, où le gène *fliY* est choisi dans le groupe consistant en :
(a) un ADN comprenant la séquence nucléotidique de SEQ ID NO:5 ou 7,
(b) un ADN qui est capable de s'hybrider avec une séquence complémentaire de la séquence nucléotidique de SEQ ID NO:5 ou 7 dans des conditions stringentes comprenant un lavage à 60°C, 0,1xSSC, 0,1 % de SDS,
(c) un ADN qui a une homologie de 95 % ou plus avec la séquence nucléotidique de SEQ ID NO:5 ou 7.

7. Procédé selon l'une quelconque des revendications 1 à 6, où ladite bactérie a en outre au moins l'une des caractéristiques suivantes :
i) elle a été modifiée pour augmenter l'activité sérine acétyltransférase en augmentant le nombre de copies d'un gène codant la sérine acétyltransférase, et/ou modifiant une séquence de contrôle de l'expression d'un gène codant la sérine acétyltransférase,
ii) elle a été modifiée pour augmenter l'expression du gène *yeaS en* augmentant le nombre de copies du gène *yeaS,* et/ou modifiant une séquence de contrôle de l'expression du gène *yeaS,*
iii) elle a été modifiée pour incorporer un gène *serA* mutant codant une 3-phosphoglycérate déshydrogénase mutante pour laquelle la rétro-inhibition est éliminée ou atténuée,
iv) elle a été modifiée pour augmenter l'expression des gènes de cluster *cysPTWAM* codant les protéines du système de transport sulfate/thiosulfate.

8. Procédé selon l'une quelconque des revendications 1 à 7, où ladite bactérie appartient au genre *Pantoea.*

9. Procédé selon la revendication 8, où ladite bactérie est *Pantoea ananatis.*

10. Procédé selon l'une quelconque des revendications 1 à 7, où ladite bactérie est *Escherichia coli.*


### SEQUENCE LISTING

<110> Ajinomoto Co., Inc.
<120> An L-cystein producing bacterium and a method for producing L-cysteine
<130> EPA-64886
<150> JP2009-059792
   <151> 2009-03-12
<160> 62
<170> PatentIn version 3.5
<210> 1
   <211> 1392
   <212> DNA
   <213> Escherichia coli
<220>
   <221> CDS
   <222> (1)..(1392)
<400> 1
   atg aac ttt cca tta att gcg aac atc gtg gtg ttc gtt gta ctg ctg      48
   Met Asn Phe Pro Leu Ile Ala Asn Ile Val Val Phe Val Val Leu Leu
   1               5                   10                  15
   ttt gcg ctg gct cag acc cgc cat aaa cag tgg agt ctg gcg aaa aaa      96
   Phe Ala Leu Ala Gln Thr Arg His Lys Gln Trp Ser Leu Ala Lys Lys
               20                  25                  30
   gtg ctg gtg ggt ctg gtg atg ggt gtg gtt ttt ggc ctt gcc ctg cat     144
   Val Leu Val Gly Leu Val Met Gly Val Val Phe Gly Leu Ala Leu His
           35                  40                  45
   acc att tat ggt tct gac agc cag gta ctt aaa gat tct gta cag tgg     192
   Thr Ile Tyr Gly Ser Asp Ser Gln Val Leu Lys Asp Ser Val Gln Trp
       50                  55                  60
   ttt aac atc gtt ggt aac ggc tat gtt caa ctg ctg caa atg atc gtt     240
   Phe Asn Ile Val Gly Asn Gly Tyr Val Gln Leu Leu Gln Met Ile Val
   65                  70                  75                  80
   atg ccg tta gtc ttc gcc tct att ctg agc gcg gtt gcc cgt ctg cat     288
   Met Pro Leu Val Phe Ala Ser Ile Leu Ser Ala Val Ala Arg Leu His
                   85                  90                  95
   aac gca tct cag tta ggc aaa atc agt ttt ctg acc atc ggt acg ctt     336
   Asn Ala Ser Gln Leu Gly Lys Ile Ser Phe Leu Thr Ile Gly Thr Leu
               100                 105                 110
   ttg ttt acc acg ctg att gcg gcg ctg gtc ggt gtg ctg gtc acc aac     384
   Leu Phe Thr Thr Leu Ile Ala Ala Leu Val Gly Val Leu Val Thr Asn
           115                 120                 125
   ctg ttt ggt ttg acg gct gaa ggt ctg gtt cag ggt ggt gca gaa act     432
   Leu Phe Gly Leu Thr Ala Glu Gly Leu Val Gln Gly Gly Ala Glu Thr
       130                 135                 140
   gca cgt ctg aac gcc att gaa agt aac tat gtt ggt aaa gtc tct gat     480
   Ala Arg Leu Asn Ala Ile Glu Ser Asn Tyr Val Gly Lys Val Ser Asp
   145                 150                 155                 160
   ctg agc gtt ccg cag ctg gtc ttg tcc ttt atc ccg aaa aac ccg ttt     528
   Leu Ser Val Pro Gln Leu Val Leu Ser Phe Ile Pro Lys Asn Pro Phe
                   165                 170                 175
   gcc gat ctt acc gga gcc aat ccg acg tca att atc agc gtg gta att     576
   Ala Asp Leu Thr Gly Ala Asn Pro Thr Ser Ile Ile Ser Val Val Ile
               180                 185                 190
   ttt gcc gca ttc ctc ggc gta gct gca ttg aaa ctg ctg aag gat gat     624
   Phe Ala Ala Phe Leu Gly Val Ala Ala Leu Lys Leu Leu Lys Asp Asp
           195                 200                 205
   gcg ccg aaa ggt gaa cgc gtc tta gcc gct atc gat acc cta caa agc     672
   Ala Pro Lys Gly Glu Arg Val Leu Ala Ala Ile Asp Thr Leu Gln Ser
       210                 215                 220
   tgg gtg atg aaa ctg gtt cgc ctg gtc atg cag ttg acc cct tac ggc      720
   Trp Val Met Lys Leu Val Arg Leu Val Met Gln Leu Thr Pro Tyr Gly
   225                 230                 235                 240
   gtt ctg gct cta atg acc aaa gtg gtt gca ggt tct aac ttg caa gac      768
   Val Leu Ala Leu Met Thr Lys Val Val Ala Gly Ser Asn Leu Gln Asp
                   245                 250                 255
   atc atc aaa ctg gga agt ttc gtt gtc gcg tcc tac ctc ggt ctg ctg      816
   Ile Ile Lys Leu Gly Ser Phe Val Val Ala Ser Tyr Leu Gly Leu Leu
               260                 265                 270
   att atg ttt gca gtg cat ggc att ctg ctg ggc att aat ggc gtg agt      864
   Ile Met Phe Ala Val His Gly Ile Leu Leu Gly Ile Asn Gly Val Ser
           275                 280                 285
   ccg ctg aag tac ttc cgt aag gta tgg cct gtg ctg acg ttt gcc ttt      912
   Pro Leu Lys Tyr Phe Arg Lys Val Trp Pro Val Leu Thr Phe Ala Phe
       290                 295                 300
   acc agc cgt tcc agt gct gcg tct atc cca ctg aat gtg gaa gca caa      960
   Thr Ser Arg Ser Ser Ala Ala Ser Ile Pro Leu Asn Val Glu Ala Gln
   305                 310                 315                 320
   acg cgt cgt ctg ggc gtt cct gaa tcc atc gcc agt ttc gcc gcc tct     1008
   Thr Arg Arg Leu Gly Val Pro Glu Ser Ile Ala Ser Phe Ala Ala Ser
                   325                 330                 335
   ttc ggt gca acc att ggt cag aac ggc tgc gcc ggt ttg tat ccg gca     1056
   Phe Gly Ala Thr Ile Gly Gln Asn Gly Cys Ala Gly Leu Tyr Pro Ala
               340                 345                 350
   atg ctg gcg gtg atg gtt gcg cct acg gtt ggc att aac ccg ctg gac     1104
   Met Leu Ala Val Met Val Ala Pro Thr Val Gly Ile Asn Pro Leu Asp
           355                 360                 365
   ccg atg tgg att gcg acg ctg gtc ggt att gtt acc gtt agt tcc gca     1152
   Pro Met Trp Ile Ala Thr Leu Val Gly Ile Val Thr Val Ser Ser Ala
       370                 375                 380
   ggc gtt gcc ggt gtc ggt ggt ggt gca act ttc gcc gca ctg att gta     1200
   Gly Val Ala Gly Val Gly Gly Gly Ala Thr Phe Ala Ala Leu Ile Val
   385                 390                 395                 400
   ctg cct gcg atg ggc ctg cca gta acc ctg gtg gcg ctg tta atc tcc     1248
   Leu Pro Ala Met Gly Leu Pro Val Thr Leu Val Ala Leu Leu Ile Ser
                   405                 410                 415
   gtt gaa ccg ctt atc gac atg ggc cgt acg gcg tta aac gtt agt ggc     1296
   Val Glu Pro Leu Ile Asp Met Gly Arg Thr Ala Leu Asn Val Ser Gly
               420                 425                 430
   tcg atg aca gct ggc acg ctg acc agc cag tgg ctg aag caa acc gat     1344
   Ser Met Thr Ala Gly Thr Leu Thr Ser Gln Trp Leu Lys Gln Thr Asp
           435                 440                 445
   aaa gcc att ctg gat agc gaa gac gac gcc gaa ctg gca cac cat taa     1392
   Lys Ala Ile Leu Asp Ser Glu Asp Asp Ala Glu Leu Ala His His
       450                 455                 460
<210> 2
   <211> 463
   <212> PRT
   <213> Escherichia coli
<400> 2
   Met Asn Phe Pro Leu Ile Ala Asn Ile Val Val Phe Val Val Leu Leu
   1 5 10 15
   Phe Ala Leu Ala Gln Thr Arg His Lys Gln Trp Ser Leu Ala Lys Lys
   20 25 30
   Val Leu Val Gly Leu Val Met Gly Val Val Phe Gly Leu Ala Leu His
   35 40 45
   Thr Ile Tyr Gly Ser Asp Ser Gln Val Leu Lys Asp Ser Val Gln Trp
   50 55 60
   Phe Asn Ile Val Gly Asn Gly Tyr Val Gln Leu Leu Gln Met Ile Val
   65 70 75 80
   Met Pro Leu Val Phe Ala Ser Ile Leu Ser Ala Val Ala Arg Leu His
   85 90 95
   Asn Ala Ser Gln Leu Gly Lys Ile Ser Phe Leu Thr Ile Gly Thr Leu
   100 105 110
   Leu Phe Thr Thr Leu Ile Ala Ala Leu Val Gly Val Leu Val Thr Asn
   115 120 125
   Leu Phe Gly Leu Thr Ala Glu Gly Leu Val Gln Gly Gly Ala Glu Thr
   130 135 140
   Ala Arg Leu Asn Ala Ile Glu Ser Asn Tyr Val Gly Lys Val Ser Asp
   145 150 155 160
   Leu Ser Val Pro Gln Leu Val Leu Ser Phe Ile Pro Lys Asn Pro Phe
   165 170 175
   Ala Asp Leu Thr Gly Ala Asn Pro Thr Ser Ile Ile Ser Val Val Ile
   180 185 190
   Phe Ala Ala Phe Leu Gly Val Ala Ala Leu Lys Leu Leu Lys Asp Asp
   195 200 205
   Ala Pro Lys Gly Glu Arg Val Leu Ala Ala Ile Asp Thr Leu Gln Ser
   210 215 220
   Trp Val Met Lys Leu Val Arg Leu Val Met Gln Leu Thr Pro Tyr Gly
   225 230 235 240
   Val Leu Ala Leu Met Thr Lys Val Val Ala Gly Ser Asn Leu Gln Asp
   245 250 255
   Ile Ile Lys Leu Gly Ser Phe Val Val Ala Ser Tyr Leu Gly Leu Leu
   260 265 270
   Ile Met Phe Ala Val His Gly Ile Leu Leu Gly Ile Asn Gly Val Ser
   275 280 285
   Pro Leu Lys Tyr Phe Arg Lys Val Trp Pro Val Leu Thr Phe Ala Phe
   290 295 300
   Thr Ser Arg Ser Ser Ala Ala Ser Ile Pro Leu Asn Val Glu Ala Gln
   305 310 315 320
   Thr Arg Arg Leu Gly Val Pro Glu Ser Ile Ala Ser Phe Ala Ala Ser
   325 330 335
   Phe Gly Ala Thr Ile Gly Gln Asn Gly Cys Ala Gly Leu Tyr Pro Ala
   340 345 350
   Met Leu Ala Val Met Val Ala Pro Thr Val Gly Ile Asn Pro Leu Asp
   355 360 365
   Pro Met Trp Ile Ala Thr Leu Val Gly Ile Val Thr Val Ser Ser Ala
   370 375 380
   Gly Val Ala Gly Val Gly Gly Gly Ala Thr Phe Ala Ala Leu Ile Val
   385 390 395 400
   Leu Pro Ala Met Gly Leu Pro Val Thr Leu Val Ala Leu Leu Ile Ser
   405 410 415
   Val Glu Pro Leu Ile Asp Met Gly Arg Thr Ala Leu Asn Val Ser Gly
   420 425 430
   Ser Met Thr Ala Gly Thr Leu Thr Ser Gln Trp Leu Lys Gln Thr Asp
   435 440 445
   Lys Ala Ile Leu Asp Ser Glu Asp Asp Ala Glu Leu Ala His His
   450 455 460
<210> 3
   <211> 1392
   <212> DNA
   <213> Pantoea ananatis
<220>
   <221> CDS
   <222> (1)..(1392)
<400> 3
   atg gat att cct ctt acg ctt aac gtc gtg gca ttt gtg ctg cta ctg 48
   Met Asp Ile Pro Leu Thr Leu Asn Val Val Ala Phe Val Leu Leu Leu
   1 5 10 15
   ctt ttg ctg tca cgc ctt ggc cgg gca aac tgg agc ctg tcg aaa aag 96
   Leu Leu Leu Ser Arg Leu Gly Arg Ala Asn Trp Ser Leu Ser Lys Lys
   20 25 30
   gtt ctg act ggc ctg gtg ctg ggt gtg gtg ttt ggc ctc gcg ctc cag 144
   Val Leu Thr Gly Leu Val Leu Gly Val Val Phe Gly Leu Ala Leu Gln
   35 40 45
   ctg att tat ggc ggc aac agc gat atc gtt aaa gcc tct atc ggc tgg 192
   Leu Ile Tyr Gly Gly Asn Ser Asp Ile Val Lys Ala Ser Ile Gly Trp
   50 55 60
   ttt aac atc gtg ggt aac ggt tat gtt cag ctg tta cag atg att gtg 240
   Phe Asn Ile Val Gly Asn Gly Tyr Val Gln Leu Leu Gln Met Ile Val
   65 70 75 80
   atg ccg ctg gtg ttc gtg tcg att ctc agc gcc gtg gcc cgc ctg cac        288
   Met Pro Leu Val Phe Val Ser Ile Leu Ser Ala Val Ala Arg Leu His
                   85                  90                  95
   aat gcc tcc tca ctg ggg aaa atc agt ttt ctg acc atc ggc gta ctg        336
   Asn Ala Ser Ser Leu Gly Lys Ile Ser Phe Leu Thr Ile Gly Val Leu
               100                 105                 110
   ctg ttt aca acg gca att tct gcc ctg atc ggc gtt ttt gtc acc ggc        384
   Leu Phe Thr Thr Ala Ile Ser Ala Leu Ile Gly Val Phe Val Thr Gly
           115                 120                 125
   ctg ttt cac ctt aac gcg acc ggt ctg gta cag ggc gcc cag gaa acg        432
   Leu Phe His Leu Asn Ala Thr Gly Leu Val Gln Gly Ala Gln Glu Thr
       130                 135                 140
   gct cgc ctg agc gcg att cag agc aac tac gtt ggt aaa gtc gct gat        480
   Ala Arg Leu Ser Ala Ile Gln Ser Asn Tyr Val Gly Lys Val Ala Asp
   145                 150                 155                 160
   ttg aca gtg cct cag tta atc ctg tca ttc att cct aaa aac ccg ttt        528
   Leu Thr Val Pro Gln Leu Ile Leu Ser Phe Ile Pro Lys Asn Pro Phe
                   165                 170                 175
   gcc gac tta acc ggt gcc agc ccg acg tcc atc atc agc gtg gtc atc        576
   Ala Asp Leu Thr Gly Ala Ser Pro Thr Ser Ile Ile Ser Val Val Ile
               180                 185                 190
   ttc gcc gcg ttc ctg ggt gtg gct gcc ctg cag ttg cac aag gat gat        624
   Phe Ala Ala Phe Leu Gly Val Ala Ala Leu Gln Leu His Lys Asp Asp
           195                 200                 205
   gag gta aaa ggc cag cgg gta ctg acc gcc att gat acg ctg cag tcg        672
   Glu Val Lys Gly Gln Arg Val Leu Thr Ala Ile Asp Thr Leu Gln Ser
       210                 215                 220
   tgg gtg atg aag ctg gtt cgc ctg att atg aag ctg aca cct tat ggt        720
   Trp Val Met Lys Leu Val Arg Leu Ile Met Lys Leu Thr Pro Tyr Gly
   225                 230                 235                 240
   gtg ctg gcg ctg atg acc aaa gtc gtt gcc ggc tcc aac gtg cag gac        768
   Val Leu Ala Leu Met Thr Lys Val Val Ala Gly Ser Asn Val Gln Asp
                   245                 250                 255
   atc atc aag ctg ggc agc ttt gtt gtg gca tcc tat ttg ggc ctg acc        816
   Ile Ile Lys Leu Gly Ser Phe Val Val Ala Ser Tyr Leu Gly Leu Thr
               260                 265                 270
   ctt atg ttt gtg gtt cat gcg ctg ctc ctg tcg gtc aac ggc atc aat        864
   Leu Met Phe Val Val His Ala Leu Leu Leu Ser Val Asn Gly Ile Asn
           275                 280                 285
   cct atg cgc ttc ttc cgc aaa gtg tgg ccg gta ttg acc ttt gcc ttt        912
   Pro Met Arg Phe Phe Arg Lys Val Trp Pro Val Leu Thr Phe Ala Phe
       290                 295                 300
   acc agc cgc tcc agc gcc gcc agc att cca ctg aac gtg gag gcg caa        960
   Thr Ser Arg Ser Ser Ala Ala Ser Ile Pro Leu Asn Val Glu Ala Gln
   305                 310                 315                 320
   acc cgc cgc ctt ggc gta cct gaa tcg att gcc agc ttc tcg gcc tca       1008
   Thr Arg Arg Leu Gly Val Pro Glu Ser Ile Ala Ser Phe Ser Ala Ser
                   325                 330                 335
   ttt ggt gcc acc att gga cag aac ggc tgt gcc ggt ctt tat ccg acg       1056
   Phe Gly Ala Thr Ile Gly Gln Asn Gly Cys Ala Gly Leu Tyr Pro Thr
               340                 345                 350
   atg ctg gca gtg atg gtc gcc ccg acg gtc ggc atc aat cct ttt gat       1104
   Met Leu Ala Val Met Val Ala Pro Thr Val Gly Ile Asn Pro Phe Asp
           355                 360                 365
   ccg ctg tgg att gcc acg ctg gtc ggc att gtg aca ttg agt tca gca       1152
   Pro Leu Trp Ile Ala Thr Leu Val Gly Ile Val Thr Leu Ser Ser Ala
       370                 375                 380
   ggc gta gcc ggt gtc ggc ggc gga gcg acc ttt gcc gcg ctg atc gtc       1200
   Gly Val Ala Gly Val Gly Gly Gly Ala Thr Phe Ala Ala Leu Ile Val
   385                 390                 395                 400
   ctg cct gcg atg ggg ctg ccg gtc acg ctg gtc gcg ctg ctg att tcc       1248
   Leu Pro Ala Met Gly Leu Pro Val Thr Leu Val Ala Leu Leu Ile Ser
                   405                 410                 415
   att gag cct ctg att gac atg gga cga aca gcg tta aac gtc aat ggc       1296
   Ile Glu Pro Leu Ile Asp Met Gly Arg Thr Ala Leu Asn Val Asn Gly
               420                 425                 430
   tca atg acc gca gga tcg ctc acc agt cgc tgg ctg ggc ctg acc gat       1344
   Ser Met Thr Ala Gly Ser Leu Thr Ser Arg Trp Leu Gly Leu Thr Asp
           435                 440                 445
   aaa cgc gtt tta gag cgc agc gaa cac agt gaa tta gag cac agc taa      1392
   Lys Arg Val Leu Glu Arg Ser Glu His Ser Glu Leu Glu His Ser
       450                 455                     460
<210> 4
   <211> 463
   <212> PRT
   <213> Pantoea ananatis
<400> 4
   Met Asp Ile Pro Leu Thr Leu Asn Val Val Ala Phe Val Leu Leu Leu
   1 5 10 15
   Leu Leu Leu Ser Arg Leu Gly Arg Ala Asn Trp Ser Leu Ser Lys Lys
   20 25 30
   Val Leu Thr Gly Leu Val Leu Gly Val Val Phe Gly Leu Ala Leu Gln
   35 40 45
   Leu Ile Tyr Gly Gly Asn Ser Asp Ile Val Lys Ala Ser Ile Gly Trp
   50 55 60
   Phe Asn Ile Val Gly Asn Gly Tyr Val Gln Leu Leu Gln Met Ile Val
   65 70 75 80
   Met Pro Leu Val Phe Val Ser Ile Leu Ser Ala Val Ala Arg Leu His
   85 90 95
   Asn Ala Ser Ser Leu Gly Lys Ile Ser Phe Leu Thr Ile Gly Val Leu
   100 105 110
   Leu Phe Thr Thr Ala Ile Ser Ala Leu Ile Gly Val Phe Val Thr Gly
   115 120 125
   Leu Phe His Leu Asn Ala Thr Gly Leu Val Gln Gly Ala Gln Glu Thr
   130 135 140
   Ala Arg Leu Ser Ala Ile Gln Ser Asn Tyr Val Gly Lys Val Ala Asp
   145 150 155 160
   Leu Thr Val Pro Gln Leu Ile Leu Ser Phe Ile Pro Lys Asn Pro Phe
   165 170 175
   Ala Asp Leu Thr Gly Ala Ser Pro Thr Ser Ile Ile Ser Val Val Ile
   180 185 190
   Phe Ala Ala Phe Leu Gly Val Ala Ala Leu Gln Leu His Lys Asp Asp
   195 200 205
   Glu Val Lys Gly Gln Arg Val Leu Thr Ala Ile Asp Thr Leu Gln Ser
   210 215 220
   Trp Val Met Lys Leu Val Arg Leu Ile Met Lys Leu Thr Pro Tyr Gly
   225 230 235 240
   Val Leu Ala Leu Met Thr Lys Val Val Ala Gly Ser Asn Val Gln Asp
   245 250 255
   Ile Ile Lys Leu Gly Ser Phe Val Val Ala Ser Tyr Leu Gly Leu Thr
   260 265 270
   Leu Met Phe Val Val His Ala Leu Leu Leu Ser Val Asn Gly Ile Asn
   275 280 285
   Pro Met Arg Phe Phe Arg Lys Val Trp Pro Val Leu Thr Phe Ala Phe
   290 295 300
   Thr Ser Arg Ser Ser Ala Ala Ser Ile Pro Leu Asn Val Glu Ala Gln
   305 310 315 320
   Thr Arg Arg Leu Gly Val Pro Glu Ser Ile Ala Ser Phe Ser Ala Ser
   325 330 335
   Phe Gly Ala Thr Ile Gly Gln Asn Gly Cys Ala Gly Leu Tyr Pro Thr
   340 345 350
   Met Leu Ala Val Met Val Ala Pro Thr Val Gly Ile Asn Pro Phe Asp
   355 360 365
   Pro Leu Trp Ile Ala Thr Leu Val Gly Ile Val Thr Leu Ser Ser Ala
   370 375 380
   Gly Val Ala Gly Val Gly Gly Gly Ala Thr Phe Ala Ala Leu Ile Val
   385 390 395 400
   Leu Pro Ala Met Gly Leu Pro Val Thr Leu Val Ala Leu Leu Ile Ser
   405 410 415
   Ile Glu Pro Leu Ile Asp Met Gly Arg Thr Ala Leu Asn Val Asn Gly
   420 425 430
   Ser Met Thr Ala Gly Ser Leu Thr Ser Arg Trp Leu Gly Leu Thr Asp
   435 440 445
   Lys Arg Val Leu Glu Arg Ser Glu His Ser Glu Leu Glu His Ser
   450 455 460
<210> 5
   <211> 801
   <212> DNA
   <213> Escherichia coli
<220>
   <221> CDS
   <222> (1)..(801)
<400> 5
   atg aaa tta gca cat ctg gga cgt cag gca ttg atg ggt gtg atg gcc      48
   Met Lys Leu Ala His Leu Gly Arg Gln Ala Leu Met Gly Val Met Ala
   1               5                   10                  15
   gtg gcg ctg gtt gcg ggc atg agc gtt aaa agt ttt gca gat gaa ggt      96
   Val Ala Leu Val Ala Gly Met Ser Val Lys Ser Phe Ala Asp Glu Gly
               20                  25                  30
   ctg ctt aat aaa gtt aaa gag cgc ggc acg ctg ctg gta ggg ctg gaa      144
   Leu Leu Asn Lys Val Lys Glu Arg Gly Thr Leu Leu Val Gly Leu Glu
           35                  40                  45
   gga act tat ccg ccg ttc agt ttt cag gga gat gac ggc aaa tta acc      192
   Gly Thr Tyr Pro Pro Phe Ser Phe Gln Gly Asp Asp Gly Lys Leu Thr
       50                  55                  60
   ggt ttt gaa gtg gaa ttt gcc caa cag ctg gca aaa cat ctt ggc gtt      240
   Gly Phe Glu Val Glu Phe Ala Gln Gln Leu Ala Lys His Leu Gly Val
   65                  70                  75                  80
   gag gcg tca cta aaa ccg acc aaa tgg gac ggt atg ctg gcg tcg ctg      288
   Glu Ala Ser Leu Lys Pro Thr Lys Trp Asp Gly Met Leu Ala Ser Leu
                   85                  90                  95
   gac tct aaa cgt att gat gtg gtg att aat cag gtc acc att tct gat      336
   Asp Ser Lys Arg Ile Asp Val Val Ile Asn Gln Val Thr Ile Ser Asp
               100                 105                 110
   gag cgc aag aaa aaa tac gat ttc tca acc ccg tac acc att tct ggt      384
   Glu Arg Lys Lys Lys Tyr Asp Phe Ser Thr Pro Tyr Thr Ile Ser Gly
           115                 120                 125
   att cag gcg ctg gtg aaa aaa ggt aac gaa ggc acc att aaa aca gcc      432
   Ile Gln Ala Leu Val Lys Lys Gly Asn Glu Gly Thr Ile Lys Thr Ala
       130                 135                 140
   gat gat ctg aaa ggc aaa aaa gtg ggg gtc ggt ctg ggc acc aac tat      480
   Asp Asp Leu Lys Gly Lys Lys Val Gly Val Gly Leu Gly Thr Asn Tyr
   145                 150                 155                 160
   gaa gag tgg ctg cgg cag aat gtt cag ggc gtc gat gtg cgt acc tat      528
   Glu Glu Trp Leu Arg Gln Asn Val Gln Gly Val Asp Val Arg Thr Tyr
                   165                 170                 175
   gat gat gac ccg acc aaa tat cag gat ctg cgc gta ggg cgt atc gat      576
   Asp Asp Asp Pro Thr Lys Tyr Gln Asp Leu Arg Val Gly Arg Ile Asp
               180                 185                 190
   gcg atc ctc gtt gat cgt ctg gcg gcg ctg gat ctg gtg aag aaa acc      624
   Ala Ile Leu Val Asp Arg Leu Ala Ala Leu Asp Leu Val Lys Lys Thr
           195                 200                 205
   aac gat acg ctg gca gta acc ggt gaa gca ttc tcc cgt cag gag tct      672
   Asn Asp Thr Leu Ala Val Thr Gly Glu Ala Phe Ser Arg Gln Glu Ser
       210                 215                 220
   ggc gtg gcg ctg cgt aaa gga aat gag gac ctg ctg aaa gca gtg aat      720
   Gly Val Ala Leu Arg Lys Gly Asn Glu Asp Leu Leu Lys Ala Val Asn
   225                 230                 235                 240
   gat gca att gcg gaa atg caa aaa gat ggc act ctg caa gcc ctt tcc      768
   Asp Ala Ile Ala Glu Met Gln Lys Asp Gly Thr Leu Gln Ala Leu Ser
                   245                 250                 255
   gaa aaa tgg ttt ggt gct gat gtg acc aaa taa                          801
   Glu Lys Trp Phe Gly Ala Asp Val Thr Lys
               260                 265
<210> 6
   <211> 266
   <212> PRT
   <213> Escherichia coli
<400> 6
   Met Lys Leu Ala His Leu Gly Arg Gln Ala Leu Met Gly Val Met Ala
   1               5                   10                  15
   Val Ala Leu Val Ala Gly Met Ser Val Lys Ser Phe Ala Asp Glu Gly
               20                  25                  30
   Leu Leu Asn Lys Val Lys Glu Arg Gly Thr Leu Leu Val Gly Leu Glu
           35                  40                  45
   Gly Thr Tyr Pro Pro Phe Ser Phe Gln Gly Asp Asp Gly Lys Leu Thr
       50                  55                  60
   Gly Phe Glu Val Glu Phe Ala Gln Gln Leu Ala Lys His Leu Gly Val
   65                  70                  75                  80
   Glu Ala Ser Leu Lys Pro Thr Lys Trp Asp Gly Met Leu Ala Ser Leu
                   85                  90                  95
   Asp Ser Lys Arg Ile Asp Val Val Ile Asn Gln Val Thr Ile Ser Asp
               100                 105                 110
   Glu Arg Lys Lys Lys Tyr Asp Phe Ser Thr Pro Tyr Thr Ile Ser Gly
           115                 120                 125
   Ile Gln Ala Leu Val Lys Lys Gly Asn Glu Gly Thr Ile Lys Thr Ala
       130                 135                 140
   Asp Asp Leu Lys Gly Lys Lys Val Gly Val Gly Leu Gly Thr Asn Tyr
   145                 150                 155                 160
   Glu Glu Trp Leu Arg Gln Asn Val Gln Gly Val Asp Val Arg Thr Tyr
                   165                 170                 175
   Asp Asp Asp Pro Thr Lys Tyr Gln Asp Leu Arg Val Gly Arg Ile Asp
               180                 185                 190
   Ala Ile Leu Val Asp Arg Leu Ala Ala Leu Asp Leu Val Lys Lys Thr
           195                 200                 205
   Asn Asp Thr Leu Ala Val Thr Gly Glu Ala Phe Ser Arg Gln Glu Ser
       210                 215                 220
   Gly Val Ala Leu Arg Lys Gly Asn Glu Asp Leu Leu Lys Ala Val Asn
   225                 230                 235                 240
   Asp Ala Ile Ala Glu Met Gln Lys Asp Gly Thr Leu Gln Ala Leu Ser
                   245                 250                 255
   Glu Lys Trp Phe Gly Ala Asp Val Thr Lys
               260                 265
<210> 7
   <211> 801
   <212> DNA
   <213> Pantoea ananatis
<220>
   <221> CDS
   <222> (1)..(801)
<400> 7
   atg gct ttc tca cag att cgt cgc cag gtg gtg acg gga atg atg gcg      48
   Met Ala Phe Ser Gln Ile Arg Arg Gln Val Val Thr Gly Met Met Ala
   1               5                   10                  15
   gtt gcg ctg gtg gca ggc ttc agc gtt aaa acg ttt gcg gca gac gat      96
   Val Ala Leu Val Ala Gly Phe Ser Val Lys Thr Phe Ala Ala Asp Asp
               20                  25                  30
   tta ctg gcg cag gtt aaa agc aaa ggc gag ctg cgc gtc ggt ctg gaa      144
   Leu Leu Ala Gln Val Lys Ser Lys Gly Glu Leu Arg Val Gly Leu Glu
           35                  40                  45
   ggc acc tat cct cct ttc agt ttt cag gat gaa aag ggc aag ctg acg      192
   Gly Thr Tyr Pro Pro Phe Ser Phe Gln Asp Glu Lys Gly Lys Leu Thr
       50                  55                  60
   ggc ttt gaa gtg gag ttt gct cag gat ctg gcc aaa cat atg ggg gtt      240
   Gly Phe Glu Val Glu Phe Ala Gln Asp Leu Ala Lys His Met Gly Val
   65                  70                  75                  80
   aaa gcc gtc ctg aag cca acc aag tgg gat ggc atg tta gcg gcg ctg      288
   Lys Ala Val Leu Lys Pro Thr Lys Trp Asp Gly Met Leu Ala Ala Leu
                   85                  90                  95
   gat tcg aaa cgc atc gat gtg gtc atc aat cag gtg acg atc tcc gat      336
   Asp Ser Lys Arg Ile Asp Val Val Ile Asn Gln Val Thr Ile Ser Asp
               100                 105                 110
   gag cgt aaa aag aaa tat gat ttc tct acg ccg tat acg att tct ggc      384
   Glu Arg Lys Lys Lys Tyr Asp Phe Ser Thr Pro Tyr Thr Ile Ser Gly
           115                 120                 125
   gtg cag gcg ctg acg ctg aag aaa aac gcg ggc agc atc act aag cca      432
   Val Gln Ala Leu Thr Leu Lys Lys Asn Ala Gly Ser Ile Thr Lys Pro
       130                 135                 140
   gaa gac ctc gca ggg aag aaa gtc ggt gtg ggc ctg ggg acc aac tac      480
   Glu Asp Leu Ala Gly Lys Lys Val Gly Val Gly Leu Gly Thr Asn Tyr
   145                 150                 155                 160
   gag cag tgg ctg cgc gcg aac gta aaa ggt gtg gat atc cgc act tat      528
   Glu Gln Trp Leu Arg Ala Asn Val Lys Gly Val Asp Ile Arg Thr Tyr
                   165                 170                 175
   gat gat gac cca acc aaa tat cag gat ctg cgt tct ggt cgc gtt gat      576
   Asp Asp Asp Pro Thr Lys Tyr Gln Asp Leu Arg Ser Gly Arg Val Asp
               180                 185                 190
   gcg att ctg gtg gat cgc ctg gct gct ctg gat ctg gtg aag aaa acg      624
   Ala Ile Leu Val Asp Arg Leu Ala Ala Leu Asp Leu Val Lys Lys Thr
           195                 200                 205
   ggc gat acc atg gcg gta gcc ggt ccg gca ttc tcg cgt ctg gaa gcg      672
   Gly Asp Thr Met Ala Val Ala Gly Pro Ala Phe Ser Arg Leu Glu Ala
       210                 215                 220
   ggc gtt gcg ctg cgt aag ggc aac gaa gat tta ctg aag gcg atc gat      720
   Gly Val Ala Leu Arg Lys Gly Asn Glu Asp Leu Leu Lys Ala Ile Asp
   225                 230                 235                 240
   cag gcc att gcg gaa atg cag aaa gac ggc acc ctt aaa aag ctg tca      768
   Gln Ala Ile Ala Glu Met Gln Lys Asp Gly Thr Leu Lys Lys Leu Ser
                   245                 250                 255
   gaa aaa tgg ttt ggc gcg gac gtt act aaa taa                          801
   Glu Lys Trp Phe Gly Ala Asp Val Thr Lys
               260                 265
<210> 8
   <211> 266
   <212> PRT
   <213> Pantoea ananatis
<400> 8
   Met Ala Phe Ser Gln Ile Arg Arg Gln Val Val Thr Gly Met Met Ala
   1               5                   10                  15
   Val Ala Leu Val Ala Gly Phe Ser Val Lys Thr Phe Ala Ala Asp Asp
               20                  25                  30
   Leu Leu Ala Gln Val Lys Ser Lys Gly Glu Leu Arg Val Gly Leu Glu
           35                  40                  45
   Gly Thr Tyr Pro Pro Phe Ser Phe Gln Asp Glu Lys Gly Lys Leu Thr
       50                  55                  60
   Gly Phe Glu Val Glu Phe Ala Gln Asp Leu Ala Lys His Met Gly Val
   65                  70                  75                  80
   Lys Ala Val Leu Lys Pro Thr Lys Trp Asp Gly Met Leu Ala Ala Leu
                   85                  90                  95
   Asp Ser Lys Arg Ile Asp Val Val Ile Asn Gln Val Thr Ile Ser Asp
               100                 105                 110
   Glu Arg Lys Lys Lys Tyr Asp Phe Ser Thr Pro Tyr Thr Ile Ser Gly
           115                 120                 125
   Val Gln Ala Leu Thr Leu Lys Lys Asn Ala Gly Ser Ile Thr Lys Pro
       130                 135                 140
   Glu Asp Leu Ala Gly Lys Lys Val Gly Val Gly Leu Gly Thr Asn Tyr
   145                 150                 155                 160
   Glu Gln Trp Leu Arg Ala Asn Val Lys Gly Val Asp Ile Arg Thr Tyr
                   165                 170                 175
   Asp Asp Asp Pro Thr Lys Tyr Gln Asp Leu Arg Ser Gly Arg Val Asp
               180                 185                 190
   Ala Ile Leu Val Asp Arg Leu Ala Ala Leu Asp Leu Val Lys Lys Thr
           195                 200                 205
   Gly Asp Thr Met Ala Val Ala Gly Pro Ala Phe Ser Arg Leu Glu Ala
       210                 215                 220
   Gly Val Ala Leu Arg Lys Gly Asn Glu Asp Leu Leu Lys Ala Ile Asp
   225                 230                 235                 240
   Gln Ala Ile Ala Glu Met Gln Lys Asp Gly Thr Leu Lys Lys Leu Ser
                   245                 250                 255
   Glu Lys Trp Phe Gly Ala Asp Val Thr Lys
               260                 265
<210> 9
   <211> 1422
   <212> DNA
   <213> Escherichia coli
<220>
   <221> CDS
   <222> (301)..(1119)
<400> 9
   tcggtcaggg catggatgta caaagcgcgc aggagaagat tggtcaggtg gtggaaggct       60
   accgcaatac gaaagaagtc cgcgaactgg cgcatcgctt cggcgttgaa atgccaataa      120
   ccgaggaaat ttatcaagta ttatattgcg gaaaaaacgc gcgcgaggca gcattgactt      180
   tactaggtcg tgcacgcaag gacgagcgca gcagccacta accccaggga acctttgtta      240
   ccgctatgac ccggcccgcg cagaacgggc cggtcattat ctcatcgtgt ggagtaagca      300
   atg tcg tgt gaa gaa ctg gaa att gtc tgg aac aat att aaa gcc gaa        348
   Met Ser Cys Glu Glu Leu Glu Ile Val Trp Asn Asn Ile Lys Ala Glu
   1               5                   10                  15
   gcc aga acg ctg gcg gac tgt gag cca atg ctg gcc agt ttt tac cac        396
   Ala Arg Thr Leu Ala Asp Cys Glu Pro Met Leu Ala Ser Phe Tyr His
               20                  25                  30
   gcg acg cta ctc aag cac gaa aac ctt ggc agt gca ctg agc tac atg        444
   Ala Thr Leu Leu Lys His Glu Asn Leu Gly Ser Ala Leu Ser Tyr Met
           35                  40                  45
   ctg gcg aac aag ctg tca tcg cca att atg cct gct att gct atc cgt        492
   Leu Ala Asn Lys Leu Ser Ser Pro Ile Met Pro Ala Ile Ala Ile Arg
       50                  55                  60
   gaa gtg gtg gaa gaa gcc tac gcc gct gac ccg gaa atg atc gcc tct        540
   Glu Val Val Glu Glu Ala Tyr Ala Ala Asp Pro Glu Met Ile Ala Ser
   65                  70                  75                  80
   gcg gcc tgt gat att cag gcg gtg cgt acc cgc gac ccg gca gtc gat        588
   Ala Ala Cys Asp Ile Gln Ala Val Arg Thr Arg Asp Pro Ala Val Asp
                   85                  90                  95
   aaa tac tca acc ccg ttg tta tac ctg aag ggt ttt cat gcc ttg cag        636
   Lys Tyr Ser Thr Pro Leu Leu Tyr Leu Lys Gly Phe His Ala Leu Gln
               100                 105                 110
   gcc tat cgc atc ggt cac tgg ttg tgg aat cag ggg cgt cgc gca ctg        684
   Ala Tyr Arg Ile Gly His Trp Leu Trp Asn Gln Gly Arg Arg Ala Leu
           115                 120                 125
   gca atc ttt ctg caa aac cag gtt tct gtg acg ttc cag gtc gat att        732
   Ala Ile Phe Leu Gln Asn Gln Val Ser Val Thr Phe Gln Val Asp Ile
       130                 135                 140
   cac ccg gca gca aaa att ggt cgc ggt atc atg ctt gac cac gcg aca        780
   His Pro Ala Ala Lys Ile Gly Arg Gly Ile Met Leu Asp His Ala Thr
   145                 150                 155                 160
   ggc atc gtc gtt ggt gaa acg gcg gtg att gaa aac gac gta tcg att        828
   Gly Ile Val Val Gly Glu Thr Ala Val Ile Glu Asn Asp Val Ser Ile
                   165                 170                 175
   ctg caa tct gtg acg ctt ggc ggt acg ggt aaa tct ggt ggt gac cgt        876
   Leu Gln Ser Val Thr Leu Gly Gly Thr Gly Lys Ser Gly Gly Asp Arg
               180                 185                 190
   cac ccg aaa att cgt gaa ggt gtg atg att ggc gcg ggc gcg aaa atc        924
   His Pro Lys Ile Arg Glu Gly Val Met Ile Gly Ala Gly Ala Lys Ile
           195                 200                 205
   ctc ggc aat att gaa gtt ggg cgc ggc gcg aag att ggc gca ggt tcc        972
   Leu Gly Asn Ile Glu Val Gly Arg Gly Ala Lys Ile Gly Ala Gly Ser
       210                 215                 220
   gtg gtg ctg caa ccg gtg ccg ccg cat acc acc gcc gct ggc gtt ccg       1020
   Val Val Leu Gln Pro Val Pro Pro His Thr Thr Ala Ala Gly Val Pro
   225                 230                 235                 240
   gct cgt att gtc ggt aaa cca gac agc gat aag cca tca atg gat atg       1068
   Ala Arg Ile Val Gly Lys Pro Asp Ser Asp Lys Pro Ser Met Asp Met
                   245                 250                 255
   gac cag cat ttc aac ggt att aac cat aca ttt gag tat ggg gat ggg       1116
   Asp Gln His Phe Asn Gly Ile Asn His Thr Phe Glu Tyr Gly Asp Gly
               260                 265                 270
   atc taatgtcctg tgatcgtgcc ggatgcgatg taatcatcta tccggcctac            1169
   Ile
   agtaactaat ctctcaatac cgctcccgga taccccaact gccgccaggc ttcatacacc     1229
   actaccgaca ccgcattgga cagattcatg ctgcggctgt ccggcaccat cggaatgcga     1289
   attttttgtt cagcgggcag ggcatcaaga atgctcgctg gcaggccgcg tgtttccggg      1349
   ccgaacatca gataatcgcc atcctgatag cttacggcgc tgtgagcagg tgtacctttc     1409
   gtggtgaggg cga                                                        1422
<210> 10
   <211> 273
   <212> PRT
   <213> Escherichia coli
<400> 10
   Met Ser Cys Glu Glu Leu Glu Ile Val Trp Asn Asn Ile Lys Ala Glu
   1               5                   10                  15
   Ala Arg Thr Leu Ala Asp Cys Glu Pro Met Leu Ala Ser Phe Tyr His
               20                  25                  30
   Ala Thr Leu Leu Lys His Glu Asn Leu Gly Ser Ala Leu Ser Tyr Met
           35                  40                  45
   Leu Ala Asn Lys Leu Ser Ser Pro Ile Met Pro Ala Ile Ala Ile Arg
       50                  55                  60
   Glu Val Val Glu Glu Ala Tyr Ala Ala Asp Pro Glu Met Ile Ala Ser
   65                  70                  75                  80
   Ala Ala Cys Asp Ile Gln Ala Val Arg Thr Arg Asp Pro Ala Val Asp
                   85                  90                  95
   Lys Tyr Ser Thr Pro Leu Leu Tyr Leu Lys Gly Phe His Ala Leu Gln
               100                 105                 110
   Ala Tyr Arg Ile Gly His Trp Leu Trp Asn Gln Gly Arg Arg Ala Leu
           115                 120                 125
   Ala Ile Phe Leu Gln Asn Gln Val Ser Val Thr Phe Gln Val Asp Ile
       130                 135                 140
   His Pro Ala Ala Lys Ile Gly Arg Gly Ile Met Leu Asp His Ala Thr
   145                 150                 155                 160
   Gly Ile Val Val Gly Glu Thr Ala Val Ile Glu Asn Asp Val Ser Ile
                   165                 170                 175
   Leu Gln Ser Val Thr Leu Gly Gly Thr Gly Lys Ser Gly Gly Asp Arg
               180                 185                 190
   His Pro Lys Ile Arg Glu Gly Val Met Ile Gly Ala Gly Ala Lys Ile
           195                 200                 205
   Leu Gly Asn Ile Glu Val Gly Arg Gly Ala Lys Ile Gly Ala Gly Ser
       210                 215                 220
   Val Val Leu Gln Pro Val Pro Pro His Thr Thr Ala Ala Gly Val Pro
   225                 230                 235                 240
   Ala Arg Ile Val Gly Lys Pro Asp Ser Asp Lys Pro Ser Met Asp Met
                   245                 250                 255
   Asp Gln His Phe Asn Gly Ile Asn His Thr Phe Glu Tyr Gly Asp Gly
               260                 265                 270
   Ile
<210> 11
   <211> 669
   <212> DNA
   <213> Pantoea ananatis
<220>
   <221> CDS
   <222> (1)..(669)
<400> 11
   atg cag gaa agt tta caa ctg gtg ctg gac tca gca ccc ttt ctt ctt      48
   Met Gln Glu Ser Leu Gln Leu Val Leu Asp Ser Ala Pro Phe Leu Leu
   1               5                   10                  15
   aag ggc gcg ctg ttc acg ctg cag ctc agt att ggc ggt atg ttt ttc      96
   Lys Gly Ala Leu Phe Thr Leu Gln Leu Ser Ile Gly Gly Met Phe Phe
               20                  25                  30
   ggg ctg ata ctg ggc ttt ttg ctg gcg ctg atg cgc ctg tcc cgc ttc      144
   Gly Leu Ile Leu Gly Phe Leu Leu Ala Leu Met Arg Leu Ser Arg Phe
           35                  40                  45
   tgg ccc gtg aac tgg ctg gcg cgt atc tac gtg tcc atc ttt cgt ggc      192
   Trp Pro Val Asn Trp Leu Ala Arg Ile Tyr Val Ser Ile Phe Arg Gly
       50                  55                  60
   acg ccg ctt atc gcc cag ctg ttt atg atc tac tac ggt ctg ccg cag      240
   Thr Pro Leu Ile Ala Gln Leu Phe Met Ile Tyr Tyr Gly Leu Pro Gln
   65                  70                  75                  80
   ttt ggc att gag ctg gat ccc att ccc tcg gcg atg att ggg ctg tcg      288
   Phe Gly Ile Glu Leu Asp Pro Ile Pro Ser Ala Met Ile Gly Leu Ser
                   85                  90                  95
   ctt aac atg gcg gcc tat gcc tca gaa tct ctg cgc ggc gcg att gct      336
   Leu Asn Met Ala Ala Tyr Ala Ser Glu Ser Leu Arg Gly Ala Ile Ala
               100                 105                 110
   gcc atc gag cgc ggc cag tgg gaa gcg gcg gcc agt atc ggc atg acg      384
   Ala Ile Glu Arg Gly Gln Trp Glu Ala Ala Ala Ser Ile Gly Met Thr
           115                 120                 125
   ccg tgg caa acg ctg cgt cgg gtt gtt tta ccg cag gcc gcc cgc acc      432
   Pro Trp Gln Thr Leu Arg Arg Val Val Leu Pro Gln Ala Ala Arg Thr
       130                 135                 140
   gca ctc ccg cct ttg ggt aac agc ttt atc agt ctg gta aaa gat acc      480
   Ala Leu Pro Pro Leu Gly Asn Ser Phe Ile Ser Leu Val Lys Asp Thr
   145                 150                 155                 160
   tcg ctg gct gcc acg atc cag gtt ccg gaa ctg ttt cgt cag gcg cag      528
   Ser Leu Ala Ala Thr Ile Gln Val Pro Glu Leu Phe Arg Gln Ala Gln
                   165                 170                 175
   ctg att act tca cgc acg ttg gag gtg ttc acc atg tat ctg gcg gct      576
   Leu Ile Thr Ser Arg Thr Leu Glu Val Phe Thr Met Tyr Leu Ala Ala
               180                 185                 190
   tcg ctg atc tat tgg gtg atg gca acc gta ctg tca gca ttg cag aac      624
   Ser Leu Ile Tyr Trp Val Met Ala Thr Val Leu Ser Ala Leu Gln Asn
           195                 200                 205
   cga ctg gaa gca cac gtt aac cgt cag gat cag gag gcg aaa tga          669
   Arg Leu Glu Ala His Val Asn Arg Gln Asp Gln Glu Ala Lys
       210                 215                 220
<210> 12
   <211> 222
   <212> PRT
   <213> Pantoea ananatis
<400> 12
   Met Gln Glu Ser Leu Gln Leu Val Leu Asp Ser Ala Pro Phe Leu Leu
   1 5 10 15
   Lys Gly Ala Leu Phe Thr Leu Gln Leu Ser Ile Gly Gly Met Phe Phe
   20 25 30
   Gly Leu Ile Leu Gly Phe Leu Leu Ala Leu Met Arg Leu Ser Arg Phe
   35 40 45
   Trp Pro Val Asn Trp Leu Ala Arg Ile Tyr Val Ser Ile Phe Arg Gly
   50 55 60
   Thr Pro Leu Ile Ala Gln Leu Phe Met Ile Tyr Tyr Gly Leu Pro Gln
   65 70 75 80
   Phe Gly Ile Glu Leu Asp Pro Ile Pro Ser Ala Met Ile Gly Leu Ser
   85 90 95
   Leu Asn Met Ala Ala Tyr Ala Ser Glu Ser Leu Arg Gly Ala Ile Ala
   100 105 110
   Ala Ile Glu Arg Gly Gln Trp Glu Ala Ala Ala Ser Ile Gly Met Thr
   115 120 125
   Pro Trp Gln Thr Leu Arg Arg Val Val Leu Pro Gln Ala Ala Arg Thr
   130 135 140
   Ala Leu Pro Pro Leu Gly Asn Ser Phe Ile Ser Leu Val Lys Asp Thr
   145 150 155 160
   Ser Leu Ala Ala Thr Ile Gln Val Pro Glu Leu Phe Arg Gln Ala Gln
   165 170 175
   Leu Ile Thr Ser Arg Thr Leu Glu Val Phe Thr Met Tyr Leu Ala Ala
   180 185 190
   Ser Leu Ile Tyr Trp Val Met Ala Thr Val Leu Ser Ala Leu Gln Asn
   195 200 205
   Arg Leu Glu Ala His Val Asn Arg Gln Asp Gln Glu Ala Lys
   210 215 220
<210> 13
   <211> 783
   <212> DNA
   <213> Pantoea ananatis
<220>
   <221> CDS
   <222> (1)..(753)
<400> 13
   atg agt gct att gaa gtt cgc cag ttg gtg aaa aag ttt aac gga caa      48
   Met Ser Ala Ile Glu Val Arg Gln Leu Val Lys Lys Phe Asn Gly Gln
   1               5                   10                  15
   acg gta ctg cac ggc atc gat ctc gat gtc gcc ccg ggc gaa atc gtc      96
   Thr Val Leu His Gly Ile Asp Leu Asp Val Ala Pro Gly Glu Ile Val
               20                  25                  30
   gcg ata atc ggc ccc agt ggc tca ggt aaa acc acg ctg ttg cgc agc      144
   Ala Ile Ile Gly Pro Ser Gly Ser Gly Lys Thr Thr Leu Leu Arg Ser
           35                  40                  45
   atc aac ctc ctt gag gtc ccg gat gct ggc cgc att aaa gtg ggt gac      192
   Ile Asn Leu Leu Glu Val Pro Asp Ala Gly Arg Ile Lys Val Gly Asp
       50                  55                  60
   atc acc att gac gcc agc ctg ggc atg aac aga cag aaa gag cgg gtg      240
   Ile Thr Ile Asp Ala Ser Leu Gly Met Asn Arg Gln Lys Glu Arg Val
   65                  70                  75                  80
   cgg atg ttg cgt cag cag gtg ggt ttt gtc ttt caa aac ttc aat tta      288
   Arg Met Leu Arg Gln Gln Val Gly Phe Val Phe Gln Asn Phe Asn Leu
                   85                  90                  95
   ttt ccg cat cgt tcg gtg ctg gaa aat att att gaa ggt ccg gtg att      336
   Phe Pro His Arg Ser Val Leu Glu Asn Ile Ile Glu Gly Pro Val Ile
               100                 105                 110
   gtg aag cgg gaa gcg aaa gcg gac gcg gtt gcc cgc gcg cgc agc ctg      384
   Val Lys Arg Glu Ala Lys Ala Asp Ala Val Ala Arg Ala Arg Ser Leu
           115                 120                 125
   ctt gaa aaa gtt gga ctc aac ggc aag gaa gac agc tac cca cgg cgc      432
   Leu Glu Lys Val Gly Leu Asn Gly Lys Glu Asp Ser Tyr Pro Arg Arg
       130                 135                 140
   ctg tcc ggt ggt cag cag cag cgt gtc gcc att gcc cgt gcg ttg gcg      480
   Leu Ser Gly Gly Gln Gln Gln Arg Val Ala Ile Ala Arg Ala Leu Ala
   145                 150                 155                 160
   atg cag cca gaa gtc att ttg ttt gat gaa ccg acc tct gcg ctg gat      528
   Met Gln Pro Glu Val Ile Leu Phe Asp Glu Pro Thr Ser Ala Leu Asp
                   165                 170                 175
   ccg gaa ctg gtg ggt gaa gta ctg aac acc att cgc gga ctg gct cag      576
   Pro Glu Leu Val Gly Glu Val Leu Asn Thr Ile Arg Gly Leu Ala Gln
               180                 185                 190
   gaa aaa cgc acc atg gtt atc gtg acg cac gag atg agc ttt gcc cgc      624
   Glu Lys Arg Thr Met Val Ile Val Thr His Glu Met Ser Phe Ala Arg
           195                 200                 205
   gac gtc gcc gac cgc gcc att ttt atg gat cag ggc cgt gtt gtt gaa      672
   Asp Val Ala Asp Arg Ala Ile Phe Met Asp Gln Gly Arg Val Val Glu
       210                 215                 220
   cag ggc cct gct aag gca ctc ttc agc gat cct cag gag ccg cgt acc      720
   Gln Gly Pro Ala Lys Ala Leu Phe Ser Asp Pro Gln Glu Pro Arg Thr
   225                 230                 235                 240
   cgg cag ttt ctg aat aaa ttt ctt aat caa taa acccggtgcc gccagaagtt   773
   Arg Gln Phe Leu Asn Lys Phe Leu Asn Gln
                   245                 250
   ggcggcgtaa                                                           783
<210> 14
   <211> 250
   <212> PRT
   <213> Pantoea ananatis
<400> 14
   Met Ser Ala Ile Glu Val Arg Gln Leu Val Lys Lys Phe Asn Gly Gln
   1               5                   10                  15
   Thr Val Leu His Gly Ile Asp Leu Asp Val Ala Pro Gly Glu Ile Val
               20                  25                  30
   Ala Ile Ile Gly Pro Ser Gly Ser Gly Lys Thr Thr Leu Leu Arg Ser
           35                  40                  45
   Ile Asn Leu Leu Glu Val Pro Asp Ala Gly Arg Ile Lys Val Gly Asp
       50                  55                  60
   Ile Thr Ile Asp Ala Ser Leu Gly Met Asn Arg Gln Lys Glu Arg Val
   65                  70                  75                  80
   Arg Met Leu Arg Gln Gln Val Gly Phe Val Phe Gln Asn Phe Asn Leu
                   85                  90                  95
   Phe Pro His Arg Ser Val Leu Glu Asn Ile Ile Glu Gly Pro Val Ile
               100                 105                 110
   Val Lys Arg Glu Ala Lys Ala Asp Ala Val Ala Arg Ala Arg Ser Leu
           115                 120                 125
   Leu Glu Lys Val Gly Leu Asn Gly Lys Glu Asp Ser Tyr Pro Arg Arg
       130                 135                 140
   Leu Ser Gly Gly Gln Gln Gln Arg Val Ala Ile Ala Arg Ala Leu Ala
   145                 150                 155                 160
   Met Gln Pro Glu Val Ile Leu Phe Asp Glu Pro Thr Ser Ala Leu Asp
                   165                 170                 175
   Pro Glu Leu Val Gly Glu Val Leu Asn Thr Ile Arg Gly Leu Ala Gln
               180                 185                 190
   Glu Lys Arg Thr Met Val Ile Val Thr His Glu Met Ser Phe Ala Arg
           195                 200                 205
   Asp Val Ala Asp Arg Ala Ile Phe Met Asp Gln Gly Arg Val Val Glu
       210                 215                 220
   Gln Gly Pro Ala Lys Ala Leu Phe Ser Asp Pro Gln Glu Pro Arg Thr
   225                 230                 235                 240
   Arg Gln Phe Leu Asn Lys Phe Leu Asn Gln
                   245                 250
<210> 15
   <211> 1039
   <212> DNA
   <213> Escherichia coli
<220>
   <221> CDS
   <222> (201)..(839)
<400> 15
   tgcggataac ggtagaattt ttacgccagt attttgccga gcactacccg aatttttcac      60
   tggagcatgc ctgattaatg attcaattat cgggttgata tcaggttaaa acctgatttt     120
   ctcctttcta agccgctaca gattggttag catattcacc tttaatcgcg catgatcgaa     180
   agataattaa agaggttaat gtg ttc gct gaa tac ggg gtt ctg aat tac tgg     233
   acc tat ctg gtt ggg gcc att ttt att gtg ttg gtg cca ggg cca aat       281
   acc ctg ttt gta ctc aaa aat agc gtc agt agc ggt atg aaa ggc ggt       329
   tat ctt gcg gcc tgc ggt gta ttt att ggc gat gcg gta ttg atg ttt       377
   ctg gca tgg gct gga gtg gcg aca tta att aag acc acc ccg ata tta       425
   ttc aac att gta cgt tat ctt ggt gcg ttt tat ttg ctc tat ctg ggg       473
   agt aaa att ctt tac gcg acc ctg aag ggt aaa aat agc gag gcc aaa       521
   tcc gat gag ccc caa tac ggt gct att ttt aaa cgc gcg tta att ttg       569
   agc ctg act aat ccg aaa gcc att ttg ttc tat gtg tcg ttt ttc gta       617
   cag ttt atc gat gtt aat gcc cca cat acg gga att tca ttc ttt att       665
   ctg gcg gcg acg ctg gaa ctg gtg agt ttc tgc tat ttg agc ttc ctg       713
   att ata tct ggt gct ttt gtc acg cag tac ata cgt acc aaa aag aaa       761
   ctg gct aaa gtt ggc aac tca ctg att ggt ttg atg ttc gtg ggt ttc       809
   gct gcc cga ctg gcg acg ctg caa tcc tga tgctttcagc ccgcgttgtc         859
   gcgggcttcc catctataat cctccctgat tcttcgctga tatggtgcta aaaagtaacc     919
   aataaatggt atttaaaatg caaattatca ggcgtaccct gaaacggctg gaataaaccg     979
   ttttcagcgc attcaccgaa ggagggaaaa ggatgcttca aatcccacag aattatattc    1039
<210> 16
   <211> 212
   <212> PRT
   <213> Escherichia coli
<400> 16
<210> 17
   <211> 1779
   <212> DNA
   <213> Pantoea ananatis
<220>
   <221> CDS
   <222> (301)..(1536)
<400> 17
   gtcaaaaccc tcaaaaaata aagcaccggg cgcacaacag ggcgcccgct ttttttgatt     60
   taaaaaaact tttctcacca ggctgaaatt tggtgactta tgtcacataa ccgtcatcgg    120
   cagcgggttc gttcttctcg atgcggccaa cccacgattt tgtctggcaa agtacgtcct    180
   ctgagccctg ccatgctggc ggtcaggcaa tcgtttgtat tgccgcaggc gatttttttg    240
   atattttgac agacggctga ctgcgttcag tcctcgttga attctgaata gggttgggaa    300
   atg gca aag gta tca ctg gaa aaa gac aaa att aag ttc ctg ctg gtg      348
   Met Ala Lys Val Ser Leu Glu Lys Asp Lys Ile Lys Phe Leu Leu Val
   1               5                   10                  15
   gaa ggt gtc cat cag agc gcg ctg gaa aat ctt cgt gct gca ggt tac      396
   Glu Gly Val His Gln Ser Ala Leu Glu Asn Leu Arg Ala Ala Gly Tyr
               20                  25                  30
   acc aat att gaa ttc cac aaa ggc gca ctg gat gcc gag gcg tta aaa      444
   Thr Asn Ile Glu Phe His Lys Gly Ala Leu Asp Ala Glu Ala Leu Lys
           35                  40                  45
   gct tcc gct cgc gat gcg cat ttt atc ggt atc cgt tcc cgt tcc caa      492
   Ala Ser Ala Arg Asp Ala His Phe Ile Gly Ile Arg Ser Arg Ser Gln
       50                  55                  60
   ctg acc gaa gag att ttt gcc gct gca gaa aaa ctg gta gcg gtg ggc      540
   Leu Thr Glu Glu Ile Phe Ala Ala Ala Glu Lys Leu Val Ala Val Gly
   65                  70                  75                  80
   tgt ttc tgt atc gga acg aat cag gtt gat tta aat gcc gca gcg aaa      588
   Cys Phe Cys Ile Gly Thr Asn Gln Val Asp Leu Asn Ala Ala Ala Lys
                   85                  90                  95
   cgc ggt atc ccg gtt ttt aac gca cct ttc tca aat acg cgc tct gtg      636
   Arg Gly Ile Pro Val Phe Asn Ala Pro Phe Ser Asn Thr Arg Ser Val
               100                 105                 110
   gcc gag ctg gtt att ggc gag atg ctg ctg atg ctg cgc ggt gtt ccg      684
   Ala Glu Leu Val Ile Gly Glu Met Leu Leu Met Leu Arg Gly Val Pro
           115                 120                 125
   gaa gcg aat gcc aaa gcg cac cgt ggt atc tgg aat aaa atc gcc aaa      732
   Glu Ala Asn Ala Lys Ala His Arg Gly Ile Trp Asn Lys Ile Ala Lys
       130                 135                 140
   ggc tct ttt gaa gcg cgc ggt aaa aag ctg ggt atc att ggc tat ggc      780
   Gly Ser Phe Glu Ala Arg Gly Lys Lys Leu Gly Ile Ile Gly Tyr Gly
   145                 150                 155                 160
   cat atc ggt atg caa ctg ggc gtg ctg gca gaa agt ctg ggc atg cac      828
   His Ile Gly Met Gln Leu Gly Val Leu Ala Glu Ser Leu Gly Met His
                   165                 170                 175
   gtt tac ttc tat gac atc gaa aac aag ctg ccg ttg ggc aac gca tca      876
   Val Tyr Phe Tyr Asp Ile Glu Asn Lys Leu Pro Leu Gly Asn Ala Ser
               180                 185                 190
   cag gtt cgt agc ctg acg cag ttg cta aat atg agt gac gtt gtc agc      924
   Gln Val Arg Ser Leu Thr Gln Leu Leu Asn Met Ser Asp Val Val Ser
           195                 200                 205
   ctg cat gtc ccg gaa acc gcc tct acg caa aat atg att tct gcc aat      972
   Leu His Val Pro Glu Thr Ala Ser Thr Gln Asn Met Ile Ser Ala Asn
       210                 215                 220
   gag ctg gct cag atg aag cct ggc ggc ctg ctg ata aat gcc tca cgc     1020
   Glu Leu Ala Gln Met Lys Pro Gly Gly Leu Leu Ile Asn Ala Ser Arg
   225                 230                 235                 240
   ggc acc gtg gta gat att cct gct ttg tgc gaa gcg ctg gcc agc aag     1068
   Gly Thr Val Val Asp Ile Pro Ala Leu Cys Glu Ala Leu Ala Ser Lys
                   245                 250                 255
   cag gtt ggt ggc gct gcg att gat gtg ttc cct gta gag ccg gcg acc     1116
   Gln Val Gly Gly Ala Ala Ile Asp Val Phe Pro Val Glu Pro Ala Thr
               260                 265                 270
   aac agc gat ccg ttt gtt tcc cca ctg agc gaa ttc gac aac gtt atc     1164
   Asn Ser Asp Pro Phe Val Ser Pro Leu Ser Glu Phe Asp Asn Val Ile
           275                 280                 285
   ctg acg ccg cac atc ggg gga tcg acg gaa gaa gct cag gag aat atc     1212
   Leu Thr Pro His Ile Gly Gly Ser Thr Glu Glu Ala Gln Glu Asn Ile
       290                 295                 300
   ggg att gaa gtc gcg ggc aag ctg gcg aaa tat tcg gat aac ggt tca      1260
   Gly Ile Glu Val Ala Gly Lys Leu Ala Lys Tyr Ser Asp Asn Gly Ser
   305                 310                 315                 320
   acg ctg tcc gcc gtc aat ttc ccg gaa gtg tca ttg ccg atg cac ggc      1308
   Thr Leu Ser Ala Val Asn Phe Pro Glu Val Ser Leu Pro Met His Gly
                   325                 330                 335
   att agc gcc agt cgt ctg ctg cat att cac gaa aac cgt ccg ggc gtt      1356
   Ile Ser Ala Ser Arg Leu Leu His Ile His Glu Asn Arg Pro Gly Val
               340                 345                 350
   ctc acc gcg atc aac cag att ttc gct gaa caa ggc atc aac att gcc      1404
   Leu Thr Ala Ile Asn Gln Ile Phe Ala Glu Gln Gly Ile Asn Ile Ala
           355                 360                 365
   gct cag tac ctg caa acc tct ccg atg atg ggt tat gtg gtc atc gac      1452
   Ala Gln Tyr Leu Gln Thr Ser Pro Met Met Gly Tyr Val Val Ile Asp
       370                 375                 380
   att gat gct gag cac gaa ctg gca gag aaa gct ctg caa ctg atg aag      1500
   Ile Asp Ala Glu His Glu Leu Ala Glu Lys Ala Leu Gln Leu Met Lys
   385                 390                 395                 400
   gcg att ccg gga acg att cgc gcc cgc ctg ctt tac tgatcccacg           1546
   Ala Ile Pro Gly Thr Ile Arg Ala Arg Leu Leu Tyr
                   405                 410
   ctgtcaccta cccgggcaca caagcatgcc cgggtttatt catcccatag ccacagtttt    1606
   gatggcgtca gcacggccgg caaaggaatg tcccacgccg ctgtaggcag cgcgtcaacc    1666
   cgctgacagt catgagcgat gcccaccggt aaaaacccat gctgtttcca gttctgtaag    1726
   gtgcgatcgt agaagccgcc ccccattcct aaacgctgtc cggcgcgatc gaa           1779
<210> 18
   <211> 412
   <212> PRT
   <213> Pantoea ananatis
<400> 18
   Met Ala Lys Val Ser Leu Glu Lys Asp Lys Ile Lys Phe Leu Leu Val
   1 5 10 15
   Glu Gly Val His Gln Ser Ala Leu Glu Asn Leu Arg Ala Ala Gly Tyr
   20 25 30
   Thr Asn Ile Glu Phe His Lys Gly Ala Leu Asp Ala Glu Ala Leu Lys
   35 40 45
   Ala Ser Ala Arg Asp Ala His Phe Ile Gly Ile Arg Ser Arg Ser Gln
   50 55 60
   Leu Thr Glu Glu Ile Phe Ala Ala Ala Glu Lys Leu Val Ala Val Gly
   65 70 75 80
   Cys Phe Cys Ile Gly Thr Asn Gln Val Asp Leu Asn Ala Ala Ala Lys
   85 90 95
   Arg Gly Ile Pro Val Phe Asn Ala Pro Phe Ser Asn Thr Arg Ser Val
   100 105 110
   Ala Glu Leu Val Ile Gly Glu Met Leu Leu Met Leu Arg Gly Val Pro
   115 120 125
   Glu Ala Asn Ala Lys Ala His Arg Gly Ile Trp Asn Lys Ile Ala Lys
   130 135 140
   Gly Ser Phe Glu Ala Arg Gly Lys Lys Leu Gly Ile Ile Gly Tyr Gly
   145 150 155 160
   His Ile Gly Met Gln Leu Gly Val Leu Ala Glu Ser Leu Gly Met His
   165 170 175
   Val Tyr Phe Tyr Asp Ile Glu Asn Lys Leu Pro Leu Gly Asn Ala Ser
   180 185 190
   Gln Val Arg Ser Leu Thr Gln Leu Leu Asn Met Ser Asp Val Val Ser
   195 200 205
   Leu His Val Pro Glu Thr Ala Ser Thr Gln Asn Met Ile Ser Ala Asn
   210 215 220
   Glu Leu Ala Gln Met Lys Pro Gly Gly Leu Leu Ile Asn Ala Ser Arg
   225 230 235 240
   Gly Thr Val Val Asp Ile Pro Ala Leu Cys Glu Ala Leu Ala Ser Lys
   245 250 255
   Gln Val Gly Gly Ala Ala Ile Asp Val Phe Pro Val Glu Pro Ala Thr
   260 265 270
   Asn Ser Asp Pro Phe Val Ser Pro Leu Ser Glu Phe Asp Asn Val Ile
   275 280 285
   Leu Thr Pro His Ile Gly Gly Ser Thr Glu Glu Ala Gln Glu Asn Ile
   290 295 300
   Gly Ile Glu Val Ala Gly Lys Leu Ala Lys Tyr Ser Asp Asn Gly Ser
   305 310 315 320
   Thr Leu Ser Ala Val Asn Phe Pro Glu Val Ser Leu Pro Met His Gly
   325 330 335
   Ile Ser Ala Ser Arg Leu Leu His Ile His Glu Asn Arg Pro Gly Val
   340 345 350
   Leu Thr Ala Ile Asn Gln Ile Phe Ala Glu Gln Gly Ile Asn Ile Ala
   355 360 365
   Ala Gln Tyr Leu Gln Thr Ser Pro Met Met Gly Tyr Val Val Ile Asp
   370 375 380
   Ile Asp Ala Glu His Glu Leu Ala Glu Lys Ala Leu Gln Leu Met Lys
   385 390 395 400
   Ala Ile Pro Gly Thr Ile Arg Ala Arg Leu Leu Tyr
   405 410
<210> 19
   <211> 4403
   <212> DNA
   <213> Pantoea ananatis
<220>
   <221> CDS
   <222> (301)..(1311)
<220>
   <221> CDS
   <222> (1317)..(2147)
<220>
   <221> CDS
   <222> (2150)..(3022)
<400> 19
   tacagcggaa cctggcacgg gccagaaggg ttgatgccgt cggatgacac actcaagagc      60
   tggacgctca gcaaaattgt ctggcagcgc taagtctttt ttcacaccgc tcaaccgcag     120
   ggcataaccg gccctgcgcg tccaattctg tttttcgtct gtcttttccc gccgccttat     180
   gcctttttcg actttgaaat cagcaaacga tatataaaac cgttacgggt ttacgctgag      240
   ttataaataa actgctgtat ctgcagatga gatctgcatc aaatttcctc agggtgaacc     300
   atg acc tta cca gcg atg aaa aaa atc gtg agc gga ctc gca ctg tcg       348
   Met Thr Leu Pro Ala Met Lys Lys Ile Val Ser Gly Leu Ala Leu Ser
   1               5                   10                  15
   ctg agt ctg gcc ggt gcc gca aac gcg acc gag ctg ttg aac agc tct       396
   Leu Ser Leu Ala Gly Ala Ala Asn Ala Thr Glu Leu Leu Asn Ser Ser
               20                  25                  30
   tac gat gtc gca cgt gaa tta ttt gtc gcc ctg aat gcg cct ttt gtc       444
   Tyr Asp Val Ala Arg Glu Leu Phe Val Ala Leu Asn Ala Pro Phe Val
           35                  40                  45
   agc cag tgg gat gcc agc cat cct gac gac aag ctg acc att aag atg       492
   Ser Gln Trp Asp Ala Ser His Pro Asp Asp Lys Leu Thr Ile Lys Met
       50                  55                  60
   tcc cat gcc ggg tca tcc aaa cag gcg ctg gcg atc ctg caa ggc ctg       540
   Ser His Ala Gly Ser Ser Lys Gln Ala Leu Ala Ile Leu Gln Gly Leu
   65                  70                  75                  80
   cgt gcc gat gtg gtg acc tat aac cag gtc acc gat gtg cag gtg ctg       588
   Arg Ala Asp Val Val Thr Tyr Asn Gln Val Thr Asp Val Gln Val Leu
                   85                  90                  95
   cac gat aaa ggc aaa ctg atc cct gcc gac tgg caa acc cgc ctg ccg       636
   His Asp Lys Gly Lys Leu Ile Pro Ala Asp Trp Gln Thr Arg Leu Pro
               100                 105                 110
   aat aac agt tcg ccg ttt tac tcc acc atg gcg ttc ctg gtg cgc aag       684
   Asn Asn Ser Ser Pro Phe Tyr Ser Thr Met Ala Phe Leu Val Arg Lys
           115                 120                 125
   gga aac cca aag cag att cac gac tgg tcc gat tta acc cgt gac gat       732
   Gly Asn Pro Lys Gln Ile His Asp Trp Ser Asp Leu Thr Arg Asp Asp
       130                 135                 140
   gtg aag ctg att ttt cct aat ccc aaa acc tcg ggc aac gga cgt tat       780
   Val Lys Leu Ile Phe Pro Asn Pro Lys Thr Ser Gly Asn Gly Arg Tyr
   145 150 155 160
   acc tat ctt gct gcc tgg ggc gcc gcc agc aac act gac ggg ggc gat 828
   Thr Tyr Leu Ala Ala Trp Gly Ala Ala Ser Asn Thr Asp Gly Gly Asp
   165 170 175
   cag gct aaa acc cgc gct ttt atg aca aaa ttt ctg aaa aat gtt gaa 876
   Gln Ala Lys Thr Arg Ala Phe Met Thr Lys Phe Leu Lys Asn Val Glu
   180 185 190
   gtc ttc gat acc ggt ggc cga ggt gct acg acc acc ttt gct gaa cgc 924
   Val Phe Asp Thr Gly Gly Arg Gly Ala Thr Thr Thr Phe Ala Glu Arg
   195 200 205
   ggt ctg ggc gat gtg ttg atc agt ttt gag tct gaa gtg aat aac atc 972
   Gly Leu Gly Asp Val Leu Ile Ser Phe Glu Ser Glu Val Asn Asn Ile
   210 215 220
   cgc aac cag tac ggc aaa gac gac tac gaa gtc gtg gtg cct aaa acc 1020
   Arg Asn Gln Tyr Gly Lys Asp Asp Tyr Glu Val Val Val Pro Lys Thr
   225 230 235 240
   gat att ctc gcg gag ttt ccc gtt gcc tgg gta gat aaa aac gtc gag 1068
   Asp Ile Leu Ala Glu Phe Pro Val Ala Trp Val Asp Lys Asn Val Glu
   245 250 255
   cag aat aaa aca gcc gat gca gcg aaa gcc tat ctg acc tgg ctg tat 1116
   Gln Asn Lys Thr Ala Asp Ala Ala Lys Ala Tyr Leu Thr Trp Leu Tyr
   260 265 270
   tct cct gcg gcg cag aaa att att acg gat ttc tat tac cgc gtg aac 1164
   Ser Pro Ala Ala Gln Lys Ile Ile Thr Asp Phe Tyr Tyr Arg Val Asn
   275 280 285
   aat ccg cag tta atg gcg cag caa aaa gcc cgt ttt cct gcc acg aac 1212
   Asn Pro Gln Leu Met Ala Gln Gln Lys Ala Arg Phe Pro Ala Thr Asn
   290 295 300
   ctg ttt cgt gtt gaa gac att ttt ggc ggc tgg gat aac gtg atg aaa 1260
   Leu Phe Arg Val Glu Asp Ile Phe Gly Gly Trp Asp Asn Val Met Lys
   305 310 315 320
   acc cat ttc gcc agc ggt ggc gag cta gac cag tta tta gcg gcg ggg 1308
   Thr His Phe Ala Ser Gly Gly Glu Leu Asp Gln Leu Leu Ala Ala Gly
   325 330 335
   cgg tgatc atg ttt gca gcc agc caa aaa cgc gtc ctg ccc ggt ttc ggt 1358
   Arg Met Phe Ala Ala Ser Gln Lys Arg Val Leu Pro Gly Phe Gly
   340 345 350
   ctc agc ctg ggc acc agc ctg ctc ttt acc tgt ctg gtg ctg ctg ctg 1406
   Leu Ser Leu Gly Thr Ser Leu Leu Phe Thr Cys Leu Val Leu Leu Leu
   355 360 365
   cca atc agc gca ctg att atg cag ctg tcg cag atg acg ttg cag caa 1454
   Pro Ile Ser Ala Leu Ile Met Gln Leu Ser Gln Met Thr Leu Gln Gln
   370 375 380
   tac tgg gac gtg gtc acc aat ccg cag ctc atc gcg gcc tat aag gtc 1502
   Tyr Trp Asp Val Val Thr Asn Pro Gln Leu Ile Ala Ala Tyr Lys Val
   385 390 395
   acg ctg ctg tcg gcc ggt gtg gcc tca ctg ttt aat gcc gta ttc ggc 1550
   Thr Leu Leu Ser Ala Gly Val Ala Ser Leu Phe Asn Ala Val Phe Gly
   400 405 410 415
   atg tta atg gcg tgg atc tta acg cgt tac cgt ttt ccg ggc cgc acg 1598
   Met Leu Met Ala Trp Ile Leu Thr Arg Tyr Arg Phe Pro Gly Arg Thr
   420 425 430
   ctg ctc gat ggt ctg atg gat ctg ccg ttt gcg ctg ccg acc gcg gtt 1646
   Leu Leu Asp Gly Leu Met Asp Leu Pro Phe Ala Leu Pro Thr Ala Val
   435 440 445
   gct ggc ctg acg ctg gcc ggt ctg ttt tcc gtg aac ggc tgg tac gga 1694
   Ala Gly Leu Thr Leu Ala Gly Leu Phe Ser Val Asn Gly Trp Tyr Gly
   450 455 460
   caa tgg ttc gcg cat ttt gat atc aag atc tcc tat acc tgg atc ggt 1742
   Gln Trp Phe Ala His Phe Asp Ile Lys Ile Ser Tyr Thr Trp Ile Gly
   465 470 475
   atc gcg ctc gcg atg gcc ttc acc agt att ccg ttt gtg gtg cgt acc 1790
   Ile Ala Leu Ala Met Ala Phe Thr Ser Ile Pro Phe Val Val Arg Thr
   480 485 490 495
   gtg cag ccg gtg ctg gaa gag ctg ggg cct gaa tat gag gaa gcg gct 1838
   Val Gln Pro Val Leu Glu Glu Leu Gly Pro Glu Tyr Glu Glu Ala Ala
   500 505 510
   caa acg ctg ggc gcc acg ccc tgg cag agc ttc cgc cgg gtc gtt ctg 1886
   Gln Thr Leu Gly Ala Thr Pro Trp Gln Ser Phe Arg Arg Val Val Leu
               515                 520                 525
   cct gaa gtg gca ccg gcc tta ctt gcg ggc acc gcg ctg tcg ttt acc      1934
   Pro Glu Val Ala Pro Ala Leu Leu Ala Gly Thr Ala Leu Ser Phe Thr
           530                 535                 540
   cgc agc ctg ggc gag ttt ggt gcg gta atc ttt att gcc ggc aac atc      1982
   Arg Ser Leu Gly Glu Phe Gly Ala Val Ile Phe Ile Ala Gly Asn Ile
       545                 550                 555
   gct tgg aaa acc gaa gtg acc tcg ctg atg atc ttc gtg cgc ctg cag      2030
   Ala Trp Lys Thr Glu Val Thr Ser Leu Met Ile Phe Val Arg Leu Gln
   560                 565                 570                 575
   gag ttt gac tat ccg gca gcc agc gcc att gcc tcg gtc att ctg gcg      2078
   Glu Phe Asp Tyr Pro Ala Ala Ser Ala Ile Ala Ser Val Ile Leu Ala
                   580                 585                 590
   gca tca ctg ctg tta ctt ttc gct atc aat acc tta caa agc cgc ttt      2126
   Ala Ser Leu Leu Leu Leu Phe Ala Ile Asn Thr Leu Gln Ser Arg Phe
               595                 600                 605
   ggt cgt cgt ctg gga ggc cat ta atg gca gag att tcg caa ctc aat      2173
   Gly Arg Arg Leu Gly Gly His    Met Ala Glu Ile Ser Gln Leu Asn
           610                     615                 620
   cat gcc gac cgc cag cct gtt aac tgg gcc aag tgg ctg ctt att ggt      2221
   His Ala Asp Arg Gln Pro Val Asn Trp Ala Lys Trp Leu Leu Ile Gly
               625                 630                 635
   att ggt gcg ctg ata tcc ttg ctg ctg ctg gtc gtg ccg atg gtg tcc      2269
   Ile Gly Ala Leu Ile Ser Leu Leu Leu Leu Val Val Pro Met Val Ser
       640                 645                 650
   atc ttc tgg gag gcc ctg cat aaa gga ctg ggc gtc acc tta agt aat      2317
   Ile Phe Trp Glu Ala Leu His Lys Gly Leu Gly Val Thr Leu Ser Asn
   655                 660                 665                 670
   ctg acc gac agc gac atg ctc cat gcc ata tgg ctc acg gtg ctg gtc      2365
   Leu Thr Asp Ser Asp Met Leu His Ala Ile Trp Leu Thr Val Leu Val
                   675                 680                 685
   gca ttg att acc gtg ccg gtg aat tta gtg ttc ggc acg ctg ctg gcc      2413
   Ala Leu Ile Thr Val Pro Val Asn Leu Val Phe Gly Thr Leu Leu Ala
               690                 695                 700
   tgg ctg gtg aca cgc ttt acc ttt ccg gga cgt cag ctg ctt ttg acg      2461
   Trp Leu Val Thr Arg Phe Thr Phe Pro Gly Arg Gln Leu Leu Leu Thr
           705                 710                 715
   ctg ttc gat att ccc ttt gcg gta tcg cct gtg gtc gcc ggt ctg atg      2509
   Leu Phe Asp Ile Pro Phe Ala Val Ser Pro Val Val Ala Gly Leu Met
       720                 725                 730
   tat ctc ctg ttc tgg ggc att aac ggc ccg gcg ggc ggc tgg ctg gat      2557
   Tyr Leu Leu Phe Trp Gly Ile Asn Gly Pro Ala Gly Gly Trp Leu Asp
   735                 740                 745                 750
   gcc cat aat att cag gtg atg ttc tcc tgg cct ggc atg gtg ctg gtc      2605
   Ala His Asn Ile Gln Val Met Phe Ser Trp Pro Gly Met Val Leu Val
                   755                 760                 765
   acc gtc ttc gtt acc tgt ccg ttt gtg gtg cgc gaa ctg gtg ccg gtg      2653
   Thr Val Phe Val Thr Cys Pro Phe Val Val Arg Glu Leu Val Pro Val
               770                 775                 780
   atg ctg agc cag ggc agt cat gaa gat gaa gcc gcg gtg ctg tta ggt      2701
   Met Leu Ser Gln Gly Ser His Glu Asp Glu Ala Ala Val Leu Leu Gly
           785                 790                 795
   gcc tcg ggc tgg cag atg ttc cgt cgc gtg acg ctg ccg aat att cgc      2749
   Ala Ser Gly Trp Gln Met Phe Arg Arg Val Thr Leu Pro Asn Ile Arg
       800                 805                 810
   tgg gcc atg ctg tat ggc gtc gtg ctg acc aac gcc cgc gcg att ggt      2797
   Trp Ala Met Leu Tyr Gly Val Val Leu Thr Asn Ala Arg Ala Ile Gly
   815                 820                 825                 830
   gag ttt ggc gcg gtt tcc gtg gtt tcg ggt tct att cgc ggt gaa acc      2845
   Glu Phe Gly Ala Val Ser Val Val Ser Gly Ser Ile Arg Gly Glu Thr
                   835                 840                 845
   tac act tta ccg ctt cag gtt gaa tta ctg cat cag gat tac aac acg      2893
   Tyr Thr Leu Pro Leu Gln Val Glu Leu Leu His Gln Asp Tyr Asn Thr
               850                 855                 860
   gtg ggc gcc ttt act gcc gca gcc tta ctg acc gtg atg gca atc gtg      2941
   Val Gly Ala Phe Thr Ala Ala Ala Leu Leu Thr Val Met Ala Ile Val
           865                 870                 875
   acg ctg ttt ctg aaa agc att gtg caa tgg cgt tta gag caa cag cac      2989
   Thr Leu Phe Leu Lys Ser Ile Val Gln Trp Arg Leu Glu Gln Gln His
       880                 885                 890
   aaa cgc ctg caa ctg gag gac aat cat gag cat tgagattaac cagatcaaca    3042
   Lys Arg Leu Gln Leu Glu Asp Asn His Glu His
   895                 900                 905
   aatcctttgg tcgcacagcg gtgctgaacg atatctcact ggatattcct tctggccaga    3102
   tggtggcctt actggggccg tccggttccg gtaaaaccac gctgctgcgc atcattgctg    3162
   gactggaaca tcagaacagc ggtcagattc gttttcacga ccacgatgtc agccgcctgc    3222
   acgcccgcga tcgccaggtc ggatttgtct tccagcacta tgcgctgttc cgtcatatga    3282
   cggtcttcga caatattgcc tttggcctga ccgtgctgcc gcgccgtgag cgtccgtcca    3342
   gtgcggaaat taaaaaacgc gtcacgcgcc tgctggagat ggtgcagctt tcccatctgg    3402
   cgaaccgttt cccggcccag ctttcgggag ggcagaagca gcgcgtcgcg ctggcaagag    3462
   ccctggccgt ggaaccgcaa atcctgttgc tggatgagcc ctttggtgcg ctggacgctc    3522
   aggtgcgtaa agagctgcgc cgttggttac gtcagctgca cgaagaattg aagttcacca    3582
   gcgtgttcgt cacccacgat caggaagagg cgatggaagt ggccgatcgc gtggtggtga    3642
   tgagccaggg cagcatcgaa caggtgggga cgccggatga agtctggcgc gatcccgcca    3702
   cgcgcttcgt gctggaattc ctgggtgagg ttaaccgctt cgacggtgaa gtgcatggtt    3762
   ctcagttcca tgtcggggcg caccactggc cgttaggcta tacctctgca catcagggcg    3822
   cggtcgatct gttcctgcgc ccgtgggaaa tcgacgtttc gcgcagaagt agcctggaaa    3882
   cgccgctgcc cgttcaggtc ttagaagtga gtcctcgtgg tcacttctgg cagctggtgg    3942
   tgcagccaac gggatggcag agcgagccct tctcgctggt ctttgacggt gaacagaccg    4002
   cgccgttgcg cggcgagcgc ctgttcgtgg ggctgcagca ggccagactg taccagggcg    4062
   cgacaccgtt acgggcggtt gcctttgcac acagcgcctg ataggttgag tgaatgttaa    4122
   acgcccggag gcgcttcccg cgatccgggc tttttaatgg caaggtttgt aacctgtaga    4182
   cctgataaga cgcgcaagcg tcgcatcagg caacaccacg tatggataga gatcgtgagt    4242
   acattagaac aaacaatagg caatacgcct ctggtgaagt tgcagcgaat ggggccggat    4302
   aacggcagtg aagtgtggtt aaaactggaa ggcaataacc cggcaggttc ggtgaaagat    4362
   cgtgcggcac tttcgatgat cgtcgaggcg gaaaagcgcg g                        4403
<210> 20
   <211> 337
   <212> PRT
   <213> Pantoea ananatis
<400> 20
   Met Thr Leu Pro Ala Met Lys Lys Ile Val Ser Gly Leu Ala Leu Ser
   1               5                   10                  15
   Leu Ser Leu Ala Gly Ala Ala Asn Ala Thr Glu Leu Leu Asn Ser Ser
               20                  25                  30
   Tyr Asp Val Ala Arg Glu Leu Phe Val Ala Leu Asn Ala Pro Phe Val
           35                  40                  45
   Ser Gln Trp Asp Ala Ser His Pro Asp Asp Lys Leu Thr Ile Lys Met
       50                  55                  60
   Ser His Ala Gly Ser Ser Lys Gln Ala Leu Ala Ile Leu Gln Gly Leu
   65                  70                  75                  80
   Arg Ala Asp Val Val Thr Tyr Asn Gln Val Thr Asp Val Gln Val Leu
                   85                  90                  95
   His Asp Lys Gly Lys Leu Ile Pro Ala Asp Trp Gln Thr Arg Leu Pro
               100                 105                 110
   Asn Asn Ser Ser Pro Phe Tyr Ser Thr Met Ala Phe Leu Val Arg Lys
           115                 120                 125
   Gly Asn Pro Lys Gln Ile His Asp Trp Ser Asp Leu Thr Arg Asp Asp
       130                 135                 140
   Val Lys Leu Ile Phe Pro Asn Pro Lys Thr Ser Gly Asn Gly Arg Tyr
   145                 150                 155                 160
   Thr Tyr Leu Ala Ala Trp Gly Ala Ala Ser Asn Thr Asp Gly Gly Asp
                   165                 170                 175
   Gln Ala Lys Thr Arg Ala Phe Met Thr Lys Phe Leu Lys Asn Val Glu
               180                 185                 190
   Val Phe Asp Thr Gly Gly Arg Gly Ala Thr Thr Thr Phe Ala Glu Arg
           195                 200                 205
   Gly Leu Gly Asp Val Leu Ile Ser Phe Glu Ser Glu Val Asn Asn Ile
       210                 215                 220
   Arg Asn Gln Tyr Gly Lys Asp Asp Tyr Glu Val Val Val Pro Lys Thr
   225                 230                 235                 240
   Asp Ile Leu Ala Glu Phe Pro Val Ala Trp Val Asp Lys Asn Val Glu
                   245                 250                 255
   Gln Asn Lys Thr Ala Asp Ala Ala Lys Ala Tyr Leu Thr Trp Leu Tyr
               260                 265                 270
   Ser Pro Ala Ala Gln Lys Ile Ile Thr Asp Phe Tyr Tyr Arg Val Asn
           275                 280                 285
   Asn Pro Gln Leu Met Ala Gln Gln Lys Ala Arg Phe Pro Ala Thr Asn
       290                 295                 300
   Leu Phe Arg Val Glu Asp Ile Phe Gly Gly Trp Asp Asn Val Met Lys
   305                 310                 315                 320
   Thr His Phe Ala Ser Gly Gly Glu Leu Asp Gln Leu Leu Ala Ala Gly
                   325                 330                 335
   Arg
<210> 21
   <211> 277
   <212> PRT
   <213> Pantoea ananatis
<400> 21
   Met Phe Ala Ala Ser Gln Lys Arg Val Leu Pro Gly Phe Gly Leu Ser
   1               5                   10                  15
   Leu Gly Thr Ser Leu Leu Phe Thr Cys Leu Val Leu Leu Leu Pro Ile
               20                  25                  30
   Ser Ala Leu Ile Met Gln Leu Ser Gln Met Thr Leu Gln Gln Tyr Trp
           35                  40                  45
   Asp Val Val Thr Asn Pro Gln Leu Ile Ala Ala Tyr Lys Val Thr Leu
       50                  55                  60
   Leu Ser Ala Gly Val Ala Ser Leu Phe Asn Ala Val Phe Gly Met Leu
   65                  70                  75                  80
   Met Ala Trp Ile Leu Thr Arg Tyr Arg Phe Pro Gly Arg Thr Leu Leu
                   85                  90                  95
   Asp Gly Leu Met Asp Leu Pro Phe Ala Leu Pro Thr Ala Val Ala Gly
               100                 105                 110
   Leu Thr Leu Ala Gly Leu Phe Ser Val Asn Gly Trp Tyr Gly Gln Trp
           115                 120                 125
   Phe Ala His Phe Asp Ile Lys Ile Ser Tyr Thr Trp Ile Gly Ile Ala
       130                 135                 140
   Leu Ala Met Ala Phe Thr Ser Ile Pro Phe Val Val Arg Thr Val Gln
   145                 150                 155                 160
   Pro Val Leu Glu Glu Leu Gly Pro Glu Tyr Glu Glu Ala Ala Gln Thr
                   165                 170                 175
   Leu Gly Ala Thr Pro Trp Gln Ser Phe Arg Arg Val Val Leu Pro Glu
               180                 185                 190
   Val Ala Pro Ala Leu Leu Ala Gly Thr Ala Leu Ser Phe Thr Arg Ser
           195                 200                 205
   Leu Gly Glu Phe Gly Ala Val Ile Phe Ile Ala Gly Asn Ile Ala Trp
       210                 215                 220
   Lys Thr Glu Val Thr Ser Leu Met Ile Phe Val Arg Leu Gln Glu Phe
   225                 230                 235                 240
   Asp Tyr Pro Ala Ala Ser Ala Ile Ala Ser Val Ile Leu Ala Ala Ser
                   245                 250                 255
   Leu Leu Leu Leu Phe Ala Ile Asn Thr Leu Gln Ser Arg Phe Gly Arg
               260                 265                 270
   Arg Leu Gly Gly His
           275
<210> 22
   <211> 291
   <212> PRT
   <213> Pantoea ananatis
<400> 22
   Met Ala Glu Ile Ser Gln Leu Asn His Ala Asp Arg Gln Pro Val Asn
   1               5                   10                  15
   Trp Ala Lys Trp Leu Leu Ile Gly Ile Gly Ala Leu Ile Ser Leu Leu
               20                  25                  30
   Leu Leu Val Val Pro Met Val Ser Ile Phe Trp Glu Ala Leu His Lys
           35                  40                  45
   Gly Leu Gly Val Thr Leu Ser Asn Leu Thr Asp Ser Asp Met Leu His
                   50                  55                  60
   Ala Ile Trp Leu Thr Val Leu Val Ala Leu Ile Thr Val Pro Val Asn
   65                  70                  75                  80
   Leu Val Phe Gly Thr Leu Leu Ala Trp Leu Val Thr Arg Phe Thr Phe
                   85                  90                  95
   Pro Gly Arg Gln Leu Leu Leu Thr Leu Phe Asp Ile Pro Phe Ala Val
               100                 105                 110
   Ser Pro Val Val Ala Gly Leu Met Tyr Leu Leu Phe Trp Gly Ile Asn
           115                 120                 125
   Gly Pro Ala Gly Gly Trp Leu Asp Ala His Asn Ile Gln Val Met Phe
       130                 135                 140
   Ser Trp Pro Gly Met Val Leu Val Thr Val Phe Val Thr Cys Pro Phe
   145                 150                 155                 160
   Val Val Arg Glu Leu Val Pro Val Met Leu Ser Gln Gly Ser His Glu
                   165                 170                 175
   Asp Glu Ala Ala Val Leu Leu Gly Ala Ser Gly Trp Gln Met Phe Arg
               180                 185                 190
   Arg Val Thr Leu Pro Asn Ile Arg Trp Ala Met Leu Tyr Gly Val Val
           195                 200                 205
   Leu Thr Asn Ala Arg Ala Ile Gly Glu Phe Gly Ala Val Ser Val Val
       210                 215                 220
   Ser Gly Ser Ile Arg Gly Glu Thr Tyr Thr Leu Pro Leu Gln Val Glu
   225                 230                 235                 240
   Leu Leu His Gln Asp Tyr Asn Thr Val Gly Ala Phe Thr Ala Ala Ala
                   245                 250                 255
   Leu Leu Thr Val Met Ala Ile Val Thr Leu Phe Leu Lys Ser Ile Val
               260                 265                 270
   Gln Trp Arg Leu Glu Gln Gln His Lys Arg Leu Gln Leu Glu Asp Asn
           275                 280                 285
   His Glu His
       290
<210> 23
   <211> 1403
   <212> DNA
   <213> Pantoea ananatis
<220>
   <221> CDS
   <222> (15)..(1103)
<400> 23
   actggaggac aatc atg agc att gag att aac cag atc aac aaa tcc ttt       50
                   Met Ser Ile Glu Ile Asn Gln Ile Asn Lys Ser Phe
                   1               5                   10
   ggt cgc aca gcg gtg ctg aac gat atc tca ctg gat att cct tct ggc       98
   Gly Arg Thr Ala Val Leu Asn Asp Ile Ser Leu Asp Ile Pro Ser Gly
           15                  20                  25
   cag atg gtg gcc tta ctg ggg ccg tcc ggt tcc ggt aaa acc acg ctg      146
   Gln Met Val Ala Leu Leu Gly Pro Ser Gly Ser Gly Lys Thr Thr Leu
       30                  35                  40
   ctg cgc atc att gct gga ctg gaa cat cag aac agc ggt cag att cgt      194
   Leu Arg Ile Ile Ala Gly Leu Glu His Gln Asn Ser Gly Gln Ile Arg
   45                  50                  55                  60
   ttt cac gac cac gat gtc agc cgc ctg cac gcc cgc gat cgc cag gtc      242
   Phe His Asp His Asp Val Ser Arg Leu His Ala Arg Asp Arg Gln Val
                   65                  70                  75
   gga ttt gtc ttc cag cac tat gcg ctg ttc cgt cat atg acg gtc ttc      290
   Gly Phe Val Phe Gln His Tyr Ala Leu Phe Arg His Met Thr Val Phe
               80                  85                  90
   gac aat att gcc ttt ggc ctg acc gtg ctg ccg cgc cgt gag cgt ccg      338
   Asp Asn Ile Ala Phe Gly Leu Thr Val Leu Pro Arg Arg Glu Arg Pro
           95                  100                 105
   tcc agt gcg gaa att aaa aaa cgc gtc acg cgc ctg ctg gag atg gtg      386
   Ser Ser Ala Glu Ile Lys Lys Arg Val Thr Arg Leu Leu Glu Met Val
       110                 115                 120
   cag ctt tcc cat ctg gcg aac cgt ttc ccg gcc cag ctt tcg gga ggg      434
   Gln Leu Ser His Leu Ala Asn Arg Phe Pro Ala Gln Leu Ser Gly Gly
   125                 130                 135                 140
   cag aag cag cgc gtc gcg ctg gca aga gcc ctg gcc gtg gaa ccg caa        482
   Gln Lys Gln Arg Val Ala Leu Ala Arg Ala Leu Ala Val Glu Pro Gln
                   145                 150                 155
   atc ctg ttg ctg gat gag ccc ttt ggt gcg ctg gac gct cag gtg cgt        530
   Ile Leu Leu Leu Asp Glu Pro Phe Gly Ala Leu Asp Ala Gln Val Arg
               160                 165                 170
   aaa gag ctg cgc cgt tgg tta cgt cag ctg cac gaa gaa ttg aag ttc        578
   Lys Glu Leu Arg Arg Trp Leu Arg Gln Leu His Glu Glu Leu Lys Phe
           175                 180                 185
   acc agc gtg ttc gtc acc cac gat cag gaa gag gcg atg gaa gtg gcc        626
   Thr Ser Val Phe Val Thr His Asp Gln Glu Glu Ala Met Glu Val Ala
       190                 195                 200
   gat cgc gtg gtg gtg atg agc cag ggc agc atc gaa cag gtg ggg acg        674
   Asp Arg Val Val Val Met Ser Gln Gly Ser Ile Glu Gln Val Gly Thr
   205                 210                 215                 220
   ccg gat gaa gtc tgg cgc gat ccc gcc acg cgc ttc gtg ctg gaa ttc        722
   Pro Asp Glu Val Trp Arg Asp Pro Ala Thr Arg Phe Val Leu Glu Phe
                   225                 230                 235
   ctg ggt gag gtt aac cgc ttc gac ggt gaa gtg cat ggt tct cag ttc        770
   Leu Gly Glu Val Asn Arg Phe Asp Gly Glu Val His Gly Ser Gln Phe
               240                 245                 250
   cat gtc ggg gcg cac cac tgg ccg tta ggc tat acc tct gca cat cag        818
   His Val Gly Ala His His Trp Pro Leu Gly Tyr Thr Ser Ala His Gln
           255                 260                 265
   ggc gcg gtc gat ctg ttc ctg cgc ccg tgg gaa atc gac gtt tcg cgc        866
   Gly Ala Val Asp Leu Phe Leu Arg Pro Trp Glu Ile Asp Val Ser Arg
       270                 275                 280
   aga agt agc ctg gaa acg ccg ctg ccc gtt cag gtc tta gaa gtg agt        914
   Arg Ser Ser Leu Glu Thr Pro Leu Pro Val Gln Val Leu Glu Val Ser
   285                 290                 295                 300
   cct cgt ggt cac ttc tgg cag ctg gtg gtg cag cca acg gga tgg cag        962
   Pro Arg Gly His Phe Trp Gln Leu Val Val Gln Pro Thr Gly Trp Gln
                   305                 310                 315
   agc gag ccc ttc tcg ctg gtc ttt gac ggt gaa cag acc gcg ccg ttg       1010
   Ser Glu Pro Phe Ser Leu Val Phe Asp Gly Glu Gln Thr Ala Pro Leu
               320                 325                 330
   cgc ggc gag cgc ctg ttc gtg ggg ctg cag cag gcc aga ctg tac cag       1058
   Arg Gly Glu Arg Leu Phe Val Gly Leu Gln Gln Ala Arg Leu Tyr Gln
           335                 340                 345
   ggc gcg aca ccg tta cgg gcg gtt gcc ttt gca cac agc gcc tga           1103
   Gly Ala Thr Pro Leu Arg Ala Val Ala Phe Ala His Ser Ala
       350                 355                 360
   taggttgagt gaatgttaaa cgcccggagg cgcttcccgc gatccgggct ttttaatggc      1163
   aaggtttgta acctgtagac ctgataagac gcgcaagcgt cgcatcaggc aacaccacgt     1223
   atggatagag atcgtgagta cattagaaca aacaataggc aatacgcctc tggtgaagtt     1283
   gcagcgaatg gggccggata acggcagtga agtgtggtta aaactggaag gcaataaccc      1343
   ggcaggttcg gtgaaagatc gtgcggcact ttcgatgatc gtcgaggcgg aaaagcgcgg     1403
<210> 24
   <211> 362
   <212> PRT
   <213> Pantoea ananatis
<400> 24
   Met Ser Ile Glu Ile Asn Gln Ile Asn Lys Ser Phe Gly Arg Thr Ala
   1 5 10 15
   Val Leu Asn Asp Ile Ser Leu Asp Ile Pro Ser Gly Gln Met Val Ala
   20 25 30
   Leu Leu Gly Pro Ser Gly Ser Gly Lys Thr Thr Leu Leu Arg Ile Ile
   35 40 45
   Ala Gly Leu Glu His Gln Asn Ser Gly Gln Ile Arg Phe His Asp His
   50 55 60
   Asp Val Ser Arg Leu His Ala Arg Asp Arg Gln Val Gly Phe Val Phe
   65 70 75 80
   Gln His Tyr Ala Leu Phe Arg His Met Thr Val Phe Asp Asn Ile Ala
   85 90 95
   Phe Gly Leu Thr Val Leu Pro Arg Arg Glu Arg Pro Ser Ser Ala Glu
   100 105 110
   Ile Lys Lys Arg Val Thr Arg Leu Leu Glu Met Val Gln Leu Ser His
   115 120 125
   Leu Ala Asn Arg Phe Pro Ala Gln Leu Ser Gly Gly Gln Lys Gln Arg
   130 135 140
   Val Ala Leu Ala Arg Ala Leu Ala Val Glu Pro Gln Ile Leu Leu Leu
   145 150 155 160
   Asp Glu Pro Phe Gly Ala Leu Asp Ala Gln Val Arg Lys Glu Leu Arg
   165 170 175
   Arg Trp Leu Arg Gln Leu His Glu Glu Leu Lys Phe Thr Ser Val Phe
   180 185 190
   Val Thr His Asp Gln Glu Glu Ala Met Glu Val Ala Asp Arg Val Val
   195 200 205
   Val Met Ser Gln Gly Ser Ile Glu Gln Val Gly Thr Pro Asp Glu Val
   210 215 220
   Trp Arg Asp Pro Ala Thr Arg Phe Val Leu Glu Phe Leu Gly Glu Val
   225 230 235 240
   Asn Arg Phe Asp Gly Glu Val His Gly Ser Gln Phe His Val Gly Ala
   245 250 255
   His His Trp Pro Leu Gly Tyr Thr Ser Ala His Gln Gly Ala Val Asp
   260 265 270
   Leu Phe Leu Arg Pro Trp Glu Ile Asp Val Ser Arg Arg Ser Ser Leu
   275 280 285
   Glu Thr Pro Leu Pro Val Gln Val Leu Glu Val Ser Pro Arg Gly His
   290 295 300
   Phe Trp Gln Leu Val Val Gln Pro Thr Gly Trp Gln Ser Glu Pro Phe
   305 310 315 320
   Ser Leu Val Phe Asp Gly Glu Gln Thr Ala Pro Leu Arg Gly Glu Arg
   325 330 335
   Leu Phe Val Gly Leu Gln Gln Ala Arg Leu Tyr Gln Gly Ala Thr Pro
   340 345 350
   Leu Arg Ala Val Ala Phe Ala His Ser Ala
   355 360
<210> 25
   <211> 1512
   <212> DNA
   <213> Escherichia coli
<220>
   <221> CDS
   <222> (301)..(1212)
<400> 25
   agccgctggg gtggtacaac gaaccgctga cggtcgtgat gcatggcgac gatgccccgc      60
   agcgtggcga gcgtttattc gttggtctgc aacatgcgcg gctgtataac ggcgacgagc     120
   gtatcgaaac ccgcgatgag gaacttgctc tcgcacaaag cgcctgatag gttgagtgaa     180
   tgttaaacgc ccggaggcgc ttcccgcgat ccgggctttt taatggcaag gtttgtaacc     240
   tgtagacctg ataagacgcg caagcgtcgc atcaggcaac accacgtatg gatagagatc     300
   gtg agt aca tta gaa caa aca ata ggc aat acg cct ctg gtg aag ttg       348
   Val Ser Thr Leu Glu Gln Thr Ile Gly Asn Thr Pro Leu Val Lys Leu
   1               5                   10                  15
   cag cga atg ggg ccg gat aac ggc agt gaa gtg tgg tta aaa ctg gaa       396
   Gln Arg Met Gly Pro Asp Asn Gly Ser Glu Val Trp Leu Lys Leu Glu
               20                  25                  30
   ggc aat aac ccg gca ggt tcg gtg aaa gat cgt gcg gca ctt tcg atg       444
   Gly Asn Asn Pro Ala Gly Ser Val Lys Asp Arg Ala Ala Leu Ser Met
           35                  40                  45
   atc gtc gag gcg gaa aag cgc ggg gaa att aaa ccg ggt gat gtc tta       492
   Ile Val Glu Ala Glu Lys Arg Gly Glu Ile Lys Pro Gly Asp Val Leu
       50                  55                  60
   atc gaa gcc acc agt ggt aac acc ggc att gcg ctg gca atg att gcc       540
   Ile Glu Ala Thr Ser Gly Asn Thr Gly Ile Ala Leu Ala Met Ile Ala
   65                  70                  75                  80
   gcg ctg aaa ggc tat cgc atg aaa ttg ctg atg ccc gac aac atg agc       588
   Ala Leu Lys Gly Tyr Arg Met Lys Leu Leu Met Pro Asp Asn Met Ser
                   85                  90                  95
   cag gaa cgc cgt gcg gcg atg cgt gct tat ggt gcg gaa ctg att ctt       636
   Gln Glu Arg Arg Ala Ala Met Arg Ala Tyr Gly Ala Glu Leu Ile Leu
               100                 105                 110
   gtc acc aaa gag cag ggc atg gaa ggt gcg cgc gat ctg gcg ctg gag      684
   Val Thr Lys Glu Gln Gly Met Glu Gly Ala Arg Asp Leu Ala Leu Glu
           115                 120                 125
   atg gcg aat cgt ggc gaa gga aag ctg ctc gat cag ttc aat aat ccc      732
   Met Ala Asn Arg Gly Glu Gly Lys Leu Leu Asp Gln Phe Asn Asn Pro
       130                 135                 140
   gat aac cct tat gcg cat tac acc acc act ggg ccg gaa atc tgg cag      780
   Asp Asn Pro Tyr Ala His Tyr Thr Thr Thr Gly Pro Glu Ile Trp Gln
   145                 150                 155                 160
   caa acc ggc ggg cgc atc act cat ttt gtc tcc agc atg ggg acg acc      828
   Gln Thr Gly Gly Arg Ile Thr His Phe Val Ser Ser Met Gly Thr Thr
                   165                 170                 175
   ggc act atc acc ggc gtc tca cgc ttt atg cgc gaa caa tcc aaa ccg      876
   Gly Thr Ile Thr Gly Val Ser Arg Phe Met Arg Glu Gln Ser Lys Pro
               180                 185                 190
   gtg acc att gtc ggc ctg caa ccg gaa gag ggc agc agc att ccc ggc      924
   Val Thr Ile Val Gly Leu Gln Pro Glu Glu Gly Ser Ser Ile Pro Gly
           195                 200                 205
   att cgc cgc tgg cct acg gaa tat ctg ccg ggg att ttc aac gct tct      972
   Ile Arg Arg Trp Pro Thr Glu Tyr Leu Pro Gly Ile Phe Asn Ala Ser
       210                 215                 220
   ctg gtg gat gag gtg ctg gat att cat cag cgc gat gcg gaa aac acc     1020
   Leu Val Asp Glu Val Leu Asp Ile His Gln Arg Asp Ala Glu Asn Thr
   225                 230                 235                 240
   atg cgc gaa ctg gcg gtg cgg gaa gga ata ttc tgt ggc gtc agc tcc     1068
   Met Arg Glu Leu Ala Val Arg Glu Gly Ile Phe Cys Gly Val Ser Ser
                   245                 250                 255
   ggc ggc gcg gtt gcc gga gca ctg cgg gtg gca aaa gct aac cct gac     1116
   Gly Gly Ala Val Ala Gly Ala Leu Arg Val Ala Lys Ala Asn Pro Asp
               260                 265                 270
   gcg gtg gtg gtg gcg atc atc tgc gat cgt ggc gat cgc tac ctt tct     1164
   Ala Val Val Val Ala Ile Ile Cys Asp Arg Gly Asp Arg Tyr Leu Ser
           275                 280                 285
   acc ggg gtg ttt ggg gaa gag cat ttt agc cag ggg gcg ggg att taa     1212
   Thr Gly Val Phe Gly Glu Glu His Phe Ser Gln Gly Ala Gly Ile
       290                 295                 300
   ggattaatag catcggagac tgatgacaaa cgcaaaactg cctgatgcgc tacgcttatc   1272
   aggcctacaa ggtttctgca atatattgaa ttagcacgat tttgtaggcc ggataaggcg   1332
   tttacgccgc atccggcata aacaaagcgc acttttttaa cagttgttgc tgccgacaaa    1392
   tgcagtattt aattttcgtg aggaaacgcc gtaaggtcat caatcatttt ttgaagtatt   1452
   ggtgagtcct gaccgtcacc ccattgaaga atttttgcgg taagctgatg acgcgctagt   1512
<210> 26
   <211> 303
   <212> PRT
   <213> Escherichia coli
<400> 26
   Val Ser Thr Leu Glu Gln Thr Ile Gly Asn Thr Pro Leu Val Lys Leu
   1 5 10 15
   Gln Arg Met Gly Pro Asp Asn Gly Ser Glu Val Trp Leu Lys Leu Glu
   20 25 30
   Gly Asn Asn Pro Ala Gly Ser Val Lys Asp Arg Ala Ala Leu Ser Met
   35 40 45
   Ile Val Glu Ala Glu Lys Arg Gly Glu Ile Lys Pro Gly Asp Val Leu
   50 55 60
   Ile Glu Ala Thr Ser Gly Asn Thr Gly Ile Ala Leu Ala Met Ile Ala
   65 70 75 80
   Ala Leu Lys Gly Tyr Arg Met Lys Leu Leu Met Pro Asp Asn Met Ser
   85 90 95
   Gln Glu Arg Arg Ala Ala Met Arg Ala Tyr Gly Ala Glu Leu Ile Leu
   100 105 110
   Val Thr Lys Glu Gln Gly Met Glu Gly Ala Arg Asp Leu Ala Leu Glu
   115 120 125
   Met Ala Asn Arg Gly Glu Gly Lys Leu Leu Asp Gln Phe Asn Asn Pro
   130 135 140
   Asp Asn Pro Tyr Ala His Tyr Thr Thr Thr Gly Pro Glu Ile Trp Gln
   145 150 155 160
   Gln Thr Gly Gly Arg Ile Thr His Phe Val Ser Ser Met Gly Thr Thr
   165 170 175
   Gly Thr Ile Thr Gly Val Ser Arg Phe Met Arg Glu Gln Ser Lys Pro
   180 185 190
   Val Thr Ile Val Gly Leu Gln Pro Glu Glu Gly Ser Ser Ile Pro Gly
   195 200 205
   Ile Arg Arg Trp Pro Thr Glu Tyr Leu Pro Gly Ile Phe Asn Ala Ser
   210 215 220
   Leu Val Asp Glu Val Leu Asp Ile His Gln Arg Asp Ala Glu Asn Thr
   225 230 235 240
   Met Arg Glu Leu Ala Val Arg Glu Gly Ile Phe Cys Gly Val Ser Ser
   245 250 255
   Gly Gly Ala Val Ala Gly Ala Leu Arg Val Ala Lys Ala Asn Pro Asp
   260 265 270
   Ala Val Val Val Ala Ile Ile Cys Asp Arg Gly Asp Arg Tyr Leu Ser
   275 280 285
   Thr Gly Val Phe Gly Glu Glu His Phe Ser Gln Gly Ala Gly Ile
   290 295 300
<210> 27
   <211> 313
   <212> DNA
   <213> Escherichia coli
<400> 27
   gcatgcttcc aactgcgcta atgacgcagc tggacgaagg cgggattctc gtcttacccg 60
   taggggagga gcaccagtat ttgaaacggg tgcgtcgtcg gggaggcgaa tttattatcg 120
   ataccgtgga ggccgtgcgc tttgtccctt tagtgaaggg tgagctggct taaaacgtga 180
   ggaaatacct ggatttttcc tggttatttt gccgcaggtc agcgtatcgt gaacatcttt 240
   tccagtgttc agtagggtgc cttgcacggt aattatgtca ctggttatta accaattttt 300
   cctgggggtc gac 313
<210> 28
   <211> 312
   <212> DNA
   <213> Artificial Sequence
<220>
   <223> mutant nlpD promoter
<400> 28
   gcatgcttcc aactgcgcta atgacgcagc tggacgaagg cgggattctc gtcttacccg    60
   taggggagga gcaccagtat ttgaaacggg tgcgtcgtcg gggaggcgaa tttattatcg   120
   ataccgtgga ggccgtgcgc tttgtccctt tagtgaaggg tgagctggct taaaacgtga   180
   ggaaatacct ggatttttcc tggttatttt gccgcaggtc agcgtataat gaagatcttt   240
   tccagtgttg acaagggtcc ttgcacggtt ataatgtcac tggttattaa ccaatttttc   300
   ctgggggtcg ac                                                        312
<210> 29
   <211> 313
   <212> DNA
   <213> Artificial Sequence
<220>
   <223> mutant nlpD promoter
<400> 29
   gcatgcttcc aactgcgcta atgacgcagc tggacgaagg cgggattctc gtcttacccg    60
   taggggagga gcaccagtat ttgaaacggg tgcgtcgtcg gggaggcgaa tttattatcg   120
   ataccgtgga ggccgtgcgc tttgtccctt tagtgaaggg tgagctggct taaaacgtga   180
   ggaaatacct ggatttttcc tggttatttt gccgcaggtc agcgtataat gaagatcttt   240
   tccagtgttc agtagggtgc cttgcacggt tataatgtca ctggttatta accaattttt   300
   cctgggggtc gac                                                       313
<210> 30
   <211> 36
   <212> DNA
   <213> Artificial sequence
<220>
   <223> primer P1
<400> 30
   agctgagtcg acccccagga aaaattggtt aataac 36
<210> 31
   <211> 33
   <212> DNA
   <213> Artificial Sequence
<220>
   <223> primer P2
<400> 31
   agctgagcat gcttccaact gcgctaatga cgc 33
<210> 32
   <211> 33
   <212> DNA
   <213> Artificial Sequence
<220>
   <223> primer P3
<400> 32
   agctgatcta gaaaacagaa tttgcctggc ggc 33
<210> 33
   <211> 33
   <212> DNA
   <213> Artificial Sequence
<220>
   <223> primer P4
<400> 33
   agctgaggat ccaggaagag tttgtagaaa cgc 33
<210> 34
   <211> 32
   <212> DNA
   <213> Artificial Sequence
<220>
   <223> primer P5
<400> 34
   agctgagtcg acgtgttcgc tgaatacggg gt 32
<210> 35
   <211> 32
   <212> DNA
   <213> Artificial Sequence
<220>
   <223> primer P6
<400> 35
   agctgatcta gagaaagcat caggattgca gc 32
<210> 36
   <211> 59
   <212> DNA
   <213> Artificial Sequence
<220>
   <223> primer P7
<220>
   <221> misc-feature
   <222> (25)..(25)
   <223> n is a, c, g, or t
<220>
   <221> misc_feature
   <222> (27)..(28)
   <223> n is a, c, g, or t
<220>
   <221> misc_feature
   <222> (46)..(46)
   <223> n is a, c, g, or t
<220>
   <221> misc_feature
   <222> (49)..(49)
   <223> n is a, c, g, or t
<220>
   <221> misc_feature
   <222> (51)..(51)
   <223> n is a, c, g, or t
<400> 36
   atcgtgaaga tcttttccag tgttnannag ggtgccttgc acggtnatna ngtcactgg 59
<210> 37
   <211> 32
   <212> DNA
   <213> Artificial Sequence
<220>
   <223> primer P8
<220>
   <221> misc_feature
   <222> (16)..(20)
   <223> n is a, c, g, or t
<400> 37
   tggaaaagat cttcannnnn cgctgacctg cg 32
<210> 38
   <211> 60
   <212> DNA
   <213> Artificial Sequence
<220>
   <223> primer P9
<400> 38
   tccgctcacg atttttttca tcgctggtaa ggtcatttat cccccaggaa aaattggtta 60
<210> 39
   <211> 63
   <212> DNA
   <213> Artificial sequence
<220>
   <223> primer P10
<400> 39
   tttcacaccg ctcaaccgca gggcataacc ggcccttgaa gcctgctttt ttatactaag 60
   ttg 63
<210> 40
   <211> 20
   <212> DNA
   <213> Artificial Sequence
<220>
   <223> primer P11
<400> 40
   ctttgtccct ttagtgaagg 20
<210> 41
   <211> 44
   <212> DNA
   <213> Artificial Sequence
<220>
   <223> primer P12
<400> 41
   agctgatcta gaagctgact cgagttaatg gcctcccaga cgac 44
<210> 42
   <211> 33
   <212> DNA
   <213> Artificial Sequence
<220>
   <223> primer P13
<400> 42
   agctgagtcg acatggcaaa ggtatcactg gaa 33
<210> 43
   <211> 34
   <212> DNA
   <213> Artificial Sequence
<220>
   <223> primer P14
<400> 43
   gagaacgccc gggcgggctt cgtgaatatg cagc 34
<210> 44
   <211> 32
   <212> DNA
   <213> Artificial Sequence
<220>
   <223> primer P15
<400> 44
   agctgatcta gacgtgggat cagtaaagca gg 32
<210> 45
   <211> 22
   <212> DNA
   <213> Artificial Sequence
<220>
   <223> primer P16
<400> 45
   aaaaccgccc gggcgttctc ac 22
<210> 46
   <211> 80
   <212> DNA
   <213> Artificial sequence
<220>
   <223> primer DydjN(Pa)-F
<400> 46
   acctctgctg ctctcctgac cagggaatgc tgcattacat cggagttgct tgaagcctgc 60
   ttttttatac taagttggca 80
<210> 47
   <211> 80
   <212> DNA
   <213> Artificial Sequence
<220>
   <223> primer DydjN(Pa)-R
<400> 47
   agacaaaaac agagagaaag acctggcggt gtacgccagg tctggcgtga cgctcaagtt 60
   agtataaaaa agctgaacga 80
<210> 48
   <211> 80
   <212> DNA
   <213> Artificial Sequence
<220>
   <223> primer DfliY-FW
<400> 48
   atggctttct cacagattcg tcgccaggtg gtgacgggaa tgatggcggt tgaagcctgc 60
   ttttttatac taagttggca 80
<210> 49
   <211> 80
   <212> DNA
   <213> Artificial Sequence
<220>
   <223> primer DyecC-RV
<400> 49
   ttacgccgcc aacttctggc ggcaccgggt ttattgatta agaaatttat cgctcaagtt 60
   agtataaaaa agctgaacga 80
<210> 50
   <211> 80
   <212> DNA
   <213> Artificial Sequence
<220>
   <223> primer DcysE(Ec)-F
<400> 50
   ccggcccgcg cagaacgggc cggtcattat ctcatcgtgt ggagtaagca tgaagcctgc    60
   ttttttatac taagttggca                                                 80
<210> 51
   <211> 80
   <212> DNA
   <213> Artificial Sequence
<220>
   <223> primer DcysE(Ec)-R
<400> 51
   actgtaggcc ggatagatga ttacatcgca tccggcacga tcacaggaca cgctcaagtt    60
   agtataaaaa agctgaacga                                                80
<210> 52
   <211> 80
   <212> DNA
   <213> Artificial sequence
<220>
   <223> primer DydjN(Ec)-F
<400> 52
   cactatgact gctacgcagt gatagaaata ataagatcag gagaacgggg tgaagcctgc    60
   ttttttatac taagttggca                                                 80
<210> 53
   <211> 80
   <212> DNA
   <213> Artificial Sequence
<220>
   <223> primer DydjN(Ec)-R
<400> 53
   aaagtaaggc aacggcccct atacaaaacg gaccgttgcc agcataagaa cgctcaagtt    60
   agtataaaaa agctgaacga                                                80
<210> 54
   <211> 43
   <212> DNA
   <213> Artificial Sequence
<220>
   <223> primer ydjN(Ec)-SalIFW2
<400> 54
   acgcgtcgac atgaactttc cattaattgc gaacatcgtg gtg 43
<210> 55
   <211> 35
   <212> DNA
   <213> Artificial Sequence
<220>
   <223> primer ydjN(Ec)-xbaIRV2
<400> 55
   ctagtctaga ttaatggtgt gccagttcgg cgtcg 35
<210> 56
   <211> 29
   <212> DNA
   <213> Artificial Sequence
<220>
   <223> primer ydjN2(Pa)-salIFW
<400> 56
   acgcgtcgac atggatattc ctcttacgc 29
<210> 57
   <211> 29
   <212> DNA
   <213> Artificial Sequence
<220>
   <223> primer ydjN2(Pa)-xbaIRV
<400> 57
   tgctctagat tagctgtgct ctaattcac 29
<210> 58
   <211> 80
   <212> DNA
   <213> Artificial Sequence
<220>
   <223> primer DfliY(Ec)-FW
<400> 58
   atgaaattag cacatctggg acgtcaggca ttgatgggtg tgatggccgt tgaagcctgc 60
   ttttttatac taagttggca 80
<210> 59
   <211> 80
   <212> DNA
   <213> Artificial Sequence
<220>
   <223> primer DfliY(Ec)-RV
<400> 59
   ttatttggtc acatcagcac caaaccattt ttcggaaagg gcttgcagag cgctcaagtt    60
   agtataaaaa agctgaacga                                                80
<210> 60
   <211> 33
   <212> DNA
   <213> Artificial sequence
<220>
   <223> primer fliY(Ec)SalI-F
<400> 60
   acgcgtcgac atgaaattag cacatctggg acg 33
<210> 61
   <211> 31
   <212> DNA
   <213> Artificial Sequence
<220>
   <223> primer fliY(Ec)XbaI-R
<400> 61
   ctagtctaga ttatttggtc acatcagcac c 31
<210> 62
   <211> 147
   <212> DNA
   <213> Escherichia coli
<400> 62
   aaaacgtgag gaaatacctg gatttttcct ggttattttg ccgcaggtca gcgtatcgtg    60
   aagatctttt ccagtgttca gtagggtgcc ttgcacggta attatgtcac tggttattaa   120
   ccaatttttc ctgggggata aatgagc                                       147